# EUROPEAN PATENT APPLICATION

(11) **EP 4 663 204 A1**
(43) Date of publication of application: **17.12.2025**
(21) Application number: 24182123.0
(22) Date of filing: 13.06.2024
(51) Int. Cl.: A61K 47/55, A61P 37/06

(54) **COMPOUNDS USEFUL FOR THE MODULATION OF THE ACTIVITY OF STING**

(71) Applicant: Sulis Therapeutics ApS, 8000 Aarhus C (DK)
(72) Inventor: The designation of the inventor has not yet been filed
(74) Representative: Mewburn Ellis LLP

(57) **Abstract**

The invention relates to a compound of formula (I):

S1-L-E (I),

wherein S1 is a STING ligand, L is a Linker, and E is an Inhibitor of Apoptosis (IAP) E3 ubiquitin ligase ligand, wherein the STING ligand does not form a covalent bond with STING and wherein the STING ligand does not exhibit STING agonist activity.

## Description

### Field of the Invention

The present invention relates to compounds useful for the modulation of the activity of Stimulator of Interferon Genes (STING) and particularly, although not exclusively, to proteolysis targeting chimeric (PROTAC^{®}) compounds, also referred to herein as degraders and degrader compounds, which induce degradation of STING by binding to both STING and an E3 ubiquitin ligase, and uses of such compounds.

### Background

### Protacs^{®}

Traditional small molecule drugs reversibly (or sometimes irreversibly) bind to a target protein as a means of modulating a given biological activity. In contrast, PROTACs (also referred to as degraders or degrader compounds) bind to their target proteins, but then bring about the target protein's degradation. Having achieved this effect, the degrader is in theory able to repeat this process with another target protein. Accordingly, unlike with "traditional small molecule" inhibitors, the degrader compound-driven degradation mechanism can in theory operate in a sub-stoichiometric manner - meaning that more modest exposures of a degrader compound could still achieve a desired level of efficacy in vivo. In practice this can mean that the degradation power (DC50 and Dmax) of a degrader compound can have an improved effect than that reflected only by its binding affinity. In addition, while the ligand of a degrader may influence target protein function independent of the degrader compound, this is not necessary. For example, the target protein ligand may, when not part of a degrader compound, bind to a target protein without affecting the target protein's activity.

At a simplistic level, degrader molecules are often described as having three parts - (1) a part that is capable of binding to the target protein to be degraded, (2) a second part that is capable of binding to an E3 ubiquitin ligase, and finally, a linker that connects (1) and (2) together.

In use, the degrader binds to both the target protein and E3 ubiquitin ligase simultaneously to form a ternary complex. The E3 ligase then recruits an E2 conjugating enzyme to the ternary complex, which ubiquitinates the target protein. This has the effect of labelling the target protein for degradation by the cell's proteasome machinery. A degrader can then dissociate from the target protein and initiate another cycle of this process in a catalytic manner. Meanwhile, the ubiquitinated target proteins are recognized and degraded by the cell's proteasome machinery.

Proteolysis targeting chimeric (PROTAC^{®}) compounds are bifunctional molecules comprising a target protein ligand joined to a E3 ubiquitin ligase ligand by a linker (Békés et al., 2022). These molecules are used as modulators of targeted ubiquitination of proteins. Formation of a ternary complex between the PROTAC^{®}, the target protein and the E3 ligase leads to the specific polyubiquitination of the target protein and its subsequent intracellular degradation via the proteasome. PROTAC^{®} compounds have been shown to be capable of the targeted degradation of a range of proteins.

Over 600 human E3 ubiquitin ligases are known, however among these there are a more limited selection of ubiquitin ligases which have been identified for use in degrader compounds.

Cereblon (CRBN) is a substrate specificity receptor that associates with the Cullin-4-RING E3 ubiquitin ligase (CRL4) complex. Known ligands for CRBN include thalidomide and other immunomodulatory imide drugs (IMiDs). CRBN has been widely used as an E3 ligase in PROTAC^{®} compounds targeting a range of proteins.

The Von Hippel-Lindau (VHL) protein is a part of a multiprotein complex which possesses an E3 ubiquitin ligase activity. Similarly to CRBN, VHL ligands have been used extensively in the design of PROTAC^{®} compounds targeting a range of proteins.

The Inhibitor of Apoptosis Proteins (lAPs) family includes antiapoptotic proteins, which are commonly overexpressed in some cancer cells and promote their survival, as well as the survival of neuronal cells. Among this family, certain lAPs possess E3 ubiquitin ligase activity, such as cellular lAPs 1 and 2 (clAP-1/2) and ligands for these molecules have been incorporated into the design of PROTAC^{®} compounds.

### STING

Stimulator of Interferon Genes (STING) is an intracellular adaptor protein which plays an important role in innate immunity. STING induces type I interferon (IFN) production in addition to other proinflammatory mediators such as cytokines in response to infection or cellular damage. Cytosolic STING is activated upon binding cyclic dinucleotides (CDNs), which can be derived from bacterial sources or 2',3'-cyclic GMP-AMP (2',3'-cGAMP) generated by cyclic GMP-AMP synthase (cGAS), an enzyme that is activated by binding to double-stranded DNA in the cytoplasm. In addition to the canonical CDN-dependent activation, STING can also be activated in a non-canonical CDN-independent manner, for example through gain-of-function mutations (Decout et al., 2021).

Activation of STING leads to up-regulation of type I interferon production. Activation of type I interferon production is an approach which has been explored in the treatment of some cancers, and numerous STING agonists have been designed for this purpose. Examples of STING agonists include benzimidazoles, for example as described in WO2017/175147, WO2019/069270, WO2020/132582, WO2020/132566, and WO2020/132549 the content of each of which is hereby incorporated by reference.

WO2013/106643 describes compounds and methods for the enhanced degradation of targeted proteins and other polypeptides by an E3 ubiquitin ligase.

WO2016/197032 describes imide- based compounds, including bifunctional compounds comprising the same, which find utility as modulators of targeted ubiquitination, especially inhibitors of a variety of polypeptides and other proteins which are degraded and/or otherwise inhibited by bifunctional compounds.

WO2016/169989 describes PROTAC compounds incorporating a selective E3 Ligase IAP binding moiety (IAP binding moiety) as the degradation component, functioning to recruit target proteins to the E3 ubiquitin ligase IAP for degradation.

WO2018/066545 describes drugs that can decompose a target intracellular protein specifically and is effective for the prevention or treatment of diseases associated with the target protein. A SNIPER compound produced by linking a specific IAP ligand, through a linker, to a ligand capable of specifically binding to a target intracellular protein.

In contrast, many monogenic autoinflammatory syndromes, autoimmune diseases, inflammatory diseases, neurological disorders, metabolic diseases, cardiovascular diseases, and cancers have been linked to the overexpression of STING and activation of the cyclic GMP-AMP synthase pathway.

Monogenic autoinflammatory diseases are associated with mutations in genes that participate in innate immunity or that control innate immune homeostasis. STING-associated vasculopathy with onset in infancy (SAVI) is one such disease. SAVI is characterized by recurrent fevers, ulcerative skin lesions, vasculitis and interstitial lung disease and is mostly caused by autosomal dominant mutations in STING1 (Decout et al., 2021).

Another monogenic autoinflammatory disease is COPA syndrome, a rare early-onset autosomal dominant disease which can affect the lungs, kidneys, and joints, caused by missense mutations in the COPA gene, which encodes the COPα protein subunit of the COPI complex. This mutation impairs the binding and sorting of proteins targeted for ER retrieval, and promotes ligand-independent activation of STING-mediated signaling (Decout et al., 2021).

Aicardi--Goutières syndrome (AGS) is a further example of a monogenic autoinflammatory disease, which is characterised by early onset of progressive encephalopathy and skin lesions (known as chilblains). Mutations in a subset of genes associated with nucleic acid sensing or metabolism - specifically TREX1, and genes encoding the three RNase H2 endonuclease subunits, RNASEH2A, RNASEH2B, and RNASEH2C and the dNTPase SAMHD1 - have been associated with disturbed self-DNA metabolism and constitutive activation of the cGAS-STING pathway. TREX1 deficiency is therefore another disease linked to STING and activation of the cyclic GMP-AMP synthase pathway (Decout et al., 2021). Similarly, DNase II deficiency is a further autoinflammatory disease linked to STING and activation of the cyclic GMP-AMP synthase pathway (Rodero et al., 2017). Familial chilblain lupus is a further monogenic autoinflammatory disease which is a form of cutaneous lupus erythematosus caused by a gain-of-function in STING, or loss-of-function mutations in the nuclease genes TREX1 and SAMHD1 (Konig et al., 2017).

The autoimmune disease systemic lupus erythematosus (SLE) has also been linked with cGAS pathway activity and type I interferon induction via STING, providing a mechanism for how cGAS-STING may amplify or accelerate disease symptoms. Sjögren's syndrome is a further autoimmune disorder characterized by an increased type 1 interferon gene signature (Decout et al., 2021).

Inflammation is also a prominent hallmark of several neurodegenerative diseases, and elevated levels of type I interferons and contributions of STING have also been observed in models and disease samples of Huntington disease (Decout et al., 2021).

Other diseases and conditions linked to STING and activation of the cyclic GMP-AMP synthase pathway include rheumatoid arthritis, ischaemic brain injury, Parkinson disease, general neurodegeneration, amyotrophic lateral sclerosis and frontotemporal dementia, systemic sclerosis, age-dependent macular degeneration, traumatic brain injury, metabolic dysfunction-associated steatohepatitis (previously termed non-alcoholic steatohepatitis), alcoholic liver disease, acute pancreatitis, silica-induced fibrosis, sepsis, cardiovascular diseases, myocardial infarction, chronic heart failure, colorectal cancer, skin cancer, metastases, senescence, and aging (Decout et al., 2021, Paul et al., 2022 and Seok et al., 2023).

The identification of the link to these diseases and conditions has led increased focus on STING as a potential therapeutic target. The search for STING inhibitors has yielded several small molecules targeting STING which have anti-inflammatory effects. So far there have been two main categories of STING inhibitor design. The first uses molecules which bind non-covalently to STING and act as competitive antagonists of STING activators. Examples of this type of inhibitor molecule are isoquinolones as described in WO2019/182886, the content of which is hereby incorporated by reference. Isoquinolone compounds have been shown to bind to STING in a 2:1 stoichiometry, wherein two of their small molecules bind one STING dimer (Siu et al. 2019). Secondly, inhibitors have been designed that bind covalently to cysteine residues in the transmembrane domain of STING. Cysteine-reactive molecules are prone to being non-specific, which is due to covalent binding to cysteine in other non-target proteins, meaning STING inhibitors which bind covalently have a higher risk of off-target activities when used therapeutically. More recently, targeted STING degradation using 'molecular glue' degraders has been explored, with a one compound being identified which can induce STING degradation (Mutlu et al. 2024). However, while targeted STING degradation was demonstrated in this study, the potency of the reported degrader is relatively weak.

An ongoing need exists in the art for effective treatments of disease associated with unwanted or uncontrolled activation of STING. However, STING antagonists rely on high systemic drug exposures to maintain sufficient STING inhibition, which increases the risk of unwanted off-target effects. Furthermore, existing STING antagonists are less effective in the treatment of diseases driven by constitutive (agonist-free) activation of STING.

### Lymphoablation

Immunosuppression is needed to control some unwanted immune reactions. For instance, in the context of organ or tissue transplantation, immunosuppressives are often used to reduce the risk of transplant rejection. Rejection occurs if the host (patient) immune system mounts a strong reaction against the grafted organ or tissue, which can lead to it being destroyed. The reverse condition can also occur when white blood cells within the transplanted organ or tissue mount an immune reaction against the host (patient). This condition is called graft-versus-host disease (GvHD). Intense immunosuppression can be required to protect the host.

Anti-thymocyte globulins can be used as lymphoablative medicines. For example, Thymoglobulin (Sanofi) can be used as part of the immunosuppressive protection against transplant rejection and GvHD. Thymoglobulin is a rabbit polyclonal antibody that targets lymphocytes (T cells and B cells). It is used to prevent and treat both acute T cell mediated rejection (TCMR) and antibody mediated rejection (ABMR). Horse derived anti-thymocyte globulins are also known. However, these polyclonal antibody therapeutics have several drawbacks. Their long-term effects are not well understood. The patient's immunological recovery following treatment may be impacted and there may be long term suppression of CD4+ helper T cells (which play a critical role in the adaptive immune response). Besides its effects on lymphocyte numbers, Thymoglobulin may also affect monocyte populations, potentially impacting the innate immune system as well.

The present invention has been devised in light of the above considerations.

### Summary of the Invention

In a first aspect, the present invention provides a compound of formula (I):

*S1-L-E* (I),

wherein:
S1 is a STING ligand;
L is a Linker;
E is an Inhibitor of Apoptosis (IAP) E3 ubiquitin ligase ligand,
and pharmaceutically acceptable salts thereof,
wherein the STING ligand does not form a covalent bond with STING and
wherein the STING ligand does not exhibit STING agonist activity.

In some embodiments, L connects the STING ligand to the E3 ubiquitin ligase ligand via a chain of atoms having 10-40 atoms in shortest length.

In some embodiments, the compound is of formula (I-1): 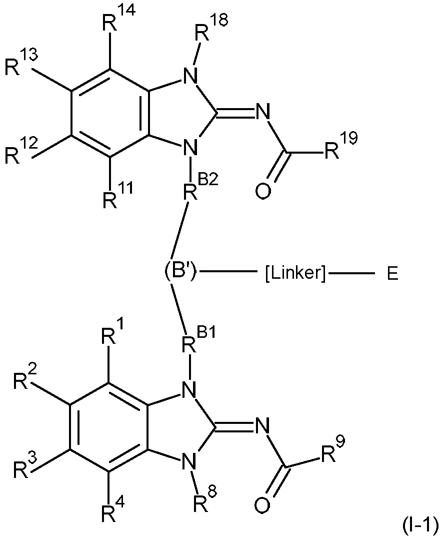
or a tautomer or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl);
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, NH(C₁₋₆ alkyl), N(C₁₋₆ alkyl)₂, CO₂(C₁₋₆ alkyl) and halogen;
R³ and R¹³ are each independently selected from H, C(=O)NH₂; optionally substituted - C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH(C₁₋₆ alkyl), NH₂, CO₂(C₁₋₆ alkyl) and halogen;
R⁸ and R¹⁸ are each independently selected from H, and C₁₋₄ alkyl;
B' taken together with R^{B1} and R^{B2} forms a linking group having 3-7 atoms in shortest length;
B' is the point of attachment to the Linker group L as follows: 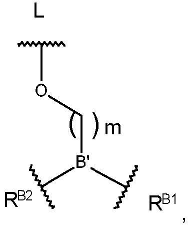
wherein m is 0 or 1;
the Linker (L) is a saturated or a partially or fully unsaturated framework comprising C and H atoms and at least one heteroatom, wherein said framework has a minimum length of from 10 to 40 atoms between B' and E; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, C!, and CN;
wherein each R^{L} is independently selected from C₁₋₆ alkyl, C₂₋₆ alkenyl, C₂₋₆ alkynyl, C₃₋₆ cycloalkyl, C₃₋₆ carbocyclyl, C₅₋₆ heterocyclyl, C₅₋₆ aryl, and C₅₋₆ heteroaryl; and
E is an Inhibitor of Apoptosis (IAP) E3 ubiquitin ligase ligand.

In a second aspect, the present invention provides the compound of the first aspect, or a pharmaceutically acceptable salt thereof, for use in a method of reducing lymphocyte count in a subject, the method comprising administering a therapeutically effective amount of the compound, or a pharmaceutically acceptable salt thereof, to a subject in need thereof.

In a third aspect, the present invention provides a compound according to the first aspect, or a pharmaceutically acceptable salt thereof, for use as a medicament.

In a fourth aspect, the present invention provides the use of a compound according to the first aspect, or a pharmaceutically acceptable salt thereof, for the manufacture of a medicament for the treatment of a disease or condition a subject in need thereof.

In a fifth aspect, the present invention provides a method of reducing lymphocyte count in a subject, comprising administering a therapeutically effective amount of a compound according to the first aspect, or a pharmaceutically acceptable salt thereof, to a subject in need thereof.

In a sixth aspect, the present invention provides a use of the compound according to the first aspect, or a pharmaceutically acceptable salt thereof, in the manufacture of a medicament for reducing lymphocyte count in a subject in need thereof.

In a seventh aspect, the present invention provides a pharmaceutical composition comprising the compound according to the first aspect, or a pharmaceutically acceptable salt thereof, and a pharmaceutically acceptable excipient.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

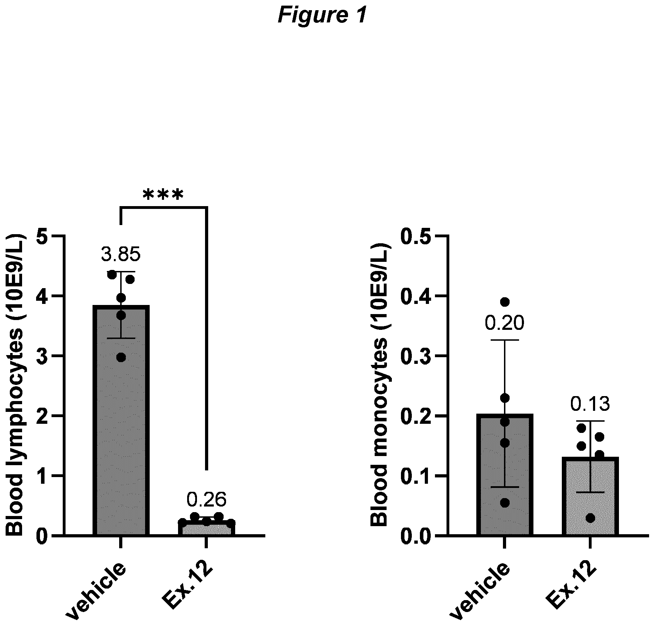

**Figure 1****.** Concentration of blood lymphocytes (left hand plot) and monocytes (right hand plot) following two subcutaneous administrations of a compound of the invention (Example 12) or a vehicle control. The compound of the invention reduces blood lymphocytes (p<0.001) without significantly affecting monocyte count (statistical analysis of the difference between vehicle and the compound of the invention by unpaired t test).

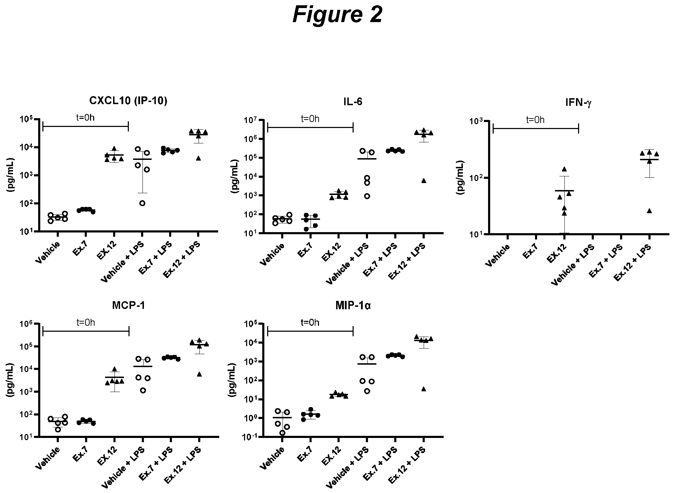

**Figure 2****.** Effect of the compound of the invention, with or without LPS, on the levels of CXCL10, IL-6, MCP-1 (CCL2), MIP-1α (CCL3) and IFN-γ. *In vivo* administration of a compound of the invention, Example compound 12, or control Example compound 7, with or without LPS. The compound of the invention achieved a broad upregulation of these cytokines and this effect is potentiated by LPS.

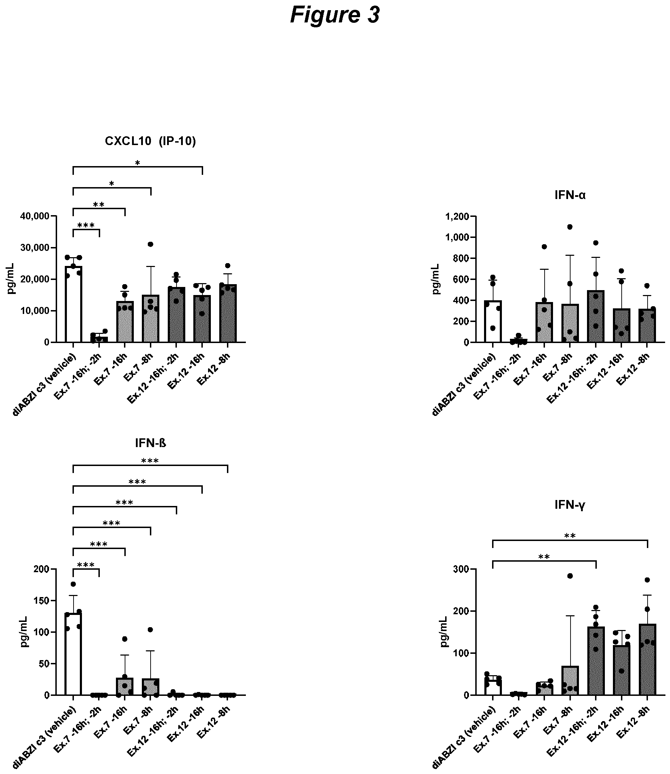

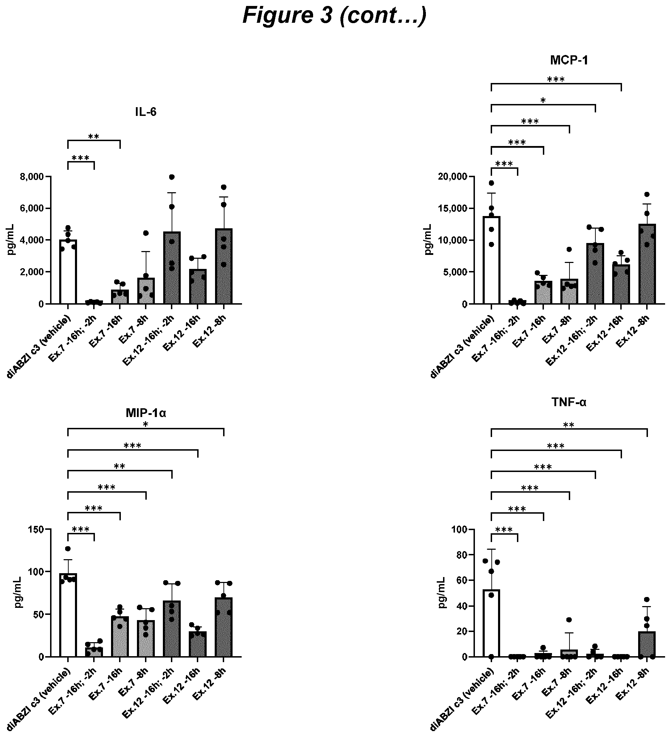

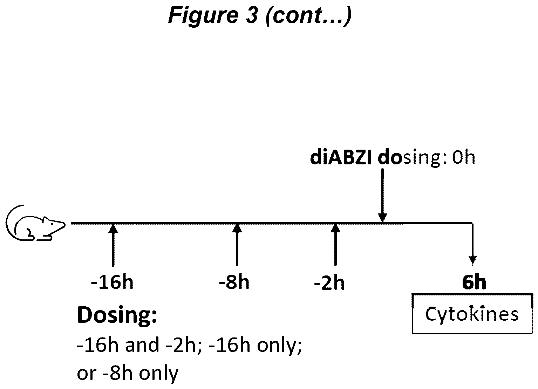

**Figure 3****.** Effect of the compound of the invention on the levels of CXCL-10 (IP-10), IFN-γ, IFN-α, IFN-β, TNF-α, MCP-1 (CCL2), MIP-1α (CCL3) and IL-6 before administration of the STING agonist diABZI c3. *In vivo* administration of Example compound 12 of the invention, or control Example compound 7; and a schematic diagram showing the dosing arms of mice pre-treated with Example compound 7 followed by 1 mg/kg diABZI c3 treatment at t = 0 hours.

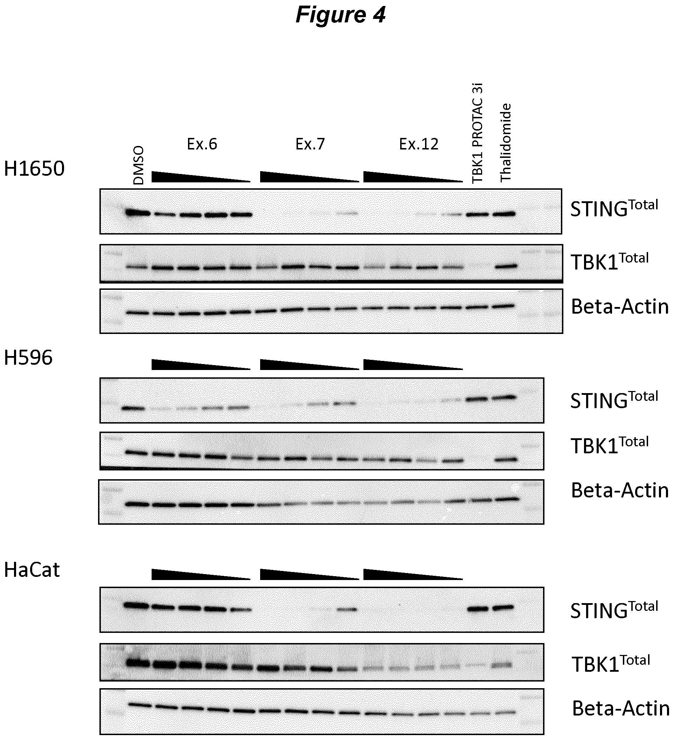

**Figure 4****.** Western Blots analysis of STING protein degradation in H1650 cells (top panel), H596 cells (middle panel), and HaCat cells (bottom panel) following treatment with vehicle control (DMSO), Example compound 6, Example compound 7, Example compound 12, positive control TBK1 PROTAC^{®} i3 or 2µM thalidomide. Cells were treated with increasing concentrations of Example compounds 6, 7, or 12. Example compounds 7 and 12 induce STING degradation in all cell lines tested at all concentrations tested. Example compound 6 induces visible degradation in H1650 and H596 at higher concentrations.

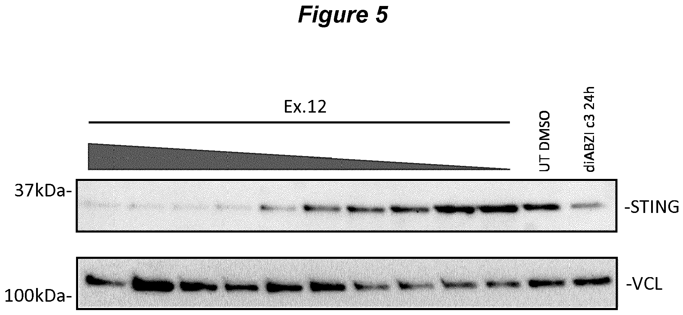

**Figure 5****.** Western Blots analysis of STING protein degradation in mouse RAW264.7 cells. RAW264.7 cells were treated with increasing doses of Example compound 12 of the invention. Western blots demonstrating STING degradation upon treatment with the compound of the invention (top panel). Graph shows a dose-dependent induction of STING degradation (bottom panel). STING protein concentrations were normalised to vinculin protein concentrations. UT DMSO = untreated DMSO.

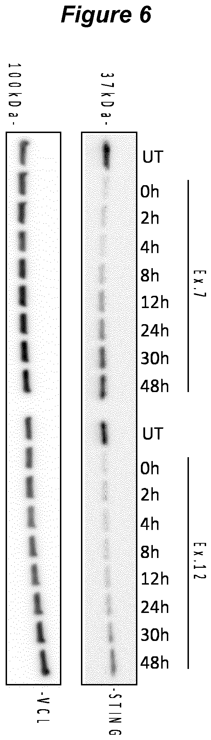

**Figure 6****.** Western blot analysis of STING protein recovery following 24 hours treatment with, and washout of, Example compound 7 (control compound) or Example compound 12 (compound of the invention). 48 hours after Example compound washout, STING protein levels fail to recover to untreated (UT DMSO) levels, indicating that Example compounds persist in cells for an extended period and exert prolonged protein degradative effects. VCL = vinculin.

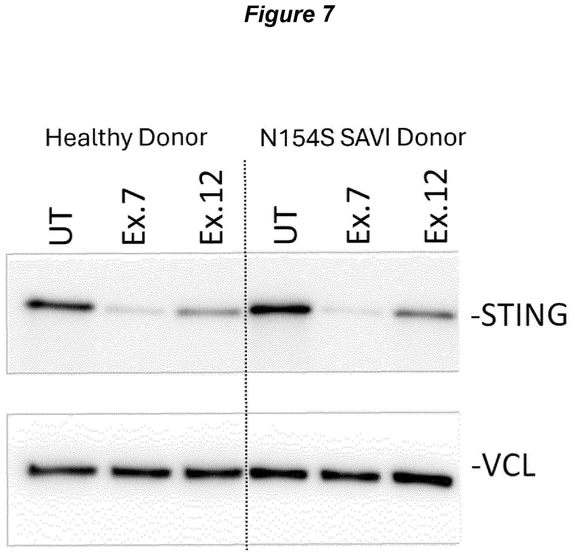

**Figure 7****.** Western blot analysis of STING protein degradation in healthy donor fibroblasts and STING-associated vasculopathy with onset in infancy (SAVI) patient fibroblasts comprising a constitutively active STING protein harboring a N154S mutation. Both control Example compound 7 and Example 12 compound of the invention induce STING degradation in healthy and patient fibroblast cells induces. UT = untreated. VCL = vinculin.

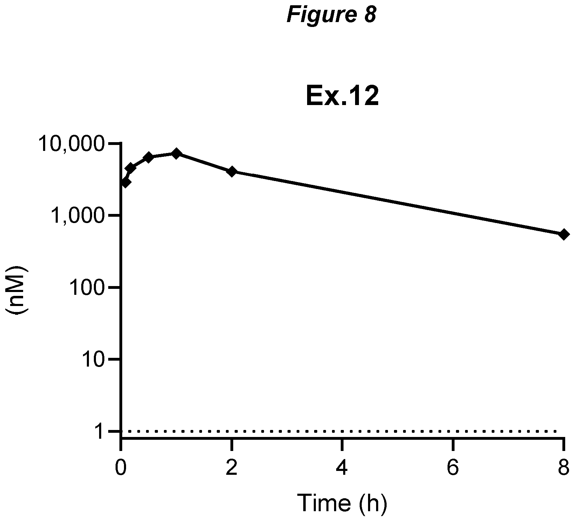

**Figure 8****.** Pharmacokinetic profiling in mice subcutaneously (SC) injected with 8 mg/kg of a compound of the invention. Plasma concentration of the compound was measured over the course of 8 hours. The dotted horizontal line represents the lower limit of quantification (1 nM).

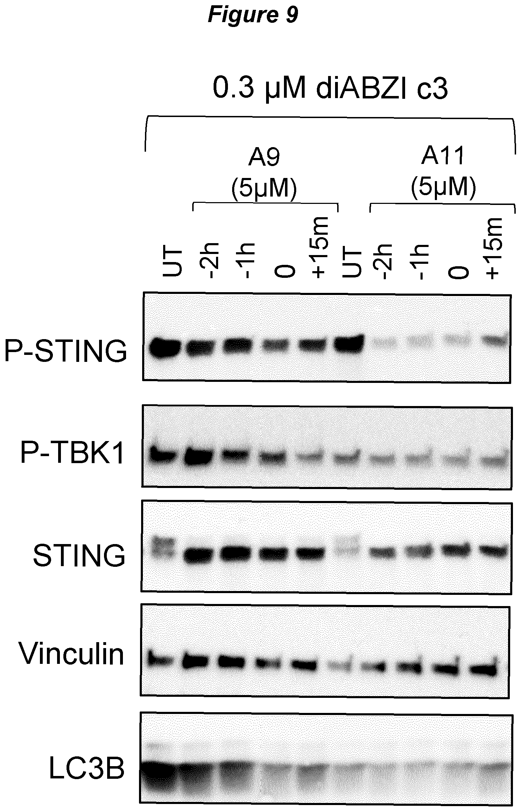

**Figure 9****.** Western Blot analysis of S1 STING ligand antagonism in human skin fibroblast. Cells were treated with 5 µM of A9 or A11 compound either before, simultaneously with, or after activation with 0.3 µM diABZI. Cells were either left untreated with Example compound (UT), pre-treated for 2 hours (-2h) before activation, pre-treated for 1 hour (-1h), treated with A9 or A11 simultaneously with diABZI c3 (0), or treated with A9 or A11 15 minutes after activation (+15m). The WB membrane was probed for p-STING, p-TBK1, STING, Vinculin and LC3B. Pretreatment, simultaneous treatment or post-treatment with either A9 or A11 inhibits diABZI c3-induced STING activation, as shown by a decrease in P-STING relative to cells treated only with diABZI c2 (UT). A9 was further demonstrated to inhibit diABZI c3-induced STING activation by a decrease in LC3B lipidation, a downstream effector of STING.

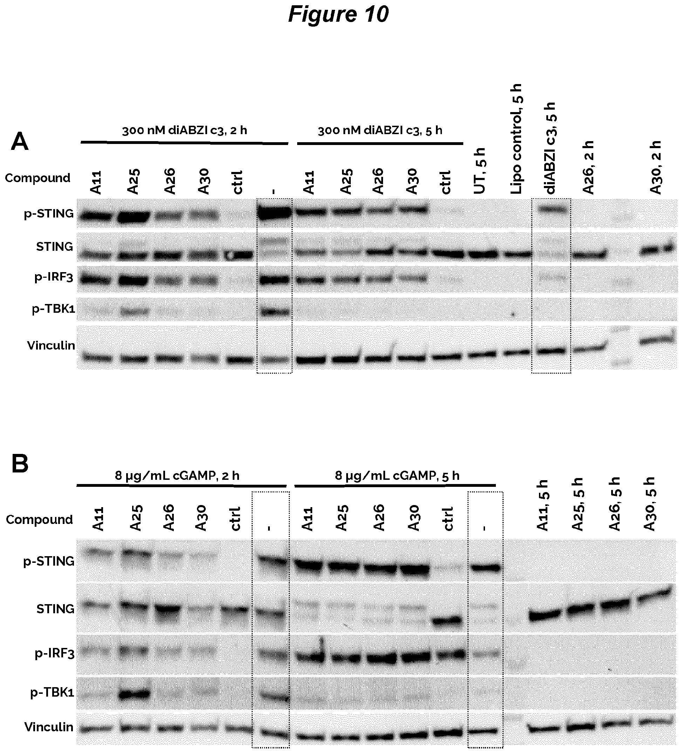

**Figure 10****.** Western Blot analysis of inhibition of STING activation in HFF-1 cells. (A) HFF-1 cells treated with 5 µM of STING binder compounds A11, A25, A26, A30, or 0.5 µM positive control compound for 15 min followed by 300 nM diABZI and incubated for 2 h or 5 h. As controls, cells were left untreated before activation with 300 nM diABZI and incubated for 2 h (-), left untreated (UT), treated with Lipofectamine (Lipo control), A26 or A30 as single agent. (B) shows HFF-1 cells treated with 5 µM of STING binder compounds A11, A25, A26, A30, or 0.5 µM STING antagonist for 15 min followed with 8 µg/mL Lipo-cGAMP and incubated for 2 h or 5 h.. As controls, cells were left untreated before activation with 8 µg/mL Lipo-cGAMP and incubated for 2 h (-), or treated with A11, A25, A26 or A30 as single agent. Western Blot membranes were probed for p-STING, STING, p-IRF3, p-TBK1, and Vinculin. Compared to cells which were left untreated prior to activation with diABZI c3 or cGAMP, STING binder compounds A11, A26 and A30 inhibited STING, IRF3 and TBK1 phosphorylation and activation. A11, A25, A26, A30 do not induce STING, IRF3 or TBK1 phosphorylation and activation alone.

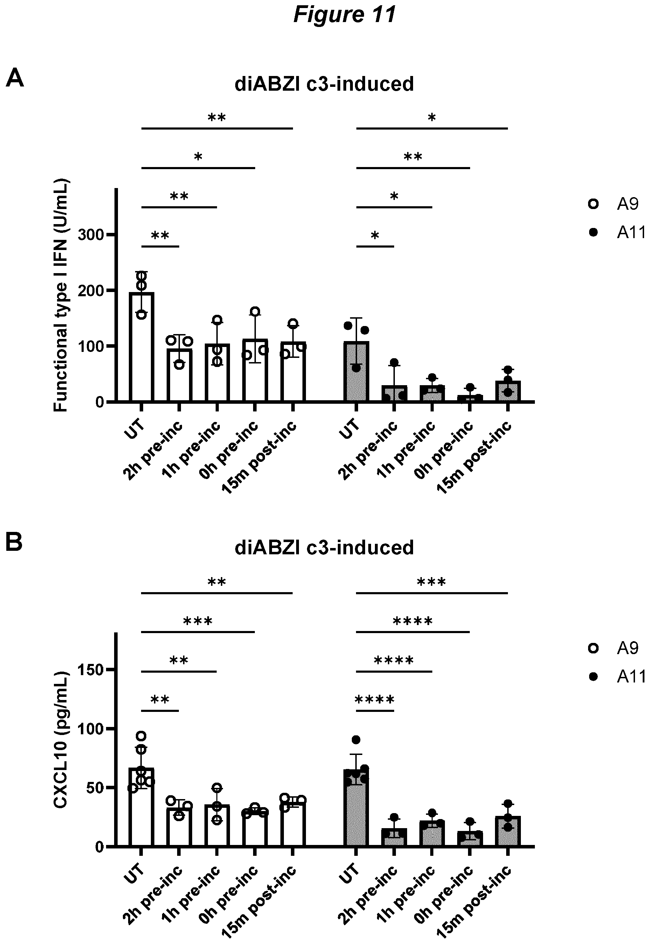

**Figure 11****.** Analysis of secretion of functional IFN-β (A) and CXCL10 (B) from human skin fibroblasts. Cells were treated with 5 µM of A9 or A11 compound either before, simultaneously with, or after activation with 0.3 µM diABZI. Cells were either left untreated with Example compound (UT), pre-treated for 2 hours before activation (-2hr), pre-treated for 1 hour before activation (-1hr), treated with A9 or A11simulataneously with diABZI c3 (0), or treated with A9 or A11 15 minutes after activation (+15m). Pretreatment, simultaneous treatment, or post-treatment with either A9 or A11 inhibited diABZI c3-induced STING activation and consequently inhibits secretion of Functional Type I IFN (A) and CXCL10 (B).

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Definitions

### Substituents

The phrase "optionally substituted" as used herein, pertains to a parent group which may be unsubstituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

Examples of substituents are described in more detail below.

Unless otherwise stated, halogen or halo is selected from chloro (Cl), fluoro (F), bromo (Br) and iodo (I), such as fluoro.
Hydroxy: -OH.
Oxo: =O (oxygen double bonded to the rest of the molecule).
C₁₋₁₂ hydrocarbon: The term "C₁₋₁₂ hydrocarbon" as used herein pertains to a monovalent moiety obtained by removing a hydrogen atom from a carbon atom of a hydrocarbon compound having from 1 to 12 carbon atoms, which may be aliphatic or alicyclic, which may be saturated or unsaturated (e.g. partially unsaturated, fully unsaturated) and may also be branched. Thus, the term "hydrocarbon" includes the terms alkyl, alkenyl, alkynyl, cycloalkyl, etc., discussed below.
C₁₋₈ alkyl: The term "C₁₋₈ alkyl" as used herein, pertains to a monovalent moiety obtained by removing a hydrogen atom from a carbon atom of a hydrocarbon compound having from 1 to 8 carbon atoms, which may be aliphatic or alicyclic, and which are saturated and may also be branched. The term "C₁₋₆ alkyl" as used herein, pertains to a monovalent moiety obtained by removing a hydrogen atom from a carbon atom of a hydrocarbon compound having from 1 to 6 carbon atoms, which may be aliphatic or alicyclic, and which are saturated. The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which are saturated. The term "C₁₋₈ alkyl" as used herein encompasses both linear and branched alkyl groups. Thus, the term "alkyl" includes the sub-classes cycloalkyl, discussed below.
Me: methyl, -CH₃
Et: ethyl, -CH₂CH₃.

Examples of saturated alkyl groups include, but are not limited to, methyl (C₁), ethyl (C₂), propyl (C₃), butyl (C₄), pentyl (C₅) hexyl (C₆), heptyl (C₇), octyl (C₈).

Examples of saturated linear alkyl groups include, but are not limited to, methyl (C₁), ethyl (C₂), n-propyl (C₃), n-butyl (C₄), n-pentyl (amyl) (C₅) and n-hexyl (C₆).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₃₋₁₀ cycloalkyl: The term "C₃₋₁₀ cycloalkyl" as used herein, pertains to an alkyl group which is also a cyclyl group; that is, a monovalent moiety obtained by removing a hydrogen atom from a ring atom of a cyclic hydrocarbon (carbocyclic) compound, which moiety has from 3 to 10 carbon atoms, including from 3 to 10 ring atoms. The carbocyclic ring may be saturated or unsaturated and may be bridged or unbridged. The ring may be a fused ring or a single ring.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) methylcyclohexane (C₇), cyclooctane (C₈), cyclononane (C₉) and cyclodecane (C₁₀);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

C₂₋₈ alkenyl: The term "C₂₋₈ alkenyl" as used herein, pertains to a non-aromatic hydrocarbon group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₈alkynyl: C₂₋₈ alkynyl: The term "C₂₋₈ alkynyl" as used herein, pertains to a hydrocarbon group having one or more carbon-carbon triple bonds.

Examples of unsaturated alkynyl groups include, but are not limited to, ethynyl (-C=CH), 1-propynyl (-C=CCHs) and 2-propynyl (propargyl, -CH₂C=CH).

Ether: -OR, or -R-O-R- wherein R is an ether substituent, for example, a C₁₋₁₂ hydrocarbon group (also referred to as a C₁₋₁₂ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

C₁₋₆ alkoxy: The term C₁₋₆ alkoxy as used herein, pertains to an OR group, wherein R is an C₁₋₆ alkyl group. Examples of C₁₋₆ alkoxy groups include, but are not limited to, Ome, Oet (ethoxy), -O(nPr) (n-propoxy), -O(iPr) (isopropoxy), O(nBu) (n-butoxy), O(sBu) (sec-butoxy), O(iBu) (isobutoxy), and O(tBu) (tert-butoxy).

C₃₋₄ alkenoxy: The term C₃₋₄ alkenoxy as used herein, pertains to an OR group, wherein R is an C₃₋₄ alkenyl group. Examples of C₃₋₄ alkenoxy include O-CH=CH-CH₃, O-C(CH₃)=CH₂, or O-butenyl.

C₃₋₄ alkynoxy: The term C₃₋₄ alkynoxy as used herein, pertains to an OR group, wherein R is a C₃₋₄alkynyl group. Examples of C₃₋₄ alkynoxy groups include O-CHzC=CH, OCH₂CH₂C≡CH or O-C≡CCH₃.

C₃₋₁₀ heteroalkyl; The term C₃₋₁₀ heteroalkyl as used herein pertains to a C₃₋₁₀ alkyl group which chain is interrupted by 1 to 3 heteroatoms selected from O, S and N. In other words, a C₃₋₁₀ alkyl chain, which chain includes 1 to 3 heteroatoms selected from O, S and N. Examples of C₃₋₁₀ heteroalkyl groups include but are not limited to CH₂CH₂OCH₃, CH₂CH₂OCH₂CH₃, CH₂CH₂OCH₂CH₂CH₃, CH₂OCH₂CH₃, CH₂OCH₂CH₂CH₃, CH₂OCH₂CH₂CH₂CH₃, CH₂CH₂OCH₂CH₂CH₂CH₂CH₃, CH₂OCH₂CH₂CH₂CH₂CH₃, CH₂CH₂CH₂OCH₃, CH₂CH₂CH₂CH₂CH₂CH₂OCH₃, CH₂CH₂SCH₃, CH₂CH₂SCH₂CH₃, CH₂CH₂SCH₂CH₂CH₃, CH₂SCH₂CH₃, CH₂SCH₂CH₂CH₃, CH₂SCH₂CH₂CH₂CH₃, CH₂CH₂SCH₂CH₂CH₂CH₂CH₃, CH₂SCH₂CH₂CH₂CH₂CH₃, CH₂CH₂CH₂SCH₃, CH₂CH₂CH₂CH₂CH₂CH₂SCH₃, CH₂CH₂NHCH₃, CH₂CH₂NHCH₂CH₃, CH₂CH₂NHCH₂CH₂CH₃, CH₂NHCH₂CH₃, CH₂NHCH₂CH₂CH₃, CH₂NHCH₂CH₂CH₂CH₃, CH₂CH₂NHCH₂CH₂CH₂CH₂CH₃, CH₂NHCH₂CH₂CH₂CH₂CH₃, CH₂CH₂CH₂NHCH₃, CH₂CH₂CH₂CH₂CH₂CH₂NHCH₃, CH₂CH₂N(CH₂)₂, CH₂N(CH₂CH₂)₂ CH₂N(CH₂CH₂CH₂)₂, CH₂NHCH₂CH₂CH₂OCH₂, CH₂NHCH₂CH₂OCH₃, CH₂NHCH₂CH₂OCH₂CH₃, CH₂CH₂NHCH₂CH₂CH₂OCH₂CH₃, CH₂NHCH₂CH₂CH₂OCH₂CH₃, CH₂OCH₂CH₂NHCH₃, CH₂CH₂CH₂OCH₂CH₂CH₂NHCH₃, CH₂OCH₂N(CH₂)₂, CH₂N(CH₂CH₂OCH₂)₂, CH₂NHCH₂SCH₃, CH₂NHCH₂CH₂SCH₃, CH₂NHCH₂CH₂SCH₂CH₃, CH₂CH₂NHCH₂CH₂CH₂SCH₂CH₃, CH₂NHCH₂CH₂SCH₂CH₂CH₃, CH₂SCH₂CH₂NHCH₃, CH₂CH₂SCH₂CH₂CH₂CH₂NHCH₃, CH₃NHCH₂CH₂CH₂OCH₃

C₃₋₁₀ heteroalkenyl; The term C₃₋₁₀ heteroalkenyl as used herein pertains to a C₃₋₁₀ alkenyl group which chain is interrupted by 1 to 3 heteroatoms selected from O, S and N. In other words, a C₃₋₁₀ alkenyl chain, which chain includes 1 to 3 heteroatoms selected from O, S and N. Examples of C₃₋₁₀ heteroalkenyl groups include but are not limited to CH=CHOCH₃, CH₌CHOCH₂CH₃, CH₌CHOCH₂CH₂CH₃, CH₂OCH₌CH₂, CH₂OCH₂CH=CH₂, CH₂OCH₂CH=CHCH₃, CH₂CH₂OCH₂CH₌CHCH₂CH₃, CH₂OCH₂CH₂CH₂CH₌CH₂, CH=CHCH₂OCH₃, CH₂CH₂CH₌CHCH₂CH₂OCH₃, CH=CHSCH₃, CH₌CHSCH₂CH₃, CH₌CHSCH₂CH₂CH₃, CH₂SCH₌CH₂, CH₂SCH₂CH=CH₂, CH₂SCH₂CH=CHCH₃, CH₂CH₂SCH₂CH₌CHCH₂CH₃, CH₂SCH₂CH₂CH₂CH₌CH₂, CH=CHCH₂SCH₃, CH₂CH₂CH₌CHCH₂CH₂SCH₃, CH₌CHNHCH₃, CH₌CHNHCH₂CH₃, CH=CHNHCH₂CH₂CH₃, CH₂NHCH=CH₂, CH₂NHCH₂CH₌CH₂, CH₂NHCH₂CH₂CH=CH₂, CH₂CH₂NHCH₂CH₂CH₌CHCH₃, CH₂NHCH₂CH₂CH=CHCH₃, CH₂CH₌CHNHCH₃, CH₂CH₌CHCH₂CH₂CH₂NHCH₃, CH₌CHN(CH₂)₂, CH₂N(CH₌CH)₂ CH₂N(CH₂CH₌CH)₂, CH₂NHCH₂OCH₂CH=CH₂, CH₂OCH₂NHCH₂CH₂CH₌CHCH₃, CH₂NHCH₂OCH₂CH=CHCH₃, CH₂CH₌CHNHOCH₃, CH₂CH₌CHCH₂OCH₂CH₂NHCH₃

C₃₋₁₀ heteroalkynyl; The term C₃₋₁₀ heteroalkynyl as used herein pertains to a C₃₋₁₀ alkynyl group which chain is interrupted by 1 to 3 heteroatoms selected from O, S and N. In other words, a C₃₋₁₀ alkynyl chain, which chain includes 1 to 3 heteroatoms selected from O, S and N. Examples of C₃₋₁₀ heteroalkynyl groups include but are not limited to CH≡COCH₃, CH≡COCH₂CH₃, CH≡COCH₂CH₂CH₃, CH₂OC≡CH, CH₂OCH₂C≡CH, CH₂OCH₂C≡CCH₃, CH₂CH₂OCH₂C≡CCH₂CH₃, CH₂OCH₂CH₂CH₂C≡CH, CH≡CCH₂OCH₃, CH₂CH₂C≡CCH₂CH₂OCH₃, CH≡CSCH₃, CH≡CSCH₂CH₃, CH≡CSCH₂CH₂CH₃, CH₂SC≡CH, CH₂SCH₂C≡CH, CH₂SCH₂C≡CCH₃, CH₂CH₂SCH₂C≡CCH₂CH₃, CH₂SCH₂CH₂CH₂C≡CH, CH≡CCH₂SCH₃, CH₂CH₂C≡CCH₂CH₂SCH₃, CH≡CNHCH₃, CH≡CNHCH₂CH₃, CH≡CNHCH₂CH₂CH₃, CH₂NHC≡CH, CH₂NHCH₂C≡CH, CH₂NHCH₂CH₂C≡CH, CH₂CH₂NHCH₂CH₂C≡CCH₃, CH₂NHCH₂CH₂C≡CCH₃ CH₂C≡CNHCH₃, CH₂C≡CCH₂CH₂CH₂NHCH₃, CH≡CN(CH₂)₂, CH₂N(C≡CH)₂ CH₂N(CH₂C≡CH)₂.

Amino: -NR¹R², wherein R¹ and R² are independently amino substituents, for example, hydrogen, a C₁₋₆ hydrocarbon group (also referred to as C₁₋₆ alkylamino or C₁₋₆ dialkylamino), or, in the case of a "cyclic" amino group, R¹ and R², taken together with the nitrogen atom to which they are attached, form a heterocyclic ring having from 4 to 6 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to -NH₂, -NHCH₃, -NHC(CH₃)₂, -N(CH₃)₂, -N(CH₂CH₃)₂, N(C₁₋₆ alkyl)₂ and -NHPh. Examples of cyclic amino groups include, but are not limited to, aziridino, azetidino, pyrrolidino, piperidino, piperazino, morpholino, and thiomorpholino.

C₁₋₆ alkyl amido: The term C₁₋₆ alkyl amido (also referred to as carbamoyl, carbamyl, aminocarbonyl, carboxamide) has the structure -C(=O)NR¹R², wherein R¹ and R² are independently amino substituents, as defined for amino groups for example, hydrogen, a C₁₋₆ hydrocarbon group (also referred to as C₁₋₆ alkylamido or C₁₋₆ dialkylamido), or, in the case of a "cyclic" amido group, R¹ and R², taken together with the nitrogen atom to which they are attached, form a heterocyclic ring having from 4 to 6 ring atoms. Examples of amido groups include, but are not limited to, -C(=O)NH₂, -C(=O)N(C₁₋₆ alkyl)₂, C(=O)NH(C₁₋₆ alkyl), such as -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Carbonyl: a carbonyl functional group refers to a C=O bond such as the following structure R-C(=O)-R' wherein R and R' are independently carbonyl substituents which can be organic groups or H atoms for example a C₁₋₆hydrocarbon group, C₆₋₁₀ aryl, C₃₋₆ cycloalkyl, C₄₋₁₀ heterocyclyl, C₄₋₁₀ heterocyclyl or H. For example carbonyl groups include C(=O)-(C₁₋₄ alkyl), -C(=O)-(C₂₋₄ alkenyl), -C(=O)-(C₂₋₄ alkynyl), - C(=O)-(C₆₋₁₀ aryl), -C(=O)-(C₃₋₆ cycloalkyl), -C(=O)-(C₄₋₁₀ heterocyclyl), -C(=O)-( C₄₋₁₀ heterocyclyl), - C(=O)-(C₁₋₄ alkoxy), -C(=O)-(C₃₋₄ alkenoxy), -C(=O)-(C₃₋₄ alkynoxy), -C(=O)-(C₃₋₆ heteroalkyl), -C(=O)-(C₄₋₆ heteroalkenyl), or -C(=O)-(C₄₋₆ heteroalkynyl).

Carboxy (carboxylic acid): -C(=O)OH (CO₂H).

Ester: If not stated otherwise, the term "ester" in this document refers to a carboxylic ester functional group with the formula R-C(=O)-OR' wherein R and R' are independently substituents which are organic groups. For example a C₁₋₆ hydrocarbon group. R can also be hydrogen. For example an ester group can be -CO₂(C₁₋₆ alkyl).

C₁₋₆ alkyl ester: The term C₁₋₆ alkyl ester (carboxylate, carboxylic acid ester, oxycarbonyl) has the structure -C(=O)OR, wherein R is a C₁₋₆ hydrocarbon group. For example, CO₂(C₁₋₆ alkyl) or CO₂C₁₋₄ alkyl. Examples of ester groups include, but are not limited to, -C(=O)OCH₃, -C(=O)OCH₂CH₃ and -C(=O)OC(CH₃)₃.

Amide: an amide functional group which has the structure R-C(=O)N(R')(R") wherein R, R' and R" are independently amide substituents which can be organic groups or H atoms for example a C₁₋₆hydrocarbon group or H.

Aminocarbonyloxy: -OC(=O)NR¹R², wherein R' and R² are independently amino substituents, as defined for amino groups. Examples of aminocarbonyloxy groups include, but are not limited to, -OC(=O)NH₂,-OC(=O)NH-CH₃, -OC(=O)N(CH₃)₂, and -OC(=O)N(CH₂-CH₃)₂.

Phosphoric acid (phosphonooxy): -OP(=O)(OH)₂.

C₄₋₁₂ heterocyclyl: The term "C₄₋₁₂ heterocyclyl" as used herein, pertains to a monovalent moiety obtained by removing a hydrogen atom from a ring atom of a heterocyclic compound, which moiety has from 4 to 12 ring atoms, of which from 1 to 5 are ring heteroatoms. In certain embodiments, each ring has from 5 to 7 ring atoms, of which from 1 to 4 are ring heteroatoms. The ring may be saturated or unsaturated and may be bridged or unbridged. The ring may be a fused ring or a single ring. For the avoidance of doubt, substituents on the heterocyclyl ring may be linked via either a carbon atom or a heteroatom.

In this context, the prefixes (e.g. C₅₋₁₀ C₅₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrrolidine (C₅), pyrroline (e.g., 3-pyrroline, 2,5-dihydropyrrole) (C₅), 2H-pyrrole or 3H-pyrrole (C₅), piperidine (C₆), dihydropyridine (C₆), tetrahydropyridine (C₆), azepine (C₇), 1 ,2,3,4,-tetrahydroquinolone (C₁₀);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), pyrazole (C₅), piperazine (C₆), pyrimidine (C₆);
N₃: triazole (C₅)
N₄: tetrazole (C₅)
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂S₁: 6,7-dihydro-4H-thiazolo[5,4-c]pyridine-5-yl (C₉);
N₂O₁: oxadiazine (C₆);
O₁S₁: oxathiole (C₅) and oxathiane (thioxane) (C₆); and,
N₁O₁S₁: oxathiazine (C₆).

Examples of bicyclic heterocyclyl groups include, but are not limited to those derived from:

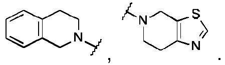

C₆₋₁₀ carboaryl: The term "C₆₋₁₀ carboaryl", as used herein, pertains to a monovalent moiety obtained by removing a hydrogen atom from an aromatic ring atom of an aromatic compound, which moiety has from 6 to 10 ring atoms and the ring atoms are all carbon atoms, as in "carboaryl groups". The ring may be a fused ring or a single ring. Examples of carboaryl groups include, but are not limited to, those derived from benzene (i.e. phenyl) (C₆), naphthalene (C₁₀) and azulene (C₁₀).

In this context, the prefixes (e.g. C₅₋₇, C₅₋₆, C₅₋₁₀, etc.) denote the number of ring atoms, or range of number of ring atoms. For example, the term "C₅₋₆ aryl" as used herein, pertains to an aryl group having 5 or 6 ring atoms.

Examples of carboaryl groups which comprise fused rings, at least one of which is an aromatic ring, include, but are not limited to, groups derived from indane (e.g. 2,3-dihydro-1H-indene) (C₉), indene (C₉), isoindene (C₉) and tetraline (1,2,3,4-tetrahydronaphthalene) (C₁₀). A tetralin has the following structure:

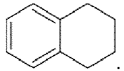

C₅₋₁₂ heteroaryl: The term "C₅₋₁₂ heteroaryl", as used herein, pertains to a monovalent moiety obtained by removing a hydrogen atom from an aromatic ring atom of an aromatic compound, which moiety has from 5 to 12 ring atoms of which from 1 to 5 are ring heteroatoms. In certain embodiments, each ring has from 5 to 7 ring atoms, of which from 1 to 4 are ring heteroatoms. For the avoidance of doubt, substituents on the heteroaryl ring may be linked via either a carbon atom or a heteroatom. The ring may be a fused ring or a single ring.

Examples of monocyclic heteroaryl groups include, but are not limited to, those derived from:
N₁: pyrrole (C₅), pyridine (C₆);
O₁: furan ( (C₅);
S₁: thiophene (C₅);
N₁O₁ : oxazole (C₅), isoxazole (C₅)
N₂O₁: oxadiazole, such as 1 ,2,5-oxadiazole (furazan) (C₅), ;
N₃O₁: oxatriazole (C₅);
N₁S₁: 1 ,3-thiazole (C₅), 1 ,2-thiazole (isothiazole) (C₅);
N₂: imidazole (C₅), pyrazole (C₅), pyridazine (1 ,2-diazine) (C₆), pyrimidine (1 ,3-diazine) (C₆) (e.g., cytosine, thymine, uracil), pyrazine (1 ,4-diazine) (C₆);
N₃: triazole (C₅), triazine (C₆); and,
N₄: tetrazole (C₅).

For the avoidance of doubt, where multiple substituents are independently selected from a given group, the selected substituents may comprise the same substituents or different substituents from within the given group.

### Linker

The PROTAC linker connects the two functional motifs of a PROTAC, a target protein binder and an E3 ligase recruiter. An optimal linker will allow for maximal interaction between the target protein and the E3 ligase resulting in efficient ubiquitination of the target protein and its ultimate degradation. The linker length can vary from 1-45 atoms in length. The length and chemical composition of the linker affects the structural rigidity, hydrophobicity and solubility of a PROTAC. The structure of the linker is described in more detail below.

### Pharmaceutically acceptable salt

The term "pharmaceutically acceptable" is used to specify that an object (for example a salt, dosage form or excipient) is suitable for use in patients. An example list of pharmaceutically acceptable salts can be found in the Handbook of Pharmaceutical Salts: Properties, Selection and Use*,* P. H. Stahl and C. G.

Wermuth, editors, Weinheim/Zürich: Wiley-VCH/VHCA, 2002. A suitable pharmaceutically acceptable salt of a compound of Formula (I-b) is, for example, an acid-addition salt. An acid addition salt of a compound of Formula (I-b) may be formed by bringing the compound into contact with a suitable inorganic or organic acid under conditions known to the skilled person. An acid addition salt may for example be formed using an inorganic acid selected from the group consisting of hydrochloric acid, hydrobromic acid, sulphuric acid and phosphoric acid. An acid addition salt may also be formed using an organic acid selected from the group consisting of trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid and para-toluenesulfonic acid.

Therefore, in one embodiment there is provided a compound of Formula (I), (I-1), (I-2), (I-2b), (I-2c) or (I2-d) or a pharmaceutically acceptable salt thereof, where the pharmaceutically acceptable salt is a hydrochloric acid, hydrobromic acid, sulphuric acid, phosphoric acid, trifluoroacetic acid, citric acid, maleic acid, oxalic acid, acetic acid, formic acid, benzoic acid, fumaric acid, succinic acid, tartaric acid, lactic acid, pyruvic acid, methanesulfonic acid, benzenesulfonic acid or para-toluenesulfonic acid salt. In one embodiment there is provided a compound of Formula (I), (I-1), (I-2), (I-2b), (I-2c) or (I2-d) or a pharmaceutically acceptable salt thereof, where the pharmaceutically acceptable salt is a methanesulfonic acid salt.

### Other forms

Compounds and salts described in this specification may exist in solvated forms and unsolvated forms. For example, a solvated form may be a hydrated form, such as a hemihydrate, a monohydrate, a dihydrate, a trihydrate or an alternative quantity thereof. The compounds of Formula (I-b) encompass all such solvated and unsolvated forms of compounds of Formula (I-b), particularly to the extent that such forms possess STING antagonist activity, as for example measured using the tests described herein.

Compounds and salts described in this specification include one or more chiral (i.e. asymmetric) centres. To the extent a structure or chemical name in this specification does not indicate the chirality, the structure or name is intended to encompass any single stereoisomer (i.e. any single chiral isomer) corresponding to that structure or name, as well as any mixture of stereoisomers (e.g. a racemate). In some embodiments, a single stereoisomer is obtained by isolating it from a mixture of isomers (e.g. a racemate) using, for example, chiral chromatographic separation. In other embodiments, a single stereoisomer is obtained through direct synthesis from, for example, a chiral starting material.

A particular enantiomer of a compound described herein may be more active than other enantiomers of the same compound.

According to one embodiment there is provided a compound of Formula (I-b), or a pharmaceutically acceptable salt thereof, which is a single enantiomer being in an enantiomeric excess (%ee) of ≥ 95, ≥ 98% or ≥ 99%. Conveniently, the single enantiomer is present in an enantiomeric excess (%ee) of ≥ 99%.

According to another embodiment there is provided a pharmaceutical composition, which comprises a compound of Formula (I-b), which is a single enantiomer being in an enantiomeric excess (%ee) of ≥ 95, ≥ 98% or ≥ 99% or a pharmaceutically acceptable salt thereof, in association with one or more pharmaceutically acceptable excipients. Conveniently, the single enantiomer is present in an enantiomeric excess (%ee) of ≥ 99%.

### Isotopes

Atoms of the compounds and salts described in this specification may exist as their isotopes. The compound of Formula (I-1) encompasses all compounds of Formula (I-1) where an atom is replaced by one or more of its isotopes (for example a compound of Formula (I-1) where one or more carbon atom is an ¹¹C or ¹³C carbon isotope, or where one or more hydrogen atoms is a ²H or ³H isotope).

### Tautomers

Compounds and salts described in this specification may exist as a mixture of tautomers. "Tautomers" are structural isomers that exist in equilibrium resulting from the migration of a hydrogen atom. The compound of Formula (I-1) includes all tautomers of compounds of Formula (I-1) particularly to the extent that such tautomers possess STING antagonist activity.

For example, the tautomeric form of compound (I-1), when R⁸ and R¹⁸ are hydrogen, can be shown as Formula (I-a1):

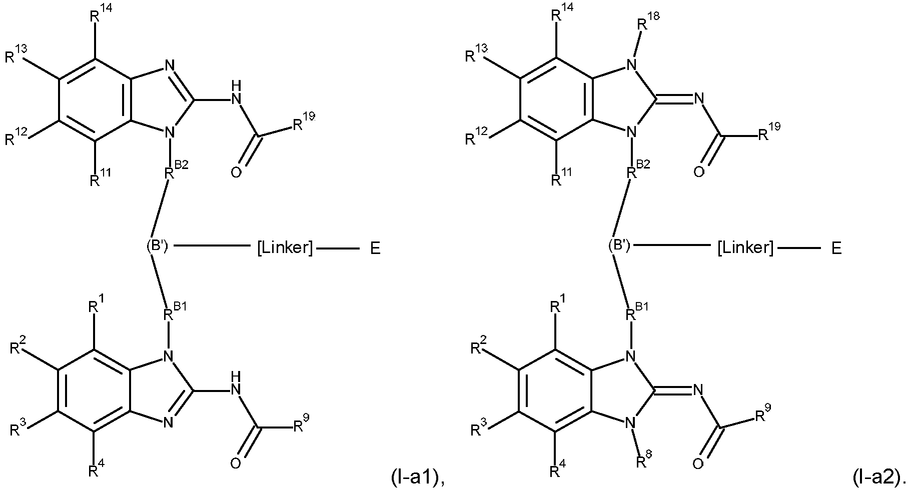

The tautomeric form of compound (I-a2), when R⁸ and R¹⁸ are hydrogen, can be shown as (I-a3):

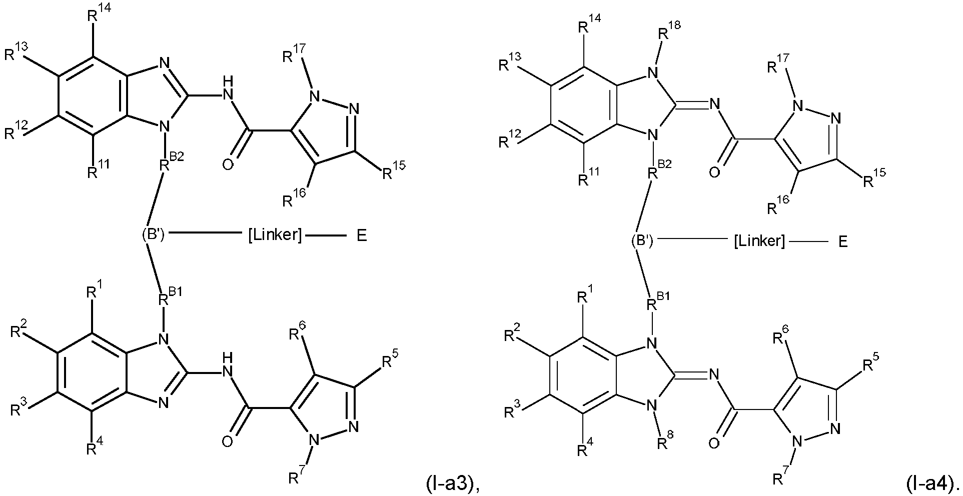

Where R⁸ and R¹⁸ are H the tautomeric forms (I-a) and (I-a1) might form.

### Crystalline forms

Compounds and salts described in this specification may be crystalline and may exhibit one or more crystalline forms. The compound of Formula (I) encompasses any crystalline or amorphous form of a compound of Formula (I), or mixture of such forms, which possesses STING antagonist activity.

It is generally known that crystalline materials may be characterised using conventional techniques such as X-Ray Powder Diffraction (XRPD), Differential Scanning Calorimetry (DSC), Thermal Gravimetric Analysis (TGA), Diffuse Reflectance Infrared Fourier Transform (DRIFT) spectroscopy, Near Infrared (NIR) spectroscopy, solution and/or solid state nuclear magnetic resonance spectroscopy. The water content of crystalline materials may be determined by Karl Fischer analysis.

### Therapy, prophylaxis and related terms

The term "therapy" is intended to have its normal meaning of dealing with a disease in order to entirely or partially relieve one, some or all of its symptoms, or to correct or compensate for the underlying pathology. The term "therapy" also includes "prophylaxis" unless there are specific indications to the contrary. The terms "therapeutic" and "therapeutically" should be interpreted in a corresponding manner.

The term "prophylaxis" is intended to have its normal meaning and includes primary prophylaxis to prevent the development of the disease and secondary prophylaxis whereby the disease has already developed and the patient is temporarily or permanently protected against exacerbation or worsening of the disease or the development of new symptoms associated with the disease.

The term "treatment" is used synonymously with "therapy". Similarly the term "treat" can be regarded as "applying therapy" where "therapy" is as defined herein.

The term "subject" to which administration is contemplated includes, but is not limited to, humans (i.e., a male or female of any age group, e.g., a paediatric subject (e.g., infant, child, adolescent) or adult subject (e.g., young adult, middle-aged adult or senior adult)) and/or other primates (e.g., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. Preferred subjects are humans.

An "effective amount", as used herein, refers to an amount that is sufficient to achieve a desired biological effect. A "therapeutically effective amount", as used herein refers to an amount that is sufficient to achieve a desired therapeutic effect. For example, a therapeutically effective amount can refer to an amount that is sufficient to improve at least one sign or symptom of the disease to be treated.

### Pharmaceutical compositions

The compounds of Formula (I), and pharmaceutically acceptable salts thereof, may be administered as pharmaceutical compositions, comprising one or more pharmaceutically acceptable excipients.

Therefore, in one embodiment there is provided a pharmaceutical composition comprising a compound of Formula (I), or a pharmaceutically acceptable salt thereof, and a pharmaceutically acceptable excipient.

The excipient(s) selected for inclusion in a particular composition will depend on factors such as the mode of administration and the form of the composition provided. Suitable pharmaceutically acceptable excipients are well known to persons skilled in the art and are described, for example, in the Handbook of Pharmaceutical Excipients, Sixth edition, Pharmaceutical Press, edited by Rowe, Ray C; Sheskey, Paul J; Quinn, Marian. Pharmaceutically acceptable excipients may function as, for example, adjuvants, diluents, carriers, stabilisers, flavourings, colorants, fillers, binders, disintegrants, lubricants, glidants, thickening agents and coating agents. As persons skilled in the art will appreciate, certain pharmaceutically acceptable excipients may serve more than one function and may serve alternative functions depending on how much of the excipient is present in the composition and what other excipients are present in the composition.

The pharmaceutical compositions may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous or intramuscular dosing), or as a suppository for rectal dosing. The compositions may be obtained by conventional procedures well known in the art. Compositions intended for oral use may contain additional components, for example, one or more colouring, sweetening, flavouring and/or preservative agents.

Suitable daily doses of the compounds disclosed herein, or a pharmaceutically acceptable salt thereof, in therapeutic treatment of humans are about 0.0001-100 mg/kg body weight.

Pharmaceutical formulations as described herein may be formulated by methods known to those skilled in the art to provide doses of the active compound in the range of 0.1 mg to 1000 mg. The daily dose will necessarily be varied depending upon the host treated, the particular route of administration, any therapies being co-administered, and the severity of the illness being treated. Accordingly, the practitioner who is treating any particular patient may determine the optimum dosage.

The pharmaceutical compositions described herein comprise compounds of Formula (I), or a pharmaceutically acceptable salt thereof, and are therefore expected to be useful in therapy.

As such, in one embodiment there is provided a pharmaceutical composition for use in therapy, comprising a compound of Formula (I), or a pharmaceutically acceptable salt thereof, and at least one pharmaceutically acceptable excipient.

In one embodiment there is provided a pharmaceutical composition for use in the treatment of a disease associated with overactivation of STING, comprising a compound of Formula (I-1), or a pharmaceutically acceptable salt thereof, and at least one pharmaceutically acceptable excipient. In one embodiment there is provided a pharmaceutical composition for use in the treatment of a disease, comprising a compound of Formula (I), or a pharmaceutically acceptable salt thereof, and at least one pharmaceutically acceptable excipient. In one embodiment there is provided a pharmaceutical composition for use in the treatment of a disease associated with overactivation of STING, comprising a compound of Formula (I), or a pharmaceutically acceptable salt thereof, and at least one pharmaceutically acceptable excipient.

### Methods of Use

The compounds described herein may be used in a method of therapy. Also provided is a method of treatment, comprising administering to a subject in need of treatment a therapeutically effective amount of a compound of Formula (I). The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound may be administered alone or in combination with other treatments, either simultaneously or sequentially dependent upon the condition to be treated.

In one embodiment there is provided a compound of Formula (I), or a pharmaceutically acceptable salt thereof, or a pharmaceutical composition comprising the compound of Formula (I) for use in therapy. In one embodiment there is provided the use of the compound of Formula (I), or a pharmaceutically acceptable salt thereof, or a pharmaceutical composition comprising the compound of Formula (I) for the manufacture of a medicament. In another embodiment, there is provided a compound of Formula (I), or a pharmaceutically acceptable salt thereof, for use as a medicament. In another embodiment, there is provided a use of the compound of Formula (I), or a pharmaceutically acceptable salt thereof, in the manufacture of a medicament for reducing lymphocyte count in a subject in need thereof. In another embodiment there is provided a method of treatment comprising administering to a subject the compound of Formula (I), or a pharmaceutically acceptable salt thereof, or a pharmaceutical composition comprising the compound of Formula (I).

In one embodiment there is provided a compound of Formula (I), or a pharmaceutically acceptable salt thereof, or a pharmaceutical composition comprising the compound of Formula (I) for use in treating a disorder. In some embodiments there is provided a method of treating a disorder or condition comprising administering to a subject the compound of Formula (I), or a pharmaceutically acceptable salt thereof, or a pharmaceutical composition comprising the compound of Formula (I).

In one embodiment there is provided a compound of Formula (I), or a pharmaceutically acceptable salt thereof, or a pharmaceutical composition comprising the compound of Formula (I) for use in reducing the lymphocyte count in a subject. In another embodiment, there is provided a method of reducing the lymphocyte count in a subject, comprising administering to a subject the compound of Formula (I), or a pharmaceutically acceptable salt thereof, or a pharmaceutical composition comprising the compound of Formula (I). In some embodiments, the subject has received an organ or tissue transplant. In some embodiments, the subject is scheduled to receive an organ or tissue transplant. The transplant may be a solid organ transplant (SOT), e.g. a kidney transplant, a liver transplant, a pancreas transplant or a heart transplant. In some embodiments, the subject has received a transplant and is suffering from graft versus host disease (GvHD). In some embodiments, the subject has received a transplant and is at risk or graft versus host disease GvHD.

In some embodiments, the disorder or condition is associated with overactivation of STING. In some embodiments, the disorder or condition is driven by constitutive STING activation. In some embodiments, the disorder or condition is driven by constitutive STING activation caused by a gain-of-function mutation.

In some embodiments, the disorder or condition is a monogenic autoinflammatory syndrome, autoimmune disease, inflammatory disease, neurological disorder, metabolic disease, cardiovascular disease, or cancer.

In some embodiments, the disorder or condition is a monogenic autoinflammatory syndrome. In some embodiments, the disorder or condition is STING-associated vasculopathy with onset in infancy (SAVI), Aicardi-Goutieres Syndrome, COPA Syndrome, Familial Chilblain Lupus, Dnase II deficiency, or TREX1 deficiency.

In some embodiments, the disorder or condition is an autoimmune disease. In some embodiments, the disorder or condition is systemic lupus erythematosus (SLE), Sjögren's syndrome, or rheumatoid arthritis.

In some embodiments, the disorder or condition is an inflammatory disease. In some embodiments, the disorder or condition is silica-induced fibrosis or sepsis.

In some embodiments, the disorder or condition is a neurological disorder. In some embodiments, the disorder or condition is ischaemic brain injury, Parkinson disease, general neurodegeneration, Huntington disease, amyotrophic lateral sclerosis and frontotemporal dementia, age-dependent macular degeneration, or traumatic brain injury.

In some embodiments, the disorder or condition is a metabolic disease. In some embodiments, the disease is non-alcoholic steatohepatitis, alcoholic liver disease, or acute pancreatitis.

In some embodiments, the disorder or condition is a cardiovascular disease. In some embodiments, the disorder or condition is myocardial infarction or chronic heart failure.

In some embodiments, the disorder or condition is cancer. In some embodiments, the disorder or condition is colorectal cancer, skin cancer, or metastases.

In some embodiments the disorder or condition is selected from monogenic autoinflammatory syndrome, STING-associated vasculopathy with onset in infancy (SAVI), Aicardi-Goutieres Syndrome, COPA Syndrome, Familial Chilblain Lupus, Dnase II deficiency, or TREX1 deficiency, an autoimmune disease, systemic lupus erythematosus (SLE), Sjögren's syndrome or a cardiovascular disease.

In some embodiments, the disorder or condition is senescence or aging. In some embodiments, the subject is a mammal. In some embodiments, the subject is a human.

In a further aspect, the invention provides a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, for use as a medicament.

In a further aspect, the invention provides a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, for the manufacture of a medicament for the treatment of a disease or condition.

In a further aspect, the invention provides a method of treatment of a disease or condition in a subject comprising administering a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently.

In a further aspect, the invention provides a use of a compound in the manufacture of a medicament for the treatment of a disease or condition, wherein the bifunctional compound is suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently.

In a further aspect, the invention provides a pharmaceutical composition comprising a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand binds to STING non-covalently, and a pharmaceutically acceptable excipient.

In a further aspect, the invention provides a method of inhibiting signaling driven by constitutive activation of STING, the method comprising administration of a therapeutically effective amount of a compound suitable for the degradation of Stimulator of Interferon Genes (STING), wherein the chimeric compound has a formula S1-L-E, wherein S1 is a STING ligand; L is a linker; E is a E3 ubiquitin ligase ligand; and wherein the STING ligand does not form a covalent bond with STING.

In some embodiments, upon non-covalent binding of S1 to STING and E to an E3 ubiquitin ligase, STING is degraded.

### Further embodiments /preferences

The following embodiments may apply to all aspects as described above or may relate to a single aspect. The embodiments may be combined together in any combination.

### S1

S1 is a STING ligand. The STING ligand does not form a covalent bond with STING. The STING ligand does not exhibit STING agonist activity. Exhibiting STING agonist activity means inducing cytokine induction or inducing phospho-STING. Therefore, the STING ligand S1 does not induce cytokine induction nor induce phospho-STING. Any standard method known in the art may be used to determine STING agonist activity. For example, STING agonist activity (or lack thereof) can be measured as described in Examples 11 and/or Example 12. For example, following the method described in Example 11, cells may be incubated with a compound comprising a S1 STING ligand. The cells may be lysed and Western Blotting performed. Western blots may be probed for phospho-STING and/or phospho-TBK1. Cells incubated with STING ligands which are not agonists will not result in detectable phospho-STING or phospho-TBK1 using the methods outlined above and in Examples 11. In another example, following the method described in Example 12, human skin fibroblasts may be pretreated for 2hours with a serial dilution of a test compounds, for example a compound comprising an S1 ligand, prior to stimulation with a STING agonist (e.g. 300 nM GSK STING agonist). Supernatants may then be harvested after STING agonist stimulation (e.g. after 20 h stimulation). IFN-a/b may be measured in the supernatants using HEK-Blue IFN-α/β bioassay (Invivogen) to determine a half-maximal inhibitory concentration of a test compound. A compound comprising an S1 ligand having an IC50 value against STING indicates that the compound is not a STING agonist.

### STING degradation

STING degradation may be quantified using any standard method in the art. For example, STING degradation may be measured using Western Blot analysis as described in Example 11 followed by quantification of protein band signal intensity (e.g. densiometric analysis). Quantification of may be performed using any commonly available software, for example Image Lab software (Biorad, Version 6.0.1). In some embodiments, incubating cells with 1 µM S1-L-E compound for 16 hours reduces relative STING protein abundance by at least 30%, at least 35%, at least 40%, at least 45%, 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with S1-L-E compound. In some embodiments, incubating cells with 5 µM S1-L-E compound for 16 hours reduces relative STING protein abundance by at least 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with S1-L-E compound. In some embodiments, incubating cells with 7.5 µM S1-L-E compound for 16 hours reduces relative STING protein abundance by at least 55%, at least 60%, at least 65%, at least 70%, at least 75%, 80%, at least 85%, at least 90%, at least 95%, at least 96%, at least 97%, at least 98%, at least 99% as compared to cells not treated with S1-L-E compound. In some embodiments, incubating cells with 10 µM S1-L-E compound for 16 hours reduces relative STING protein abundance by at least 80%, at least 85%, at least 90%, at least 95%, at least 96%, at least 97%, at least 98%, at least 99% as compared to cells not treated with S1-L-E compound. In some embodiments, the cells incubated with the S1-L-E compounds are selected from the following cell lines: H1650 cells, H596 cells, HaCat cells, HFF-1 cells, RAW264.7 cells, primary fibroblasts, primary SAVI patient fibroblasts, primary human skin fibroblasts, and THP-1 cells.

### R⁹ and R¹⁹

In some embodiments R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

In some embodiments, R⁹ and R¹⁹ are each independently selected from an optionally substituted C₅₋₆ heteroaryl or optionally substituted C₅₋₆ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

In further embodiments R⁹ and R¹⁹ are each independently selected from an optionally substituted C₅₋₆ heteroaryl or optionally substituted C₅₋₆ heterocyclyl, wherein the optional substituents are independently selected from H, halogen, C₃₋₅ cycloalkyl, optionally substituted C₁₋₄ alkyl wherein said optionally substituted alkyl is optionally substituted by one or more substituents independently selected from halogen, halo(C₁₋₄ alkyl), OH, NH₂, C₁₋₄ alkoxy, CO₂H, CO₂C₁₋₆ alkyl, N(C₁₋₆ alkyl)₂, OCONH₂ and CONH₂.

When R⁹ or R¹⁹ is an optionally substituted C₅₋₆ heteroaryl or optionally substituted C₅₋₆ heterocyclyl the optional substituent is selected from optionally substituted methyl, optionally substituted ethyl, optionally substituted propyl wherein the optional substituents are OH, NH₂, halogen or C₁₋₄ alkoxy. In further embodiments when R⁹ or R¹⁹ is an optionally substituted C₅₋₆ heteroaryl or optionally substituted C₅₋₆ heterocyclyl the optional substituent is selected from methyl and ethyl.

In some embodiments formula (I) is of formula (I-2): 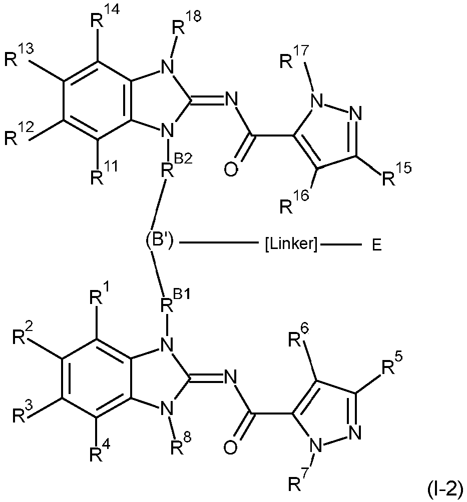
wherein R¹, R², R³, R⁴, R⁸, R¹¹, R¹², R¹³, R¹⁴, R¹⁸, R^{B1}, R^{B2}, B', Linker and E are as defined herein, wherein R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy;
R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy; and
R⁵ and R¹⁵ are each independently selected from H, cyclopropyl and C₁₋₄ alkyl.

### R¹ and R¹¹

In some embodiments R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -OP(O)(OH)₂, C₁₋₄ alkoxy, NH₂, NH(C₁₋₆ alkyl), N(C₁₋₆ alkyl)₂, CO₂(C₁₋₆ alkyl) and halogen.

When R¹ or R¹¹ are optionally substituted C₁₋₆ alkyl, the optional substituents are selected from OH, F, Br, methoxy and ethoxy. In some embodiments when R¹ or R¹¹ are optionally substituted C₁₋₆ alkyl, they are optionally substituted methyl or optionally substituted ethyl. In further embodiments, they are optionally substituted methyl. In further embodiments, they are unsubstituted methyl.

When R¹ or R¹¹ are halo, in some embodiments they are F, Br or Cl.

In some embodiments R¹ and R¹¹ are independently selected from H, OH, F and methyl.

In some embodiments R¹ is identical to R¹¹.

In some embodiments R¹ and R¹¹ are H.

### R² and R¹²

In some embodiments R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -OP(O)(OH)₂, C₁₋₄ alkoxy, N(C₁₋₆ alkyl)₂, NH₂, NH(C₁₋₆ alkyl), CO₂(C₁₋₆ alkyl) and halogen.

When R² or R¹² are optionally substituted C₁₋₆ alkyl, the optional substituents are selected from OH, F, Br, methoxy and ethoxy. In some embodiments when R² or R¹² are optionally substituted C₁₋₆ alkyl, they are optionally substituted methyl or optionally substituted ethyl. In further embodiments, they are optionally substituted methyl. In further embodiments, they are unsubstituted methyl.

When R² or R¹² are halo, in some embodiments they are F, Br or Cl.

In some embodiments R² and R¹² are independently selected from H, OH, F or methyl.

In some embodiments R² is identical to R¹².

In some embodiments R² and R¹² are H.

### R³ and R¹³

In some embodiments R³ and R¹³ are each independently selected from H, C(=O)NH₂; optionally substituted -C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen.

When R³ or R¹³ are optionally substituted -C(=O)NH(C₁₋₆ alkyl), optionally substituted C₁₋₆ alkoxy or optionally substituted CO₂C₁₋₆ alkyl, the optional substituents are selected from OH, -O-P(O)(OH)₂, methoxy, ethoxy, N(CH₃)₂, NH(CH₃), NH₂, CO₂(CH₃) and halogen.

When R³ or R¹³ are -C(=O)NH(C₁₋₆ alkyl) in some embodiments they are each independently-C(=O)NHCH₃, -C(=O)NHCH₂CH₃, -C(=O)NHCH₂CH₂CH₃.

In some embodiments R³ and R¹³ are each independently selected from H, -C(=O)NH₂ -C(=O)NHCH₃,-C(=O)NHCH₂CH₃, or -C(=O)NHCH₂CH₂CH₃.

In some embodiments R³ and R¹³ are each independently selected from -C(=O)NH(C₁₋₃alkyl) and-C(=O)NH₂.

In some embodiments R³ is identical to R¹³.

In some embodiments R³ and R¹³ are -C(=O)NH₂.

### R⁴ and R¹⁴

In some embodiments R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen.

When R⁴ or R¹⁴ are optionally substituted C₁₋₆ alkyl, the optional substituents are selected from OH, F, Br, methoxy and ethoxy. In some embodiments when R⁴ or R¹⁴ are optionally substituted C₁₋₆ alkyl, they are optionally substituted methyl or optionally substituted ethyl. In further embodiments, they are optionally substituted methyl. In further embodiments they are unsubstituted methyl.

When R⁴ or R¹⁴ are halo, in some embodiments they are F, Br or Cl.

In some embodiments R⁴ and R¹⁴ are each independently selected from H, OH, F or methyl.

In some embodiments R⁴ is identical to R¹⁴.

In some embodiments R⁴ and R¹⁴ are H.

### R⁵ and R¹⁵

In some embodiments R⁵ and R¹⁵ are each independently selected from H, cyclopropyl and C₁₋₄ alkyl and C₁₋₄ alkoxy.

In some embodiments, R⁵ and R¹⁵ are C₁₋₄ alkyl or C₁₋₄ alkoxy

In some embodiments R⁵ and R¹⁵ are H.

In some embodiments R⁵ and R¹⁵ are cyclopropyl.

When R⁵ or R¹⁵ are each independently C₁₋₄ alkyl they are methyl, ethyl, propyl or butyl. In some embodiments they are methyl or ethyl. In further embodiments they are methyl.

In some embodiments R⁵ is identical to R¹⁵.

In some embodiments R⁵ and R¹⁵ are methyl.

### R⁶ and R¹⁶

In some embodiments R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy.

When R⁶ or R¹⁶ are optionally substituted C₁₋₆ alkyl, the optional substituents are selected from OH, F, Br, methoxy and ethoxy. In some embodiments when R⁶ or R¹⁶ are optionally substituted C₁₋₆ alkyl, they are optionally substituted methyl or optionally substituted ethyl. In further embodiments, they are optionally substituted methyl. In further embodiments, they are unsubstituted methyl.

In some embodiments, R⁶ and R¹⁶ are each independently selected from H, OCH₃, halogen or C₁₋₃ alkyl.

When R⁶ or R¹⁶ are halogen, in some embodiments they are F, Br or Cl.

In some embodiments R⁶ and R¹⁶ are independently selected from H, OCH₃, F or methyl.

In some embodiments R⁶ is identical to R¹⁶.

In some embodiments R⁶ and R¹⁶ are H.

### R⁷ and R¹⁷

In some embodiments R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy.

When R⁷ or R¹⁷ are each independently optionally substituted C₁₋₄ alkyl it is an optionally substituted methyl, ethyl, propyl or butyl. When R⁷ or R¹⁷ are each independently selected from optionally substituted C₁₋₄ alkyl the optional substituents are selected from OH, halogen, methoxy and ethoxy. When R⁷ and R¹⁷ are each independently optionally substituted C₁₋₄ alkyl, in some embodiments they are methyl or ethyl.

In some embodiments R⁷ is identical to R¹⁷.

In some embodiments R⁷ and R¹⁷ are C₁₋₄ alkyl.

In some embodiments R⁷ and R¹⁷ are ethyl.

### R⁸ and R¹⁸

In some embodiments R⁸ and R¹⁸ are each independently selected from H, and C₁₋₄ alkyl.

In some embodiments when R⁸ and R¹⁸ are C₁₋₄ alkyl they are selected from methyl, ethyl, propyl or butyl.

In some embodiments R⁸ and R¹⁸ are methyl, ethyl or H. In further embodiments R⁸ and R¹⁸ are methyl or H.

In some embodiments R⁸ is identical R¹⁸.

In some embodiments R⁸ and R¹⁸ are H.

In some embodiments R⁸ and R¹⁸ are methyl.

In some embodiments R⁸ and R¹⁸ are ethyl.

### R^{B1} and R^{B2}

In some embodiments R^{B1} and R^{B2} are each independently selected from (-CH₂-)₁₋₂ or a bond.

When R^{B1} or R^{B2} are a bond, this is a bond connected directly from the N to B'.

When R^{B1} or R^{B2} are (-CH₂-)₁₋₂ they can each independently be -CH₂- or -CH₂CH₂-.

In some embodiments R^{B1} is a bond and R^{B2} is -CH₂- or -CH₂CH₂-. In some embodiments R^{B1} is a bond and R^{B2} is -CH₂- or -CH₂CH₂-. In other embodiments R^{B2} is a bond and R^{B1} is -CH₂-. In other embodiments R^{B1} is a bond and R^{B2} is -CH₂-.

In some embodiments R^{B1} and R^{B2} are both a bond.

In some embodiments R^{B1} and R^{B2} are both -CH₂- or both -CH₂-CH₂- or R^{B1} is -CH₂- and R^{B2} is -CH₂-CH₂-

In some embodiments R^{B1} is identical to R^{B2}.

In some embodiments R^{B1} and R^{B2} are both -CH₂-.

In some embodiments R^{B1} and R^{B2} are both -CH₂CH₂-.

### B'

B' is the point of attachment to the Linker group L as follows:

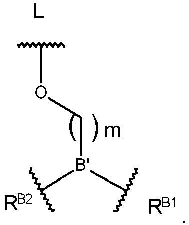

In some embodiments, R^{B1} and R^{B2} are each independently selected from (-CH₂-)₁₋₂ or a bond and B' taken together with R^{B1} and R^{B2} forms an alkyl linking group having 3-7 atoms in shortest length; and
B' is the point of attachment to the Linker group L as follows: 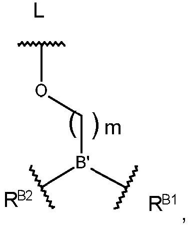
wherein B' is CH or C₁₋₅ alkyl;
wherein m is 0 or 1.

In some embodiments B' is CH, methyl, ethyl, propyl, butyl or pentyl. In further embodiments B is CH, methyl or ethyl.

In further embodiments B is CH or C₁₋₂ alkyl and B' together with R^{B1} and R^{B2} form a C₃₋₈ alkyl chain.

In further embodiments B is CH or C₁₋₂ alkyl and B' together with R^{B1} and R^{B2} form a C₅ alkyl chain.

In some embodiments m is 0.

In some embodiments m is 1.

In some embodiments m is 0 and therefore B' is bonded directly to O and B' is CH or C₁₋₂ alkyl.

In further embodiments m is 0 and therefore B' is bonded directly to O and B' together with R^{B1} and R^{B2} form a C₃₋₈ alkyl chain. In further embodiments m is 0 and therefore B' is bonded directly to O and B' together with R^{B1} and R^{B2} form a C₅ alkyl chain.

In some embodiments B' is of the formula: 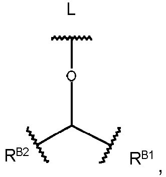
wherein R^{B1} and R^{B2} are each independently selected from (-CH₂-)_{1-2;};
wherein the wavy lines indicate the point of attachment to the Linker (L), R^{B1} and R^{B2} as shown.

In further embodiments R^{B1} and R^{B2} are each -CH₂-CH₂- and together with B' have the following structure: 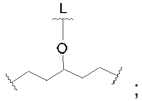 wherein the wavy line next to L represents the attachment to the linker and the wavy lines next to the alkyl groups represent the connection to the N atoms in the S1 structure.

### Symmetrical S1 compounds

In some embodiments R¹ is identical to R¹¹, R² is identical to R¹², R³ is identical to R¹³, R⁴ is identical to R¹⁴, R^{B1} is identical to R^{B2}, R⁸ is identical to R¹⁸ and R⁹ is identical to R¹⁹. In further embodiments R¹ is identical to R¹¹, R² is identical to R¹², R³ is identical to R¹³, R⁴ is identical to R¹⁴, R⁵ is identical to R¹⁵, R^{B1} is identical to R^{B2}, R⁸ is identical to R¹⁸, R⁶ is identical to R¹⁶ and R⁷ is identical to R¹⁷. Therefore, in some embodiments the compounds are symmetrical.

### Further embodiments

In some embodiments, R⁴, R¹⁴, R², R¹², R¹ and R¹¹ are each independently selected from H, OH, OCH₃, halogen or C₁₋₃ alkyl.

In some embodiments, R⁷, R⁸, R¹⁷ and R¹⁸ are C₁₋₄alkyl.

In some embodiments,
R¹ and R¹¹ are selected from H, -OH or C₁₋₃ alkoxy;
R³ and R¹³ are selected from -C(=O)N(H)(C₁₋₃alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

In further embodiments
R¹ and R¹¹ are H;
R³ and R¹³ are -C(=O)NH₂ or -C(=O)NH-CH₃;
R⁷ and R¹⁷ are ethyl; and
R⁵ and R¹⁵ are methyl.

In some embodiments
R¹, R¹¹, R⁶, R¹⁶, R⁴, R¹⁴, R² and R¹² are H;
R⁷ and R¹⁷ are ethyl;
R⁵, R¹⁵, R⁸ and R¹⁸ are methyl;
R³ and R¹³ are -C(=O)NH₂;
R^{B1} and R^{B2} are both -CH₂-;
b is 0;
a is 1;
X is -NR^{B3}R^{B4} or X is -OR^{B5};
R^{B5} is ethyl;
R^{B3} and R^{B4} are selected from H, ethyl, phenyl, pyrimidine, CH₂CH₂-O-CH₃, pyrazole substituted by methyl,
or R^{B3} and R^{B4} together form a group selected from a morpholine which is optionally substituted by phenyl, a 1,2,3,4-tetrahydroquinoline substituted with methoxy, a pyrrolidine substituted with a tetrazole or phenyl which phenyl is substituted with methoxy , or a 2-[(1,2,3,4-tetrahydro-2-isoquinolyl)carbonyl]pyrrolidine substituted with methoxy or a piperazine substituted with C(=O)CH₃.

In some embodiments compound (I) is of the Formula (I-2b): 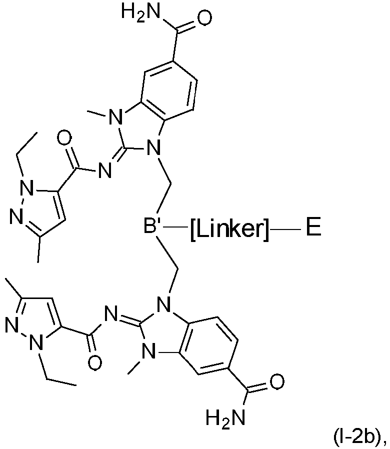 or a tautomer, or a pharmaceutically acceptable salt thereof wherein B' is a C₁₋₅ alkyl and is the connection point to the Linker.

In further embodiments compound of formula (I) is of the formula (I-2c) or (I-2d):

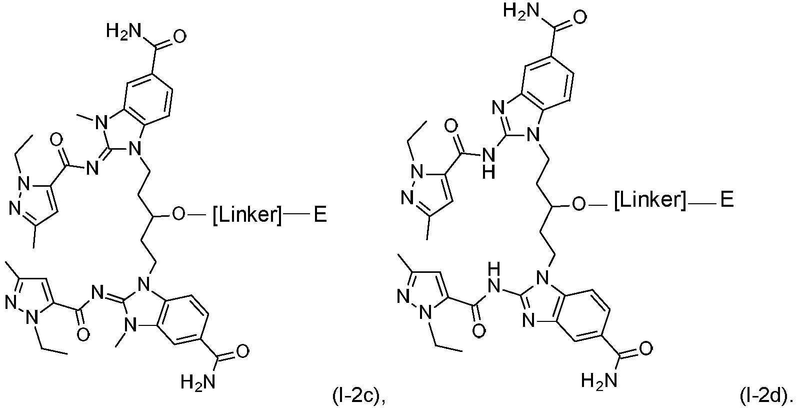

### E

In some embodiments the E3 ubiquitin ligase ligand is a Cereblon (CRBN) E3 ubiquitin ligase ligand. In some embodiments the E3 ubiquitin ligase ligand is a von Hippel-Lindau (VHL) E3 ubiquitin ligase ligand. In some embodiments the E3 ubiquitin ligase ligand is an Inhibitor of Apoptosis (IAP) E3 ubiquitin ligase ligand.

### E3

In some embodiments E has a structure according to Formula E3 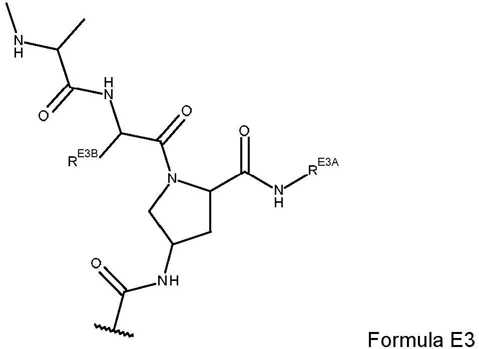 wherein
R^{E3A} is optionally substituted C₅₋₁₀ cycloalkyl or optionally substituted C₆₋₁₀ carboaryl, wherein the optional substituents are selected from C₁₋₆ alkyl, halogen and OH;
R^{E3B} is H, C₁₋₆ alkyl, or C₃₋₇ cycloalkyl; and 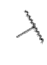
represents attachment point to L.

In some embodiments when R^{E3A} is optionally substituted C₅₋₁₀ cycloalkyl, optionally substituted C₆₋₁₀ carboaryl the optional substituents are selected from methyl, ethyl, F, Cl and OH.

When R^{E3A} is an optionally substituted C₅₋₁₀ cycloalkyl it is selected from cyclopentyl, cyclohexyl, cycloheptyl, methylcyclopropyl, dimethylcyclopropyl, methylcyclobutyl, dimethylcyclobutyl, methylcyclopentyl, dimethylcyclopentyl, methylcyclohexyl, cyclooctyl, cyclononayl, cyclodecyl, decalinyl and 2,3,3a,4,5,6,7,7a-octahydro-1H-indenyl.

When R^{E3A} is an optionally substituted C₆₋₁₀ carboaryl in some embodiments it is a single ring selected from phenyl, naphthyl and azulyl.

When R^{E3A} is an optionally substituted C₆₋₁₀ carboaryl in some embodiments it is fused rings, at least one of which is an aromatic ring, selected from indanyl (e.g. 2,3-dihydro-1H-indene), indenyl, isoindenyl (C₉) and tetralin-1-yl (e.g. 1,2,3,4-tetrahydronaphthalene).

In some embodiments, unsubstituted tetralin, optionally wherein R^{E3A} is (*1R*)-1,2,3,4-tetrahydronaphthalen-1-yl.

In some embodiments R^{E3A} is an unsubstituted tetralin-1-yl which has the following structure:

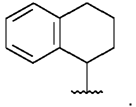

When R^{E3B} is C₃₋₇cycloalkyl it is selected from cyclopropyl, cyclobutyl, cyclopentyl, cyclohexyl, cycloheptyl, methylcyclopropyl, dimethylcyclopropyl, methylcyclobutyl, dimethylcyclobutyl, methylcyclopentyl, dimethylcyclopentyl methylcyclohexyl. In some embodiments R^{E3B} is cyclopentyl, cyclohexyl or cycloheptyl.

In some embodiments, R^{E3B} is C₃₋₇ cycloalkyl.

In some embodiments R^{E3B} is cyclohexyl.

In some embodiments R^{E3B} is cyclohexyl and R^{E3A} is an unsubstituted tetralin-1-yl.

In some embodiments E has the structure E3-1: 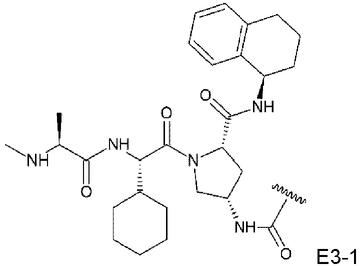 wherein 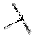 represents attachment point to L.

### E4

In some embodiments E has a structure according to Formula E4 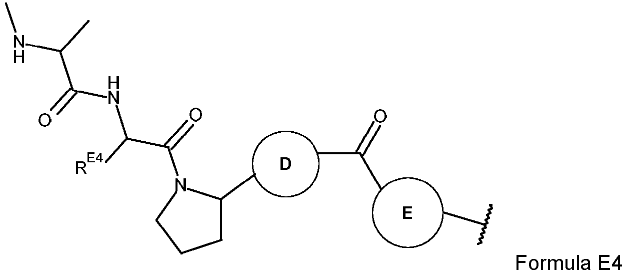 Wherein
R^{E4} is H, C₁₋₆ alkyl, or C₃₋₇ cycloalkyl;
Ring D is C₅₋₇ heterocyclyl or C₅₋₇ heteroaryl;
Ring E is C₆₋₁₀ carboaryl, or C₅₋₁₀ heteroaryl; and 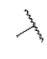
represents attachment point to L.

When R^{E4} is C₃₋₇ cycloalkyl in some embodiments it is selected from cyclopropyl, cyclobutyl, cyclopentyl, cyclohexyl, cycloheptyl, methylcyclopropyl, dimethylcyclopropyl, methylcyclobutyl, dimethylcyclobutyl, methylcyclopentyl, dimethylcyclopentyl methylcyclohexyl. In some embodiments R^{E4} is cyclopentyl, cyclohexyl or cycloheptyl.

In some embodiments R^{E4} is a C₃₋₇ cycloalkyl.

In some embodiments R^{E4} is a C₅₋₆ cycloalkyl.

In some embodiments R^{E4} is cyclohexyl.

In some embodiments Ring D is a C₅₋₇ heteroaryl.

When Ring D is a C₅₋₇ heteroaryl in some embodiments it contains 1 to 4 heteroatoms selected from O, N and S. In some embodiments it is a 5 or 6 membered heteroaryl with 1 or 2 heteroatoms. In some embodiments it is a 5 membered heteroaryl with 2 heteroatoms. In some embodiments it is a 5 membered heteroaryl with 2 heteroatoms one being N and the other being S.

In some embodiments Ring D is an N containing C₅₋₇ heteroaryl group. In some embodiments Ring D is a C₅₋₇ heteroaryl group containing one N atom and one S atom.

When Ring D is a C₅₋₇ heteroaryl in some embodiments it is a pyrrolyl, pyridyl, furanyl, thiophenyl, oxazolyl, isoxazolyl, isoxainyl, oxadiazolyl, oxatirazolyl, thiazolyl, isothiazolyl, imidazolyl, pyrazolyl, pyridazinyl, pyrimidinyl, pyrazinyl, triazolyl, triazinyl or tetrazolyl.

In some embodiments Ring D is a thiazolyl. In some embodiments Ring D is a 1,3-thiazole linked in position 2 to the pyrrolidine ring and in position 4 to C=O.

In some embodiments Ring D is

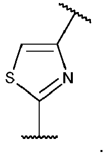

In some embodiments Ring E is a C₆ heteroaryl containing 1 heteroatom selected from O, N or S.

In some embodiments Ring E is C₆₋₁₀carboaryl.

When Ring E is C₆₋₁₀ carboaryl in some embodiments it is phenyl.

In some embodiments Ring E is

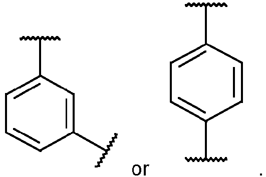

In some embodiments R^{E4} is C₅₋₆ cycloalkyl, Ring D is a C₅₋₇ heteroaryl group containing one S and one N and Ring E is phenyl.

In some embodiments E has the following structure E4-1: 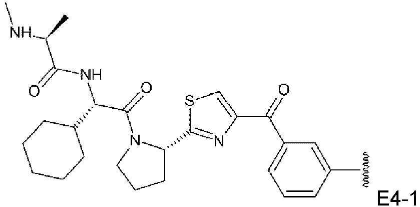 wherein 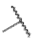 represents attachment point to L.

In some embodiments E is selected from one of the following structures:

| **E** | **Structure** | **E** | **Structure** |
|---|---|---|---|
| E3-1 | 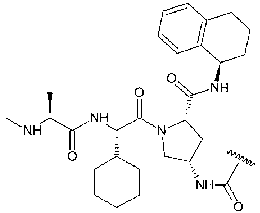 | E4-1 | 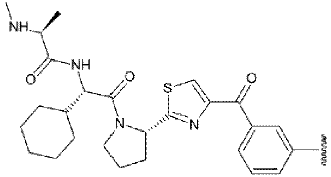 |

### Linker (L)

L is a linker. L is a linker which connects S1 to E.

In some embodiments, L is a saturated or a partially or fully unsaturated framework comprising C and H atoms and at least one heteroatom, wherein said framework has a shortest length of 10-40 atoms;
wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-COR^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₃ alkyl and cyclopropyl, and wherein the number of optional substituents is below 10;
wherein said Linker is attached via its point of attachment to an available C, N, O, or S atom at E and the O atom of S1

In some embodiments L is a saturated or a partially or fully unsaturated framework comprising C and H atoms and at least one heteroatom, wherein said framework has a shortest length of 10-40 atoms;
wherein said framework may include one or more straight and/or branched chains and/or rings and is optionally substituted on any available C atom(s) by one or more =O, OH, C₁₋₃ alkyl, F, C₁₋₃ alkoxy, NR^{L}₂, wherein each R^{L} is selected from H, C₁₋₆ alkyl;
wherein said Linker is attached via its point of attachment to an available C, N, O, or S atom at E or the O atom of S1.

In some embodiments L connects the STING ligand to the E3 ubiquitin ligase ligand via a chain of atoms having 10-40 atoms in shortest length. In further embodiments L connects the STING ligand to the E3 ubiquitin ligase ligand via a chain of atoms having 15-36 atoms in shortest length.

In some embodiments, L is a saturated or a partially or fully unsaturated framework comprising C and H atoms, and at least one heteroatom wherein said framework may include one or more of the groups selected from -O-, -C(=O)-, -N(R^{L1})-, -N(R^{L1})-C(=O)-, -C(=O)-N(R^{L1})-, -C₁₋₆alkyl-N(R^{L1}), -N(R^{L1})-C(=O)-C₁₋₆alkyl, - C₁₋₆alkyl-C(=O)-N(R^{L1})-, -C(=O)-N(R^{L1})-C₁₋₆alkyl-, -C₁₋₆alkyl-N(R^{L})-C(=O)-, and/or heterocyclic rings or heteroaryl rings,
wherein R^{L1} is selected from H, C₁₋₆alkyl.

In some embodiments L has the following structure:

*-L_{A}-[X]_{X}-L_{B}-*

wherein
L_{A} and L_{B} are each independently selected from a bond, *-C₁₋₆alkyl-, *-O-C₁₋₆alkyl-, *-C₃₋₇cycloalkyl, *-C₄₋₇heterocyclyoalkyl-, *-C₃₋₇cycloalkyl-C₃₋₇cycloalkyl-, *-C₄₋₇heterocyclyoalkyl-C₄₋₇heterocyclyoalkyl-, *-C₁₋₆alkyl-O-, *-O- , *-N(R^{L1})-, *-N(R^{L1})-C(=O)-, *-C(=O)-N(R^{L1})-, *-O-CH₂-C(=O)-N(R^{L1})-, *-N(R^{L1})-C(=O)-CH₂-O- *-N(R^{L1})-C₁₋₆alkyl-, *-C₁₋₆alkyl-N(R^{L1})-, *-N(R^{L1})-C(=O)-C₁₋₆alkyl-, *-C₁₋₆alkyl-C(=O)-N(R^{L1})-, *-C(=O)-, `-C(=O)-C₁₋₆alkyl-,*-C(=O)-N(R^{L1})-C₁₋₆alkyl-, *-C₁₋₆alkyl-N(R^{L1})-C(=O)-, and a functional group selected from carbonyl, ester, amide, carbamate and thiourea;
wherein R^{L1} is selected from H, C₁₋₆alkyl;
X is selected from: a bivalent, saturated or unsaturated, straight or branched, C₁₋₄₅ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, -N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, -S(=O)₂N(R^{L})-, - S(=O)₂N(H)-, C₅₋₇ carbocyclyl, a C₅₋₇ heterocyclyl, a C₆₋₁₀ carboaryl, or a C₅₋₁₀ heteroaryl group;
wherein R^{L} is a C₁₋₆alkyl group; and
* denotes the attachment to S1 or E.

In some embodiments X is selected from: a bivalent, saturated or unsaturated, straight or branched, C₆₋₃₆ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, - N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, - S(=O)₂N(R^{L})-, -S(=O)₂NH), C₅₋₇ carbocyclyl, a C₅₋₇ heterocyclyl, a C₆ carboaryl, or a C₅₋₇ heteroaryl group, 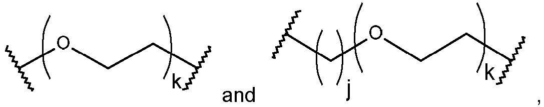 wherein k and j are each individually integers from 1 to 8;
wherein R^{L} is a C₁₋₆alkyl group.

In further embodiments X contains one or more -N(H)-C(=O)- or -C(=O)-N(H)- groups and 1 to 10 of the following group:

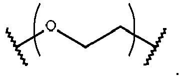

In some embodiments L has the structure:

E-(L7)ᵢ-(L6)ₕ-(L5)_{g}-(OC₂H₄)_{f}-(L4)ₑ-(L3)_{d}-(L2)_{c}-(OC₂H₄)_{b}-(NR^{X}C(=O))(L1)ₐ-S1

wherein:
L1 is C₁₋₆ alkyl;
L2 is C₁₋₆ alkyl;
L3 is - N(methyl)-C(=O)-, -NH-C(=O)-, -C(=O)NH- or C(=O)N(methyl)-;
L4 is -C(=O)-C₅₋₇ heterocyclyl-C(=O)- or -C(=O)-C₅₋₇ heteroaryl-C(=O)-;
L5 is C₁₋₁₂ alkyl wherein one or more -CH₂- groups is optionally replaced by one or more groups selected from -O-, -NH-, -NMe-, -C(=O)NH, -C(=O)NMe, --NHC(=O)-, NMeC(=O)- group;
L6 is C₁₋₆ alkyl optionally substituted by a =O group;
L7 is -NH-, -NMe-, C₃₋₆cycloalkyl, or C₄₋₆heterocyclyl;
R^{X} is H or methyl.
a is 0 or 1;
b is 0 to 8;
c is 0 or 1;
d is 0 or 1;
e is 0 or 1;
f is 0 to 8;
g is 0 or 1;
h is 0 or 1;
i is 0 to 2;
E is the ubiquitin ligase as defined in any of the preceding claims; and
S1 is S1 as defined in any of the preceding claims.

In some embodiments when L1 is C₁₋₆ alkyl it is methyl, ethyl or propyl. In some embodiments L1 is methyl.

In some embodiments a is 0. In some embodiments a is 1.

In some embodiments L1 is methyl and a is 1.

In some embodiments b is 0 to 6. In some embodiments b is 1 to 5. In some embodiments b is 2 to 5. In some embodiments b is 2, 3, 4 or 5.

In some embodiments when L2 is C₁₋₆ alkyl it is methyl, ethyl or propyl. In some embodiments L1 is methyl or ethyl. In some embodiments L2 is ethyl.

In some embodiments c is 0. In some embodiments c is 1.

In some embodiments L2 is ethyl and c is 1.

In some embodiments L3 is NHC(=O) or N(methyl)C(=O).

In some embodiments d is 0. In some embodiments d is 1.

In some embodiments L3 is NHC(=O) or N(methyl)C(=O) and d is 1.

In some embodiments e is 0.

In some embodiments e is 1.

In some embodiments when e is 1 L4 is -C(=O)-C₆ heterocyclyl-C(=O)- wherein the C₆ heterocyclyl contains 1 or 2 heteroatoms selected from O, N or S. In some embodiments the C₆ heterocyclyl is selected from piperidine, piperazine or morpholine.

In some embodiments when e is 1 L4 is -C(=O)-C₆ heteroaryl-C(=O)- wherein the C₆ heteroaryl contains 1 or 2 heteroatoms selected from O, N or S. In some embodiments the C₆ heteroaryl is selected from pyridine, pyrimidine or pyrazine.

In some embodiments when e is 1 L4 is:

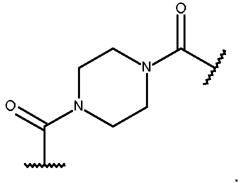

In some embodiments f is 0 to 5. In some embodiments f is 0. In some embodiments f is 1. In some embodiments f is 2. In some embodiments f is 3. In some embodiments f is 4. In some embodiments f is 5.

In some embodiments when g is 1, L5 is a C₄₋₁₀ alkyl optionally interrupted by one or more -O- groups and a C(=O)NH- group. In some embodiments when g is 1, L5 is a C₄₋₈ alkyl optionally interrupted by one or two -O- groups and a C(=O)NH- group. In some embodiments L5 is a C₆ alkyl interrupted by one -O-group and one C(=O)NH- group. In some embodiments L5 is a C₈ alkyl interrupted by two -O- groups and a C(=O)NH- group.

In some embodiments, L5 is -O-C₄H₈-NHC(=O)-C₂H₄- or -O-C₄H₈-NH-C(=O)-C₂H₄-O-C₂H₄-. In some embodiments L5 is -O-C₄H₈-NHC(=O)-C₂H₄- or -O-C₄H₈-NH-C(=O)-C₂H₄-O-C₂H₄- and g is 1.

In some embodiments g is 0.

In some embodiments h is 0.

In some embodiments when h is 1 L6 is a C₁₋₄ alkyl optionally substituted by =O.

In some embodiments L6 is methyl, ethyl, butyl or propyl substituted by =O and h is 1.

In some embodiments when h is 1 L6 is methyl. In some embodiments when h is 1 L6 is ethyl, In some embodiments when h is 1 L6 is propyl optionally substituted by =O. In some embodiments when h is 1 L6 is butyl.

In some embodiments L7 is C₃₋₆heterocyclyl. In some embodiments L7 is piperidinyl or piperazinyl.

In some embodiments L7 is -NH- or C₃₋₆heterocyclyl, and i is 1 or 2.

In some embodiments, L7 is -NH-, and i is 1.

In some embodiments i is 0. In some embodiments i is 1. In some embodiments i is 2.

In some embodiments linker L is selected from one of the following structures:

| **L** | **Structure** |
|---|---|
| LK1 | 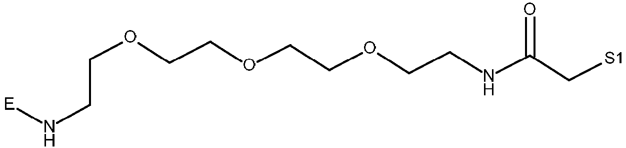 |
| LK2 | 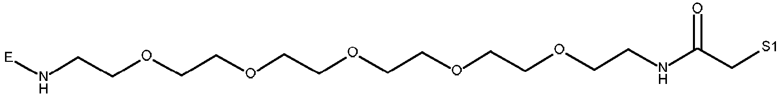 |
| LK3 | 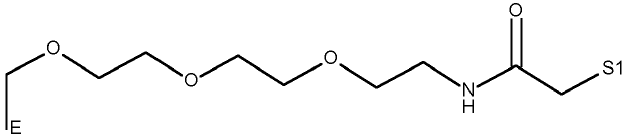 |
| LK4 | 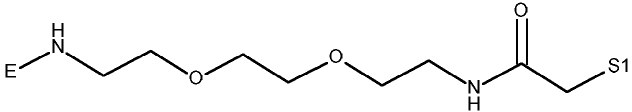 |
| LK5 | 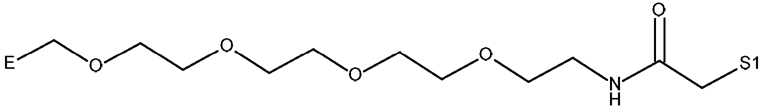 |
| LK6 | 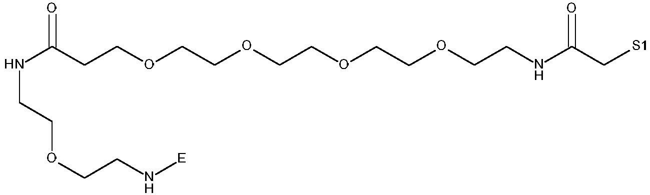 |
| LK7 | 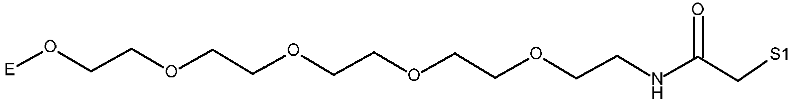 |
| LK8 | 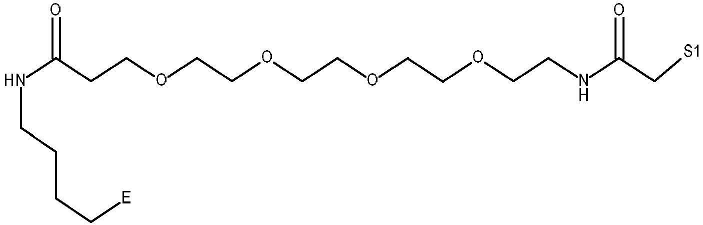 |
| LK9 | 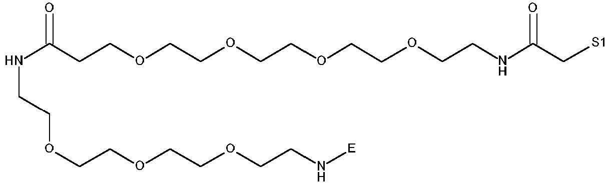 |
| LK10 | 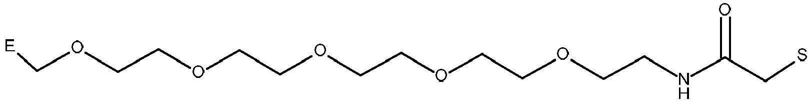 |
| LK11 | 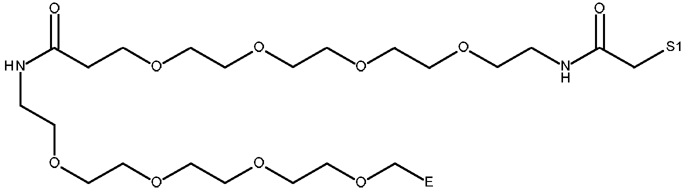 |
| LK12 | 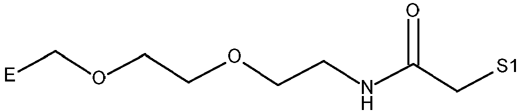 |
| LK13 | 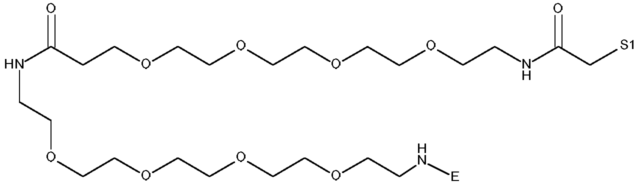 |
| LK14 | 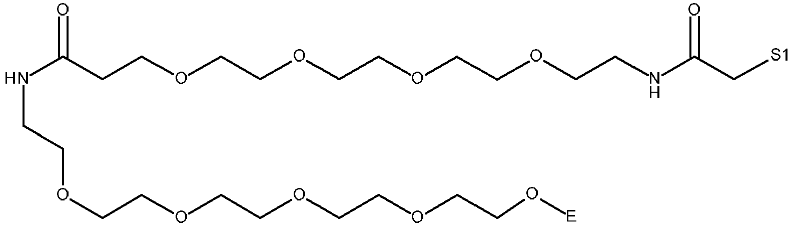 |
| LK15 | 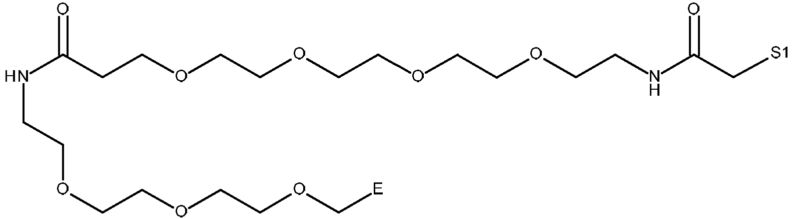 |
| LK16 | 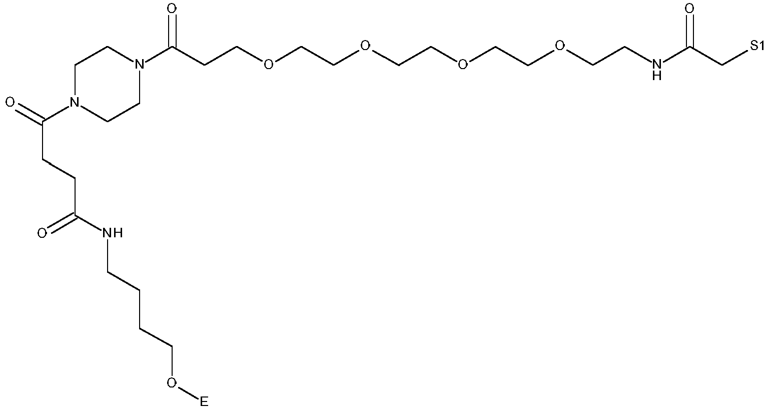 |
| LK17 | 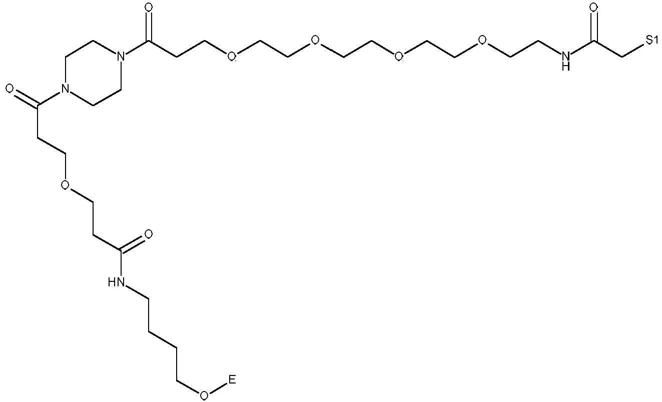 |
| LK18 | 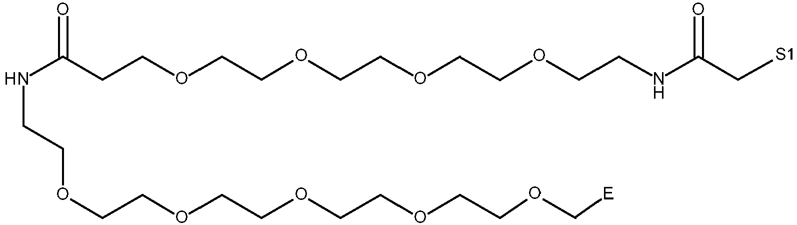 |
| LK19 | 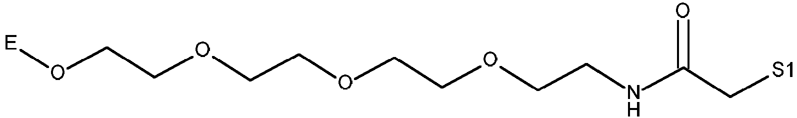 |
| LK20 | 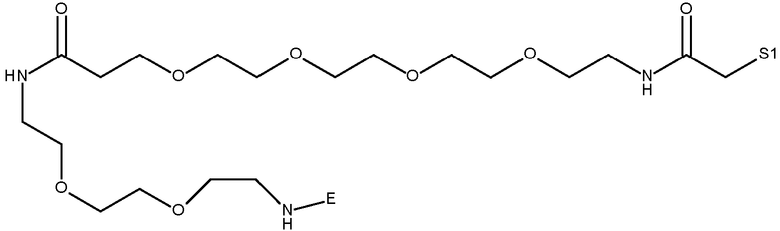 |
| LK21 | 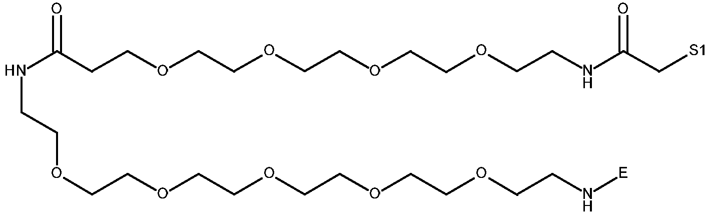 |
| LK22 | 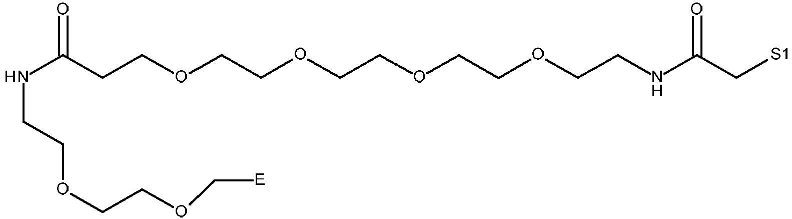 |
| LK23 | 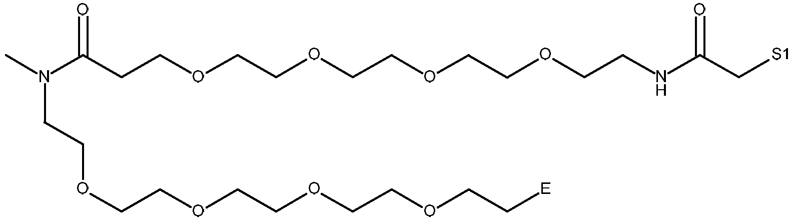 |
| LK24 | 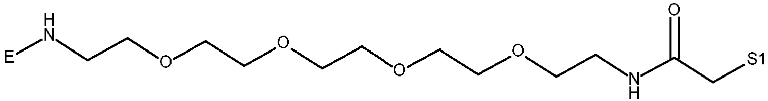 |
| LK25 | 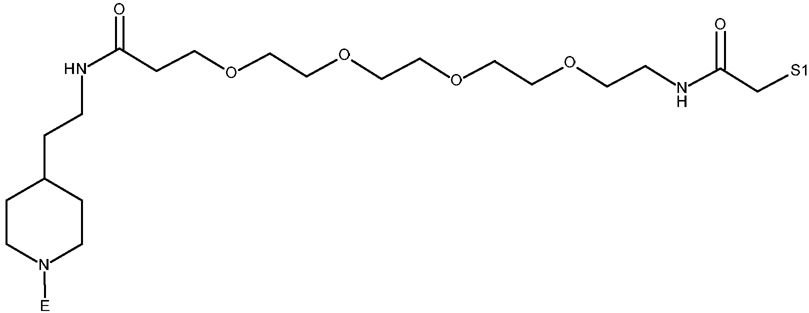 |
| LK26 | 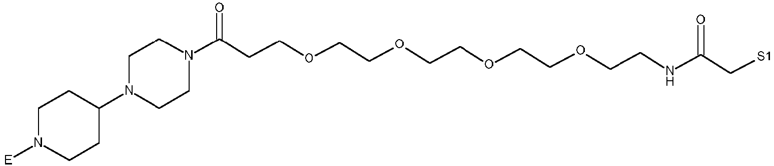 |

### S1-L-E

In some embodiments S1-L-E is selected from one of the following formulae: 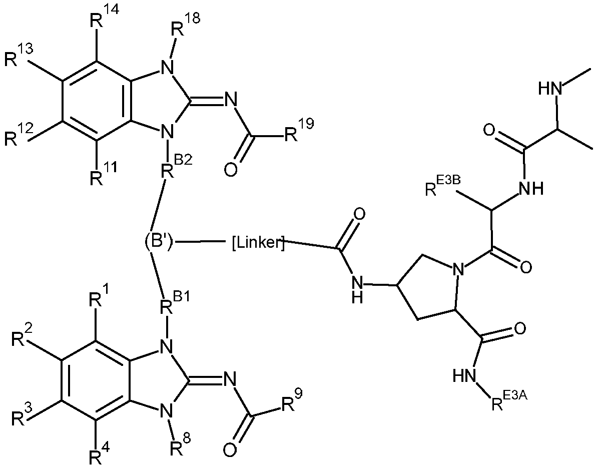 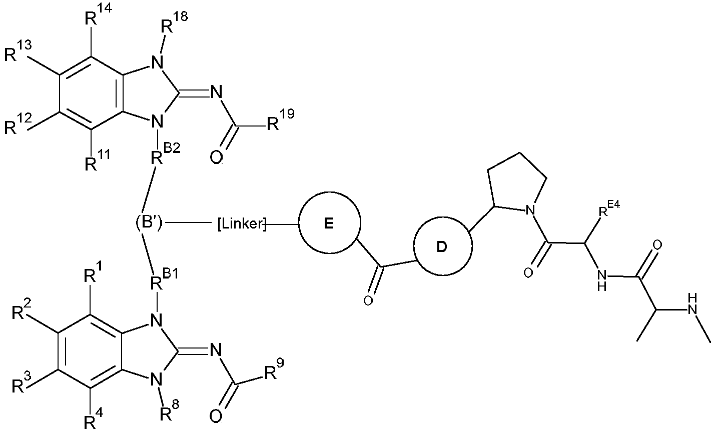 or tautomers thereof, wherein the substituents R¹, R², R³, R⁴, R⁸, R⁹, R^{B1}, B', Linker, R^{B2}, R¹¹, R¹², R¹³, R¹⁴, R¹⁸, R¹⁹, Rings D, E, R^{E3A}, R^{E3B}, and R^{E4} are as defined above.

In some embodiments, R⁹ is: 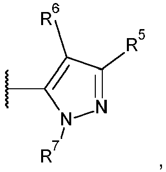 and R¹⁹ is:

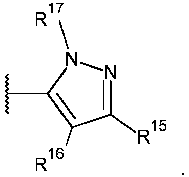

In some embodiments, R⁹ and R¹⁹are each:

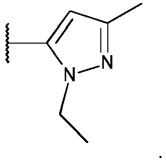

In some embodiments S1-L-E is selected from one of the following formulae:

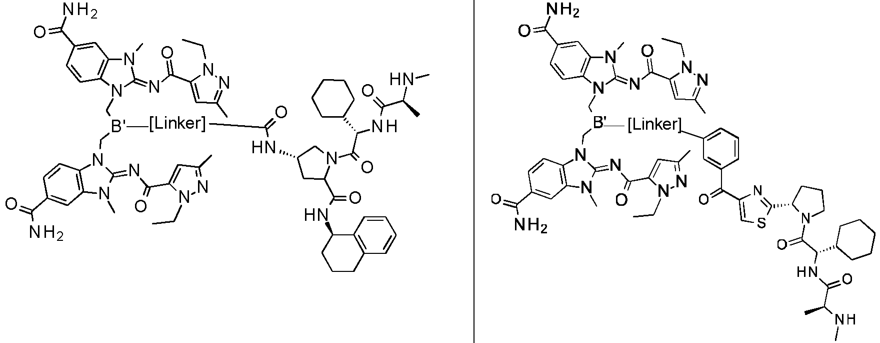

In some embodiments the compound is selected from Example compounds 1-5 or Example compounds 8-14 in Table 1.

**Table 1**

| **Example compound number** | **Structure** | **IUPAC Name** |
|---|---|---|
| 1 | 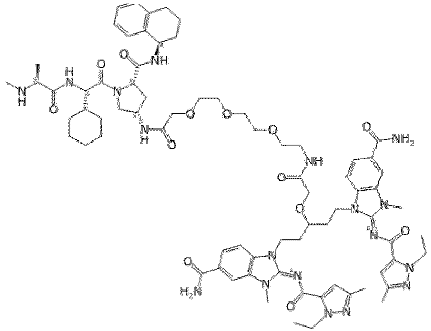 | N-[(3S,5S)-5-[NI)-1,2,3,4-tetrahydro-1-naphthylcarbamoyl]-1-{(S)-2-[(S)-2-(methylamino)propionylamino]-2-cyclohexylacetyl}-3-pyrrolidinyl]11-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9-trioxaundecanamide |
| 2 | 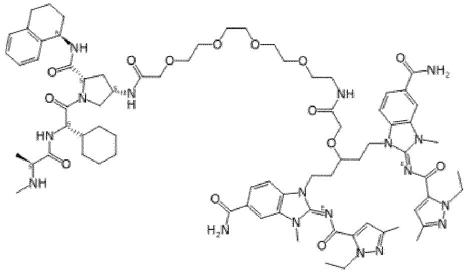 | N-[(3S,5S)-5I-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9,12-tetraoxatetradecanamide |
| 3 | 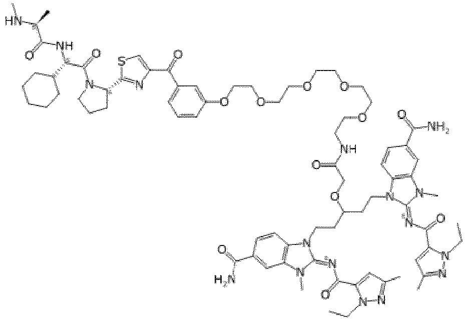 | 1-{3-[(N-2-{2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethoxy}ethylcarbam oyl)methoxy]-5-{5-carbamoyl-2-[(1-ethyl-3-methyl-5-pyrazolyl)carbonylimino]-3-methyl-2,3-dihydro-1H-1,3-benzimidazol-1-yl}pentyl}-2-[(1-ethyl-3-methyl-5-pyrazolyl)carbonylimino]-3-methyl-2,3-dihydro-1H-1,3-benzimidazole-5-carboxamide |
| 4 | 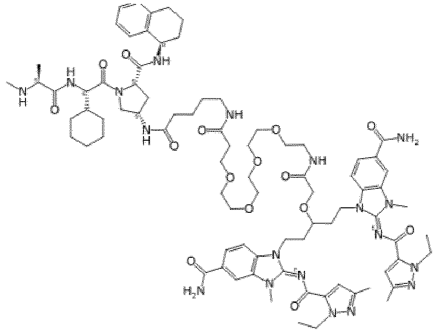 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]5-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9,12-tetraoxatetradecylcarbonylamin o)valeramide |
| 5 | 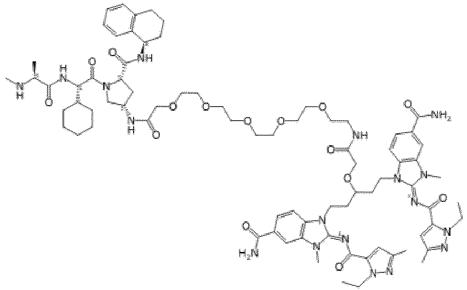 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]17-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9, 12, 15-pentaoxaheptadecanamide |
| 6 | 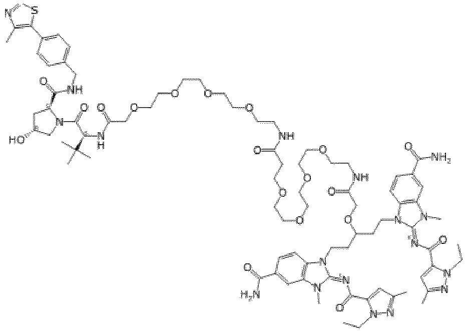 | N-[(S)-1-{[(2S,4R)-4-hydroxy-2-({[p-(4-methyl-1,3-thiazol-5-yl)phenyl]methyl}carbamoyl)-1-pyrrolidinyl]carbonyl}-2,2-dimethylpropyl] 14-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9,12-tetraoxatetradecylcarbonylamin o)-3,6,9,12-tetraoxatetradecanamide |
| 7 | 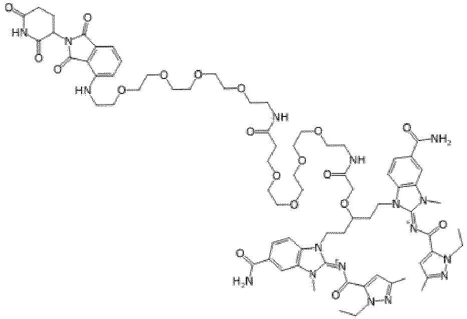 | N-(2-{2-[2-(2-{2-[2-(2,6-dioxo-3-piperidyl)-1,3-dioxo-4-isoindolinylamino]ethoxy}ethox y)ethoxy]ethoxy}ethyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-4,7,10,13-tetraoxapentadecanamide |
| 8 | 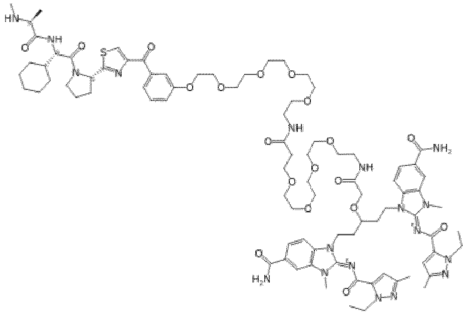 | N-(2-{2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethoxy}et-hyl)15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-4,7,10,13-tetraoxapentadecanamide |
| 9 | 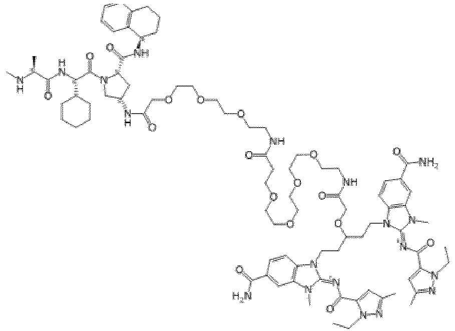 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]11-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-3,6,9,12-tetraoxatetradecylcarbonylamin o)-3,6,9-trioxaundecanamide |
| 10 | 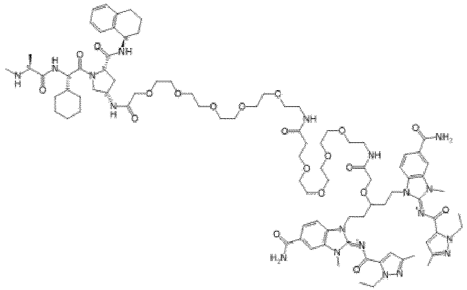 | N-{16-[N-(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinylcarbamoyl]-3,6,9,12,15-pentaoxahexadecyl}15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl]carbon ylamino}-4,7,10,13-tetraoxapentadecanamide |
| 11 | 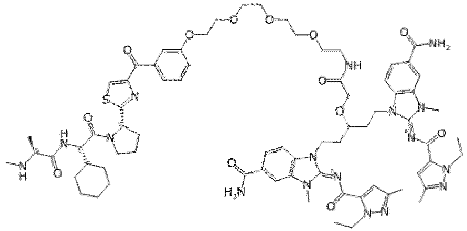 | 1-[3-({N-2-[2-(2-{2-[m-({2-[(S)-1-{(S)-2-[(S)-2-(methylamino)propionylamino]-2-cyclohexylacetyl}-2-pyrrolidinyl]-1,3-thiazol-4-yl}carbonyl)phenoxy]ethoxy}eth oxy)ethoxy]ethylcarbamoyl}met hoxy)-5-{5-carbamoyl-2-[(1 - ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| 12 | 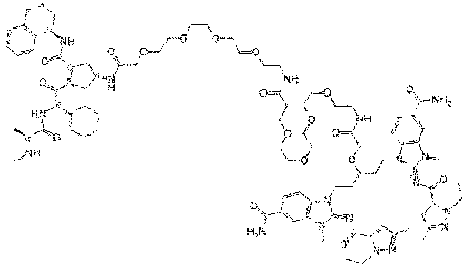 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]14-(14-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-3,6,9,12-tetraoxatetradecylcarbonylamin o)-3,6,9,12-tetraoxatetradecanamide |
| 13 | 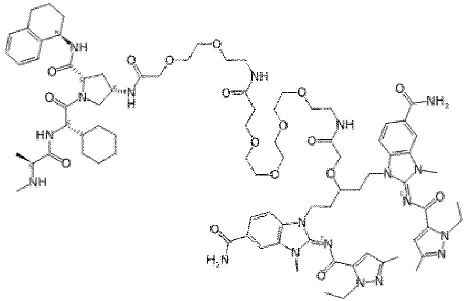 | N-[2-(2-{[N-(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinylcarbamoyl] methoxy} ethoxy)ethyl]15-{[(3-{5-carbamoyl-2-[(1-ethyl-3-methyl-5-pyrazolyl)carbonylimino]-3-methyl-2,3-dihydro-1H-1,3-benzimidazol-1-yl}-1-(2-{5-carbamoyl-2-[(1-ethyl-3-methyl-5-pyrazolyl)carbonylimino]-3-methyl-2,3-dihydro-1H-1,3-benzimidazol-1-yl}ethyl)propoxy)methyl] carbon ylamino}-4,7,10,13-tetraoxapentadecanamide |
| 14 | 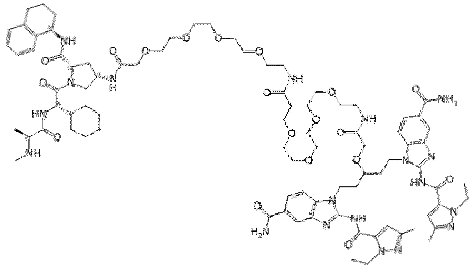 | N-[(3S,5S)-5-[N-(R)-1,2,3,4-tetrahydro-1-naphthylcarbamoyl]-1-{(S)-2-[(S)-2-(methylamino)propionylamino]-2-cyclohexylacetyl}-3-pyrrolidinyl]14-{14-[({3-[5-carbamoyl-2-(1-ethyl-3-methyl-5-pyrazolylcarbonylamino)-1H-1,3-benzimidazol-1-yl]-1-{2-[5-carbamoyl-2-(1-ethyl-3-methyl-5-pyrazolylcarbonylamino)-1H-1,3-benzimidazol-1-yl]ethyl}propoxy}methyl)carbon ylamino]-3,6,9,12-tetraoxatetradecylcarbonylamin o}-3,6,9,12-tetraoxatetradecanamide |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1- Compound of the invention depletes lymphocytes in mice

Male C57BL/6JRj *(wildtype)* mice, 8 weeks of age at arrival, were acclimatized and randomized by bodyweight into two groups of five subjects. Diet and water were administered ad libitum.

The compound of the invention was formulated at 2 mg/mL (in 5% DMSO/20% PEG200/75% PBS; pH~7.4) and was stored at room temperature for up to 4 hours before use. The compound of the invention (or 5% DMSO/20% PEG200/75% PBS; pH~7.4 'vehicle' control) was administered subcutaneously at t=0h and t=14h in the neck skin fold by using an insulin injection needle, with a dose volume of 5 mL/kg bodyweight. At t=16h, terminal blood samples were drawn with a single use 19 G injection needle from the retro-orbital sinus in mice anaesthetized with a butyrophenone fluanison (Hypnorm^{™}) and midazolam mixture. 50 µL blood were mixed with 250 µL saline in vials coated with K3-EDTA and then analysed (double determination) for haematology parameters using an impedance counter (Abacus Junior Vet 3 haematology analyser, Diatron, Hungary).

No adverse effects were observed in the animals. The mean concentration of lymphocytes in blood from animals dosed with the compound was 0.26×10⁹ cells/L, which is a reduction of 93% (p<0.001) compared to the vehicle control group (3.85×10⁹ cells/L), see Figure 1, left hand plot. However, the compound did not significantly affect the monocyte concentration in the mouse (Figure 1, right hand plot).

### EXAMPLE 2 - Effect of the compound of the invention on other white blood cell populations

Following the *in vivo* protocol of Example 1, lymphocyte, monocyte, granulocyte and overall white blood cell counts were measured using a 3-part differential analyzer in mice that had been treated with a compound of the invention, or with the same dose of an equivalent S1-L-E compound in which E is an E3 ubiquitin ligase ligand that is *not* of the Inhibitor of Apoptosis (IAP) subclass (a "control S1-L-E compound" or "control compound").

As reported in Example 1 and shown in Figure 1, the compound of the invention reduced lymphocyte count by 93% but did not significantly affect monocyte count. In contrast, the control S1-L-E compound did not significantly affect lymphocyte count but did significantly increase monocyte count by 90% (data not shown). Neither compound significantly affected overall white blood cell count. However, treatment with the compound of the invention resulted in a five-fold increase in granulocytes (data not shown).

### EXAMPLE 3 - Effect of the compound of the invention, with or without LPS, on cytokine levels

A compound of the invention (Example compound 12; "Ex 12"), or a control S1-L-E compound (control Example compound 7; "Ex 7"), were administered to subjects following the *in vivo* protocol of Example 1. 16 hours after that administration, some subjects were administered lipopolysaccharide (LPS). The compound of the invention substantially increased CXCL10 levels (-100 fold), MCP-1 (CCL2) levels (-50 fold), MIP-1α (CCL3) levels (-10 fold), IL-6 levels (-10 fold) in the serum when administered without LPS. IFN-γ levels also substantially increased above levels that were undetectable in samples from untreated subjects (below 10 mg/mL) in subjects that were administered the compound of the invention, but not those administered with the control compound.

In subjects that received LPS alone, serum levels of CXCL10, IL-6, MCP-1 (CCL2) and MIP-1α (CCL3) were raised 100-1000 fold above the levels in control subjects that received vehicle alone. (In both cases, IFN-γ levels were below the 10 pg/mL threshold of detection.) In subjects that received the compound of the invention, followed by LPS 16 hours later, CXCL10, IL-6, MCP-1 (CCL2) and MIP-1α (CCL3) levels were a further ten-fold higher than the levels in subjects that received LPS alone (see Figure 2). In contrast, CXCL10, IL-6, MCP-1 (CCL2) and MIP-1α (CCL3) levels in subjects that received S1-L-E control Example compound 7 (Ex 7), followed by LPS 16 hours later, were only further elevated to a small extent. IFN-γ levels were only detectable in subjects that had been administered the compound of the invention, with LPS treatment after 16 hours resulting in a further elevation in IFN-γ levels to a small extent. Quantification of cytokines and chemokines was performed on individual serum samples that were stored frozen (≤ -20 °C) after collection, using the electrochemiluminescence-based immunoassay and a custom mouse 5-plex panel according to the manufacturer's instructions (V-Plex assay, Mesoscale Discovery, USA).

### EXAMPLE 4 - STING inhibition in vivo

STING inhibition was measured *in vivo* by administering a compound of the invention, or a control compound, before administering the STING agonist diABZI c3. Mice were pre-treated with either Example compound 7 or Example compound 12 in three dose arms; i) pre-treatment with 10 mg/kg compound at t = -16 hours and 10 mg/kg compound at t = -2 hours; ii) pre-treatment with 10 mg/kg compound at t = -16 hours only, or iii) pre-treatment with 10 mg/kg compound at t = -8 hours only, prior to treatment with 1 mg/kg diABZI c3 at t = 0. Cytokine levels were analysed in serum samples collected 6 hours after treatment with diABZI c3. The administration schedule is shown schematically at the end of Figure 3. Quantification of CXCL10, IL-6, MCP-1 (CCL2), MIP-1α (CCL3) and IFN-γ was performed on serum samples as in EXAMPLE 3. Analysis of the serum levels of the other cytokines was performed by commercial ELISA kits according to the manufacturer's instructions (IFNα; VeriKine, PBL Assay Science, USA; IFNβ and TNFα: DuoSet, R&D Systems, USA).

### EXAMPLE 5 - Western Blot analysis for STING degradation (DC50) in H596, H1650 and HaCat cells

To assess STING degradation in H596 cells (ATCC, HTB-178) cells were seeded at a density of 100.000 cells/well in in 24-well Nunclon Delta surface treated culture plate in RPMI-1640 growth medium (supplemented with 10% FBS, 1% Pen-strep). Cells were incubated over-night at 37°C 5% CO₂ to allow adhesion. Next day growth medium was renewed and a 5x concentration of test compounds (2.5% DMSO) diluted in growth medium were added to the appropriate well. Final DMSO concentration was 0.5%. Cell culture plates were returned to the incubator for additional 24h. The same protocols were used to grow H1650 and HaCat cells except that cells were seeded at a density of 150,000 cells/well and 250,000 cells/well, respectively

Final test compound concentrations were 0.22 µM, 0.67 µM, 2 µM and 6 µM.

For cell lysis, western blotting and data analysis, the following methods were applied.

For cell lysing for Western Blot analysis cell culture plates were brought to ice, supernatant was removed by aspiration and cells washed 2x in ice-cold DPBS. Cells were lysed in 65 µL of RIPA buffer supplemented with cOmplete ULTRA Tablets protease inhibitor cocktail, PIERCE Phosphatase inhibitor mini tablet, Benzonase and 10mM NaF for 20 min on ice. Lysates were collected, cleared by centrifugation (13000xg for 10 min at 4°C). Samples were either analysed directly or stored at -80°C until analysis.

For Western Blotting, total protein concentration in each lysate was determined using PIERCE Gold BCA Assay kit according to manufactures protocol. 6 µg of total protein was mixed with 2x Laemmli buffer containing DTT, boiled for 5 min at 95°C, cooled on ice and loaded into each well of a 4-20% Criterion TGX gel. Proteins were separated by electrophoresis, by running for 1h at 150V. After separation proteins were blotted onto a PVDF membrane by semi-dry transfer using Trans-Blot turbo system at pre-defined BioRad MIDI protocol; 25V, 1A (fixed) for 30 min. Membranes were blocked for 1h in 5% skim milk in TBS-T20 on a rocker at room temperature followed by washing and exposure to primary antibodies against human STING 1:1000 (Cell Signalling, #13647) or loading controls Vinculin 1:1000 (Cell Signalling, #13901) or beta-Actin 1:10000 (Sigma, A5441) all diluted in 5% BSA in TBS-T20. Membranes were probed over night at 4°C on a rocker. Membranes were washed 3x in TBS-T20 followed by exposure to secondary HRP-conjugated anti-rabbit antibody or HRP-conjugated anti-mouse antibody diluted 1:10000 in 5% skim milk in TBS-T20 as appropriate. Membranes were incubated for 1h at room temperature on a rocker, then washed 3x in TBS-T20. Signals were revealed using clarity ECL mixed 1:1 for 1 min and imaged using ImageQuant800.

Representative western blots are shown in Figure 4 for H1650 cells (top panel), H596 (middle panel) and HaCat cells (bottom panel) treated with 6 µM, 2 µM, 0.67 µM, or 0.22 µM of control Example compounds 6 and 7, and Example compound 12 of the invention. The data demonstrate induction of STING degradation using these compounds. Compound of the invention Example compound 12 show a more potent effect of induction of STING degradation in all cells types compared to control Example compound 6, and a more potent effect of induction of STING degradation in H596 and HaCat cells compared to control Example compound 7, and a similar level of degradation to control Example compound 7 in H1650 cells.

### EXAMPLE 6 - Western Blot analysis for STING degradation in mouse RAW264.7 cells

To assess STING degradation in RAW264.7 cells, cells were seeded at a density of 60.000 cells/well in in 24-well Nunclon Delta surface treated culture plate in RPMI-1640 growth medium (supplemented with 10% FBS, 1% Pen-strep). Cells were incubated over-night at 37°C 5% CO₂ to allow adhesion. Next day growth medium was renewed and a 5x concentration of a compound of the invention (2.5% DMSO) diluted in growth medium were added to the appropriate well. Final DMSO concentration was 0.5%. Cells were treated with a titration of test compound from 19.5nM to 10 µM 24 hours prior to cells lysis and subsequently analyzed by WB using anti-STING (1:1000) and anti-VCL (1:1000) antibodies. Controls included UT (DMSO) and 1.8µM diABZI treated cells.

For cell lysis, western blotting and data analysis, the methods described above in EXAMPLE 5 cells were applied.

Figure 5 is a representative Western blot showing that Example compound 12 induces STING degradation in mouse cells in a dose-dependent manner from 19.5 nM to 10 µM.

### EXAMPLE 7 - Western Blot analysis for STING recovery following Example compound washout in HFF-1 cells

To assess STING degradation and recovery in human foreskin fibroblasts HFF-1 cells (ATCC, SCRC-1041) cells were seeded at a density of 60.000 cells/well in in 24-well Nunclon Delta surface treated culture plate in DMEM growth medium (supplemented with 10% FBS, 1% Pen-strep). Cells were incubated over-night at 37°C 5% CO₂ to allow adhesion. Next day growth medium was renewed and a 5x concentration of test compounds (2.5% DMSO) diluted in growth medium were added to the appropriate well. The final DMSO concentration were 0.5%. Cell culture plates were returned to the incubator for additional 24h.

Methods as described above for H596, H1650 and HaCat cells were applied for cell lysis and western blotting. Following 24 hours incubation with the test compounds, cell culture medium containing Example compound was removed, cells washed with fresh medium without Example compound, and incubated in fresh medium without Example compound for the indicated time.

Figure 6 shows that following incubation with either control Example compound 7or Example compound 12 of the invention, STING levels fail to recover to pre-treatment levels after 48 hours following removal of the compound. Slow rebound of STING protein after washout suggests the intracellular half-life of the compounds will allow for a prolonged protein degradation effect and that this prolonged effect will be seen after in vivo exposure.

### EXAMPLE 8 - Western Blot analysis for STING degradation in STING-associated vasculopathy with onset in infancy (SAVI) patient cells

For this study, STING degradation was assessed in primary fibroblast cells with constitutively active STING (comprising a N154S mutation), derived from SAVI patients, and compared to healthy donor primary fibroblast cells.

Cells were seeded at 50,000 cells/well and cultured under standard conditions for 24 hours. Media was replaced with test compound and incubated for 20 hours prior to analysis.

Cell culture medium was replaced with fresh medium containing 1 µM Example compound and incubated for 20 hours. For cell lysis and western blotting, the methods described above for HFF-1 cells were applied.

Figure 7 shows that the compounds induce STING degradation in health donor cells and SAVI patient cells, demonstrating that these compounds can induce degradation of not only wild-type STING but constitutively active STING expressed in patients.

### EXAMPLE 9 - Inhibition of STING-agonist-induced IFN-β release in HFF-1 cells (EC50)

To evaluate cellular effects of test compounds, we assessed inhibition of STING-agonist-induced IFN-β release in human foreskin fibroblasts HFF-1 cells (ATCC, SCRC-1041). Cells were seeded at a density of 10.000 cells/well in in 96-well Nunclon Delta surface treated culture plate in DMEM growth medium (supplemented with 10% FBS, 1% Pen-strep). Cells were incubated over-night at 37°C 5% CO₂ to allow adhesion. Next morning growth medium was renewed and a 7x concentration 3-step dilution series of test compounds (2,1% DMSO) diluted in growth medium were added to the appropriate well. Final DMSO concentration was 0.3%. Cells were further incubated for 24h followed by addition of 8x concentration of GSK STING agonist (CAS number: 2138299-34-8; diABZi c3*; diABZI), final concentration was 0.3 µM. Cells culture plates were further incubated, and supernatants were harvested after 6h and immediately stored at -20°C until determination of IFN-β release.

Cell supernatants were analysed for IFN-β release using DuoSet human IFN-β ELISA (R&D systems, DY-814) following manufactures protocol except using 96-well, half-volume plates from Corning and subsequently use half volume for all steps throughout the protocol. In brief, plates were coated o/n with a 130x dilution of capturing antibody in PBS. Wells were washed 3x with PBS-T20 followed by blocking with 1% BSA diluted in TBS-T20 for 1h. Wells were washed 3x with PBS-T20 and standards (n=2) and samples (n=3) were added to the plate and incubated for 2h at rt. Wells were washed 3x with PBS-T20 and detection antibody diluted 1:60 in 1% BSA in PBS was added to each well and incubated for 2h at rt. Plates were washed 3x with PBS-T20 and Streptavidin-HRP diluted 1:40 in 1% BSA in PBS was added pr well and incubated for 30 min at rt. The wells were washed 4x with PBS-T20 and TMB One Substrate (Promega) was added and incubated for 5-10 min in the dark. When fully developed, 1 mmol/L Sulfuric acid was added to stop the reaction. Signals were measured using BioTek Synergy H1 plate reader at 450nm and 570nm (reference).

Raw data values were quantified based on the standard curve using blank subtracted Δ450nm-570nm values generated using the Gen5 software connected to the plate reader. Concentrations were plotted into GraphPad Prism to calculate EC₅₀ values using a 4-parameter non-linear regression fitting curve, bottom constrains set for >0.

Using the HFF-1 STING inhibition assay described above, the following EC50 values of Example compounds were determined (200 nM ≤ EC50 < 1000 nM = ++; 1000 nM ≤ EC50 < 5000 nM = +):

| **Example number** | **Inhibition of STING-agonist-induced IFN-β release in HFF-1 cells, EC50 (nM)** |
|---|---|
| 14 | + |
| 24 | ++* |

| | |
|---|---|
| * incomplete inhibition | |

### EXAMPLE 10 - Pharmacokinetic profiling of Example compounds in mouse

Mice were administered with 8 mg/kg Example compound 12 by subcutaneous injection. Plasma concentration of test compound was measured over the course of 8 hours.

Subcutaneous injection led to prolonged plasma exposure in mice. 8 hours after injection, the plasma concentration of control Example compound 7 was approximately 100 nM (data not shown), while the plasma concentration of Example compound 12 is approximately 1000 nM (Figure 8). These data are also consistent with plasma concentrations following intravenous injection which show a lower clearance rate of Example compound 12 vs control Example compound 7after 8 hours.

### EXAMPLE 11 - S1 STING ligands of the Example compounds do not induce STING activation and phosphorylation

To assess whether S1 STING ligands inhibit STING activation, primary human skin fibroblasts (Figure 9) or HFF-1 cells (Figure 10) were treated with various compounds which comprise only an S1 ligand domain and a Linker domain before undergoing Western Blot analysis as described below.

Cell culture methods as described above for primary human skin fibroblast and HFF-1 cells were used in the current experiments. Methods as described above for H596, H1650 and HaCat cells were applied for cell lysis and western blotting.

Compounds A9, A11, A26 and A30 used in the following experiments share a common S1 ligand, which is also shared by Example compound 12 of the invention.

| S1 ligand | Structure | IUPAC name |
|---|---|---|
| A9 | 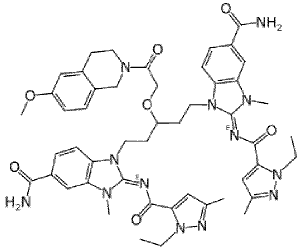 | 1-(5-{5-Carbamoyl-2-[(1-ethyl-3-methyl-5-pyrazolyl)carbonylimino]-3-methyl-2,3-dihydro-1H-1,3-benzimidazol-1-yl}-3-[2-(6-methoxy-1,2,3,4-tetrahydro-2-isoquinolyl)-2-oxoethoxy]pentyl)-2-[(1-ethyl-3-methyl-5-pyrazolyl)carbonylimino]-3-methyl-2,3-dihydro-1H-1 ,3-benzimidazole-5-carboxamide |
| A11 | 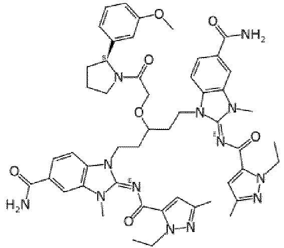 | 1-(3-{2-[(S)-2-(3-Methoxyphenyl)-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| A25 | 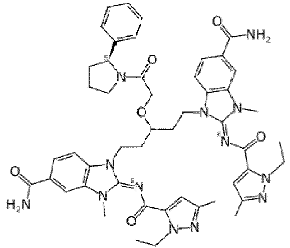 | 1-(3-{2-[(S)-2-Phenyl-1-pyrrolidinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |
| A26 | 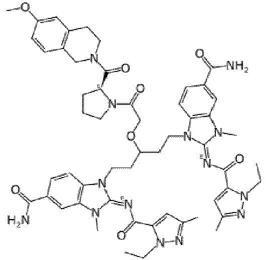 | 1-[3-(2-{(S)-2-[(6-Methoxy-1,2,3,4-tetrahydro-2-isoquinolyl)carbonyl]-1-pyrrolidinyl}-2-oxoethoxy)-5-{5-carbamoyl-2-[(1-ethyl-3-methyl-5-pyrazolyl)carbonylimino]-3-methyl-2,3-dihydro-1H-1,3-benzimidazol-1-yl}pentyl]-2-[(1-ethyl-3-methyl-5-pyrazolyl)carbonylimino]-3-methyl-2,3-dihydro-1H-1,3-benzimidazole-5-carboxamide |
| A30 | 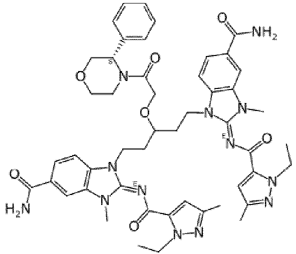 | 1-(3-{2-[(S)-3-Phenyl-4-morpholinyl]-2-oxoethoxy}-5-{5-carbamoyl-2-[(1-ethyl-3-methyl-5-pyrazolyl)carbonylimino]-3-methyl-2,3-dihydro-1H-1,3-benzimidazol-1-yl}pentyl)-2-[(1-ethyl-3-methyl-5-pyrazolyl)carbonylimino]-3-methyl-2,3-dihydro-1H-1,3-benzimidazole-5-carboxamide |

Figure 9 shows the results of Western Blots analysis of human skin fibroblasts treated with 5 µM of A9 or A11 compound either before, simultaneously with, or after activation with the 0.3 µM diABZI. Cells were incubated with A9 or A11 for 1 or 2 hours before addition of diABZI c3, treated with test compound and diABZI c3 simultaneously, or treated with A9 or A11 after 15 minutes incubation with diABZI c3. As a control, cells were also treated with diABZI c3 alone.

Figure 9 shows that under all conditions tested, A9 and A11 inhibited activation of STING, as demonstrated by a reduced intensity of P-STING. In addition, the downstream effector of active STING, TBK1, also showed reduced levels of activation as demonstrated by reduced P-TBK1 intensity compared to cells only treated with diABZI c3. Likewise, activation of the downstream effector LC3B is also reduced following treatment with Compounds A9 and A11 as demonstrated by reduced LC3B lipidation.

In Figure 10A, HFF-1 cells were treated with 5 µM of compounds A11, A25, A26, or A30, or 0.5 µM STING antagonist control for 15 min followed prior to addition of 300 nM diABZI and incubation for 2 h or 5 h. The cells were lysed and analysed on WB. As additional controls, cells were left untreated (UT), treated with Lipofectamine (Lipo control) or with compounds A26 or A30 as single agent. The WB membrane was probed for p-STING, STING, p-IRF3, p-TBK1, and Vinculin.

As expected, in cells treated only with diABZI c3 (-), STING was phosphorylated and activated as were the downstream STING effectors IRF3 and TBK1. In contrast, cells treated with STING antagonist control, STING, TBK1 and IRF3 remained largely unphosphorylated. In cells treated with compounds A11, A25, A26 and A30 prior to diABZI c3 activation, STING, TBK1 and IRF1 phosphorylation was lower compared to cells treated with diABZI c3 alone, which may indicate that these compounds are STING antagonists. In addition, in cells treated with only A26 or A30, no phosphorylation of STING, IRF1 or TBK1 was observed indicating that these compounds are not STING agonists.

In Figure 10B, similar experiments were performed as for Figure 10A, except that cells were activated with 8 µg/mL cGAMP instead of diABZI c3. In addition, the controls used in this set of experiments was A11, A25, A26 and A30 as a single agent with no cGAMP activation. In cells treated with cGAMP only (-), STING was phosphorylated and activated, as were the downstream STING effectors IRF3 and TBK1. In contrast, cells treated with STING antagonist control, STING, TBK1 and IRF3 remained largely unphosphorylated, with the exception of IRF3 after 5 hours of incubation with cGAMP. In cells treated with compounds A11, A25, A26 and A30 prior to cGAMP activation, STING, TBK1 and IRF1 phosphorylation was lower compared to cells treated with cGAMP alone (with the exception of TBK1 in lane A25), which indicates that these compounds are STING antagonists. In addition, in cells treated with only A11, A25, A26 or A30, no phosphorylation of STING, IRF1 or TBK1 was observed indicating that these compounds are not STING agonists.

It is clear from these results that the S1 ligand domain is not a STING agonist.

### EXAMPLE 12 - S1 STING ligands inhibit STING-agonist-induced IFN-β and CXCL10 release in HFF-1 cells

HFF-1 cells were assessed for inhibition of STING-agonist-induced IFN-β and CXCL10 release in cells treated with Compounds A9 and A11.

HFF-1 cells were cultured as described in Example 7 above except that cells were seeded in a 24-well plate at 30,000 cells/well.

Quantification of cytokines and chemokines was performed on culture media samples that were stored frozen (≤ -20 °C) after collection, using the electrochemiluminescence-based immunoassay and a custom mouse 5-plex panel according to the manufacturer's instructions (V-Plex assay, Mesoscale Discovery, USA).

In the first set of experiments (Figure 11A, unfilled bars), cells treated with only diABZI c3 resulted in an IFN media concentration of ~ 200 U/mL. In contrast, pre-treating cells for 1 to 2 hours prior to diABZI c3, simultaneous treatment with A9 and diABZI c3, or pre-treating cells for 15 minutes with diABZI c3 followed by A9 treatment led to a decrease in the IFN media concentration to ~100 U/mL. Similar results were observed for CXCL10 secretion: cells treated with diABZI c3 alone resulted in a CXCL10 media concentration of ~ 60 pg/mL. In contrast, cells pre-treated for 1 to 2 hours prior to diABZI c3, simultaneous treatment with A9 and diABZI c3, or pre-treating cells for 15 minutes with diABZI c3 followed by A9 treatment led to a decrease in the CXCL10 media concentration to ~30 U/mL (Figure 11B, unfiled bars).

In the second set of experiments (Figure 11A; filled bars), cells treated with only diABZI c3 resulted in ~ 100 U/mL IFN media concentration. In contrast, pre-treating cells for 1 to 2 hours prior to diABZI c3, simultaneous treatment with A11 and diABZI c3, or pre-treating cells for 15 minutes with diABZI c3 followed by A11 treatment led to a decrease in the IFN media concentration to -10 - 20 U/mL. Similar results were observed for CXCL10 secretion: cells treated with diABZI c3 alone resulted in a CLCL10 media concentration ~ 60 pg/mL. In contrast, cells pre-treated for 1 to 2 hours prior to diABZI c3, simultaneous treatment with A11 and diABZI c3, or pre-treating cells for 15 minutes with diABZI c3 followed by A11 treatment led to a decrease in the CXCL10 media concentration to -10 - 20 pg/mL (Figure 11B; filled bars).

Together, these data further demonstrate that the S1 ligand of the Example compounds do not act as STING agonists and may act as STING antagonists.

### References

A number of publications are cited above in order to more fully describe and disclose the invention and the state of the art to which the invention pertains. Full citations for these references are provided below. The entirety of each of these references is incorporated herein.
Békés et al., PROTAC targeted protein degraders: the past is prologue; Nat. Rev. Drug Discov., 2022, 21, 181-200.
Decout et al., The cGAS-STING pathway as a therapeutic target in inflammatory diseases; Nat. Rev. Immunol., 2021, 21, 548-569
Konig et al. Familial chilblain lupus due to a gain-of-function mutation in STING; Ann. Rheum. Dis., 2017, 76, 468-472
Mutlu, M., Schmidt, I., Morrison, A.I. et al. Small molecule induced STING degradation facilitated by the HECT ligase HERC4. Nat Commun 15, 4584 (2024). https://doi.org/10.1038/s41467-024-48922-w
Paul et al, Centromere defects, chromosome instability, and cGAS-STING activation in systemic sclerosis; Nat Commun. 2022, 13(1):7074.
Rodero et al., Type I interferon-mediated autoinflammation due to DNase II deficiency; Nat. Comms., 2017, 8, 2176
Seok, J.K., Kim, M., Kang, H.C. et al. Beyond DNA sensing: expanding the role of cGAS/STING in immunity and diseases. Arch. Pharm. Res. 46, 500-534 (2023). https://doi.org/10.1007/s12272-023-01452-3
Siu et al., Discovery of a Novel cGAMP Competitive Ligand of the Inactive Form of STING; ACS Med. Chem. Lett. 2019, 10, 1, 92-97

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A compound of formula (I):
***S1-L-E*** (I),
wherein:
S1 is a STING ligand;
L is a Linker;
E is an Inhibitor of Apoptosis (IAP) E3 ubiquitin ligase ligand,
and pharmaceutically acceptable salts thereof,
wherein the STING ligand does not form a covalent bond with STING and
wherein the STING ligand does not exhibit STING agonist activity.

2. The compound of claim 1, or a pharmaceutically acceptable salt thereof, wherein L connects the STING ligand to the E3 ubiquitin ligase ligand via a chain of atoms having 10-40 atoms in shortest length.

3. The compound of claim 1 or 2, wherein the compound is of formula (I-1): 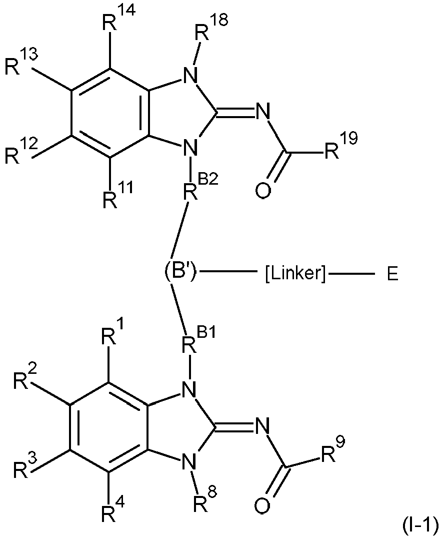
or a tautomer or pharmaceutically acceptable salt thereof,
wherein R⁹ and R¹⁹ are each independently selected from an optionally substituted C₅₋₁₀ cycloalkyl, optionally substituted C₆₋₁₀ carboaryl, optionally substituted C₅₋₁₀ heteroaryl or optionally substituted C₅₋₁₀ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl);
R¹ and R¹¹ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are independently selected from OH, -O-P(O)(OH)₂, C₁₋₄ alkoxy, NH₂, NH(C₁₋₆ alkyl), N(C₁₋₆ alkyl)₂, CO₂(C₁₋₆ alkyl) and halogen;
R³ and R¹³ are each independently selected from H, C(=O)NH₂; optionally substituted - C(=O)NH(C₁₋₆ alkyl), optionally substituted -C(=O)N(C₁₋₆ alkyl)₂, optionally substituted C₁₋₆ alkoxy or optionally substituted CO₂C₁₋₆ alkyl, wherein the optional substituents are independently selected from OH, -O-P(O)(OH)₂, C₁₋₄ alkoxy, NH(C₁₋₆ alkyl), N(C₁₋₆ alkyl)₂, NH₂, CO₂(C₁₋₆ alkyl) and halogen;
R⁴ and R¹⁴ are each independently selected from H, halogen, OH, optionally substituted C₁₋₆ alkyl, optionally substituted C₁₋₆ alkoxy, optionally substituted CO₂C₁₋₆ alkyl, and optionally substituted C₁₋₆ alkylamino wherein the optional substituents are selected from OH, -O-P(O)(OH)₂, C₁₋₄alkoxy, N(C₁₋₆ alkyl)₂, NH(C₁₋₆ alkyl), NH₂, CO₂(C₁₋₆ alkyl) and halogen;
R² and R¹² are each independently selected from H, halogen, OH, optionally substituted C₁₋₆ alkyl, optionally substituted C₁₋₆ alkoxy, optionally substituted CO₂(C₁₋₆ alkyl), and optionally substituted C₁₋₆ alkylamino wherein the optional substituents are selected from OH, -O-P(O)(OH)₂, C₁₋₄ alkoxy, N(C₁₋₆ alkyl)₂, NH(C₁₋₆ alkyl), NH₂, CO₂(C₁₋₆ alkyl) and halogen;
R⁸ and R¹⁸ are each independently selected from H, and C₁₋₄ alkyl;
B' taken together with R^{B1} and R^{B2} forms a linking group having 3-7 atoms in shortest length;
B' is the point of attachment to the Linker group L as follows: 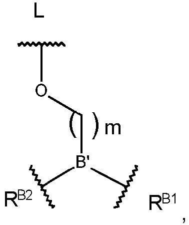
wherein m is 0 or 1;
the Linker (L) is a saturated or a partially or fully unsaturated framework comprising C and H atoms and at least one heteroatom, wherein said framework has a minimum length of from 10 to 40 atoms between B' and E; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₆ alkyl, C₂₋₆ alkenyl, C₂₋₆ alkynyl, C₃₋₆ cycloalkyl, C₃₋₆ carbocyclyl, C₅₋₆ heterocyclyl, C₅₋₆ aryl, and C₅₋₆ heteroaryl; and
E is an Inhibitor of Apoptosis (IAP) E3 ubiquitin ligase ligand.

4. The compound of claim 3, or a tautomer, or a pharmaceutically acceptable salt thereof, wherein R^{B1} and R^{B2} are each independently selected from (-CH₂-)₁₋₂ or a bond and B' taken together with R^{B1} and R^{B2} forms an alkyl linking group having 3-7 atoms in shortest length; and
B' is the point of attachment to the Linker group L as follows: 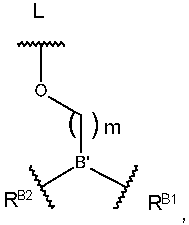
wherein B' is CH or C₁₋₅ alkyl;
wherein m is 0 or 1.

5. The compound of claim 3 or 4, or a tautomer, or a pharmaceutically acceptable salt thereof, wherein R⁹ and R¹⁹ are each independently selected from an optionally substituted C₅₋₆ heteroaryl or optionally substituted C₅₋₆ heterocyclyl, wherein the optional substituents are independently selected from H, halogen, CF₃, optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl, and optionally substituted C₁₋₄ alkoxy, wherein said optionally substituted C₃₋₅ cycloalkyl, optionally substituted C₃₋₅ carbocyclyl, optionally substituted C₄₋₅ heterocyclyl, optionally substituted C₅ heteroaryl, optionally substituted C₁₋₄ alkyl and optionally substituted C₁₋₄ alkoxy is optionally substituted by one or more substituents independently selected from F, CF₃, CI, OH, CN, NH₂, C₁₋₃ alkoxy, CO₂(C₁₋₃ alkyl), N(C₁₋₃ alkyl)₂ and NH(C₁₋₃ alkyl).

6. The compound of any one of claims 3 to 5, or a tautomer, or a pharmaceutically acceptable salt thereof, wherein the compound of formula (I) is of formula (I-2): 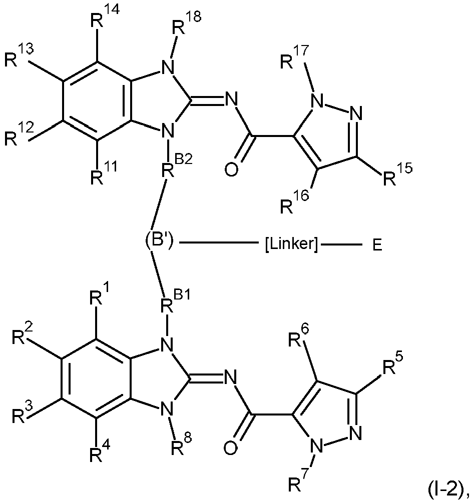
wherein R¹, R², R³, R⁴, R⁸, R¹¹, R¹², R¹³, R¹⁴, R¹⁸, R^{B1}, R^{B2}, B', Linker and E are as defined in claim 7,
wherein R⁷ and R¹⁷ are each independently selected from optionally substituted C₁₋₄ alkyl wherein the optional substituents are independently selected from halogen, -halo(C₁₋₄ alkyl), OH, and C₁₋₄ alkoxy;
R⁶ and R¹⁶ are each independently selected from H, halogen or optionally substituted C₁₋₄ alkyl or C₁₋₄ alkoxy wherein the optional substituents are independently selected from halogen, halo(C₁₋₄ alkyl), OH, and C₁₋₄ alkoxy; and
R⁵ and R¹⁵ are each independently selected from H, cyclopropyl and C₁₋₄ alkyl.

7. The compound of any of claims 3 to 6, or a tautomer, or a pharmaceutically acceptable salt thereof, wherein R⁴, R¹⁴, R², R¹², R¹ and R¹¹ are each independently selected from H, OH, OCH₃, halogen or C₁₋₃ alkyl.

8. The compound of claim 6 or 7, or a pharmaceutically acceptable salt thereof, wherein R⁶ and R¹⁶ are each independently selected from H, OCH₃, halogen or C₁₋₃ alkyl.

9. The compound of any of claims 3 to 8, or a tautomer, or a pharmaceutically acceptable salt thereof, wherein R¹ and R¹¹ are H.

10. The compound of any of claims 3 to 9, or a tautomer, or a pharmaceutically acceptable salt thereof, wherein R⁶ and R¹⁶ are H.

11. The compound of any one of claims 3 to 10, or a tautomer, or a pharmaceutically acceptable salt thereof, wherein R⁴ and R¹⁴ are H.

12. The compound of any one of claims 3 to 11, or a tautomer, or a pharmaceutically acceptable salt thereof, wherein R² and R¹² are H.

13. The compound of any one of claims 3 to 12, or a tautomer, or a pharmaceutically acceptable salt thereof, wherein each of R⁵ and R¹⁵ are C₁₋₄ alkyl or C₁₋₄ alkoxy.

14. The compound of claim 13, or a tautomer, or a pharmaceutically acceptable salt thereof, wherein R⁵ and R¹⁵ are methyl.

15. The compound of any of claims 6 to 14, or a tautomer, or a pharmaceutically acceptable salt thereof, wherein R⁷, R⁸, R¹⁷ and R¹⁸ are C₁₋₄ alkyl.

16. The compound of any of claims 6 to 15, or a tautomer, or a pharmaceutically acceptable salt thereof, wherein R⁷ and R¹⁷ are ethyl.

17. The compound of any one of claims 3-16, or a tautomer, or a pharmaceutically acceptable salt thereof, wherein R⁸ and R¹⁸ are methyl.

18. The compound of any one of claims 3-17, or a tautomer, or a pharmaceutically acceptable salt thereof, wherein R³ and R¹³ are selected from -C(=O)NH(C₁₋₃ alkyl) and -C(=O)NH₂.

19. The compound of any one of claims 3-18, or a tautomer, or a pharmaceutically acceptable salt thereof, wherein R³ and R¹³ are -C(=O)NH₂.

20. The compound of any one of claims 3-19, or a tautomer, or a pharmaceutically acceptable salt thereof, wherein:
R¹ and R¹¹ are selected from H, -OH or C₁₋₃ alkoxy;
R³ and R¹³ are selected from -C(=O)N(H)(C₁₋₃ alkyl) or -C(=O)NH₂;
R⁴ and R¹⁴ are selected from H, halogen, OH or C₁₋₄ alkyl;
R⁷ and R¹⁷ are C₁₋₄ alkyl;
R⁵ and R¹⁵ are selected from H or C₁₋₄ alkyl;
R⁶ and R¹⁶ are H; and
R⁸ and R¹⁸ are methyl.

21. The compound of claim 20, or a tautomer, or a pharmaceutically acceptable salt thereof, wherein:
R¹ and R¹¹ are H;
R³ and R¹³ are -C(=O)NH₂ or -C(=O)-NH-CH₃;
R⁷ and R¹⁷ are ethyl; and
R⁵ and R¹⁵ are methyl.

22. The compound of any one of claims 3 to 21, or a tautomer, or a pharmaceutically acceptable salt thereof, wherein R¹ is identical to R¹¹, R² is identical to R¹², R³ is identical to R¹³, R⁴ is identical to R¹⁴, R^{B1} is identical to R^{B2}, R⁸ is identical to R¹⁸ and R⁹ is identical to R¹⁹.

23. The compound any one of claims 6 to 22, or a tautomer, or a pharmaceutically acceptable salt thereof, wherein R¹ is identical to R¹¹, R² is identical to R¹², R³ is identical to R¹³, R⁴ is identical to R¹⁴, R^{B1} is identical to R^{B2}, R⁸ is identical to R¹⁸, R⁵ is identical to R¹⁵, R⁶ is identical to R¹⁶and R⁷ is identical to R17.

24. The compound of any one of claims 3 to 23, or a tautomer, or a pharmaceutically acceptable salt thereof, wherein B' is of the formula: 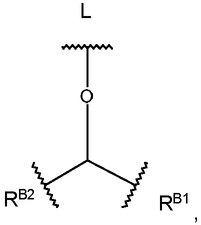
wherein R^{B1} and R^{B2} are each independently selected from (-CH₂-)₁₋₂;
wherein the wavy lines indicate the point of attachment to the Linker (L), R^{B1} and R^{B2} as shown, optionally wherein R^{B1} and R^{B2} are each -CH₂-CH₂- and together with B' have the following structure: 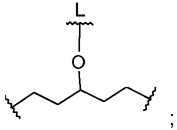
wherein the wavy line next to L represents the attachment to the linker and the wavy lines next to the alkyl groups represent the connection to the N atoms in the S1 structure.

25. The compound of any one of claims 3 to 24, or a tautomer, or a pharmaceutically acceptable salt thereof, wherein compound (I) is of the Formula (I-2b): 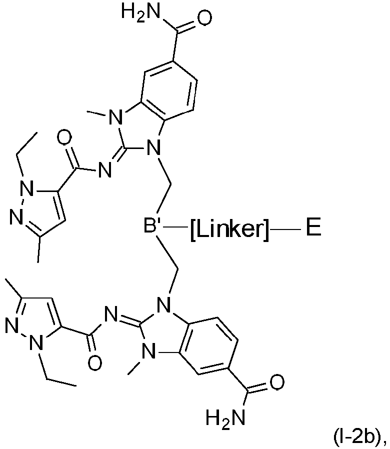 or a tautomer, or a pharmaceutically acceptable salt thereof wherein B' is a C₁₋₅ alkyl and is the connection point to the Linker.

26. The compound of any one of claims 3 to 25, or a tautomer, or a pharmaceutically acceptable salt thereof, wherein the compound of formula (I) is of the formula (I-2c) or (I-2d):

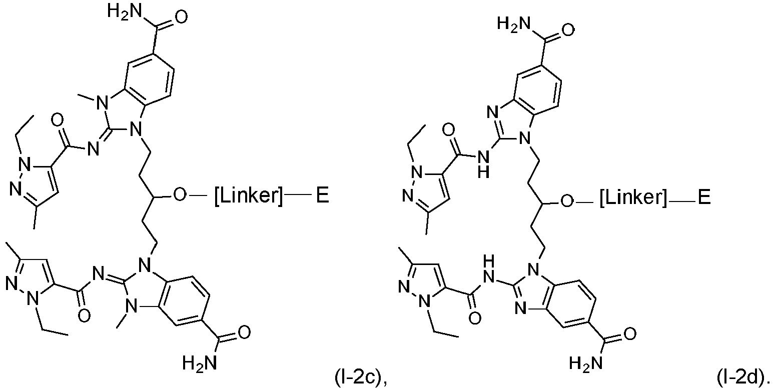

27. The compound according to any one of 1 to 26, or a tautomer, or a pharmaceutically acceptable salt thereof, wherein E has a structure according to Formula E3 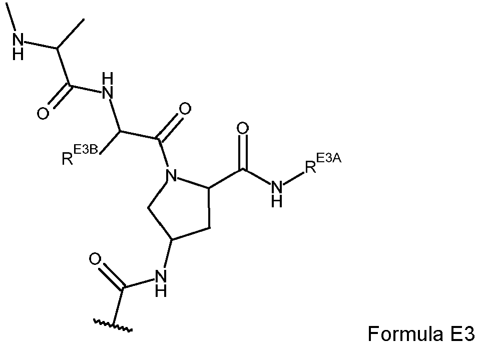 wherein
R^{E3A} is optionally substituted C₅₋₁₀ cycloalkyl or optionally substituted C₆₋₁₀ carboaryl, wherein the optional substituents are selected from C₁₋₆ alkyl, halogen and OH;
R^{E3B} is H, C₁₋₆ alkyl or C₃₋₇ cycloalkyl; and 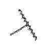
represents attachment point to L.

28. The compound according to claim 27, or a tautomer, or a pharmaceutically acceptable salt thereof, wherein R^{E3A} is an unsubstituted tetralin, optionally wherein R^{E3A} is (*1R*)-1,2,3,4-tetrahydronaphthalen-1-yl.

29. The compound according to either claim 27 or 28, or a tautomer, or a pharmaceutically acceptable salt thereof, wherein R^{E3B} is C₃₋₇ cycloalkyl.

30. The compound according to claim 29, or a tautomer, or a pharmaceutically acceptable salt thereof, wherein R^{E3B} is cyclohexyl.

31. The compound according to any one of 27 to 30, or a tautomer, or a pharmaceutically acceptable salt thereof, wherein E has the following structure: 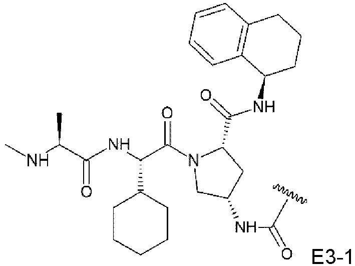 wherein 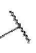 represents attachment point to L.

32. The compound according to any one of claim 1 to 26, or a tautomer, or a pharmaceutically acceptable salt thereof, wherein E has a structure according to Formula E4: 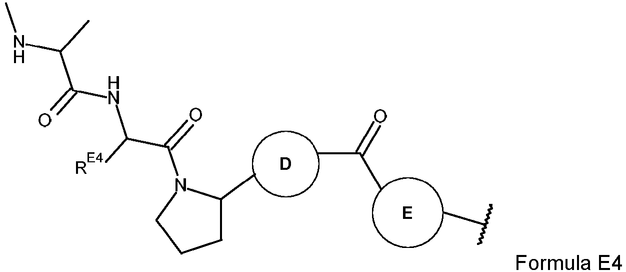 wherein
R^{E4} is H, C₁₋₆ alkyl, or C₃₋₇ cycloalkyl;
Ring D is C₅₋₇ heterocyclyl or C₅₋₇ heteroaryl;
Ring E is C₆₋₁₀ carboaryl, or C₅₋₁₀ heteroaryl; and 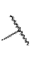
represents attachment point to L.

33. The compound according to claim 32, or a tautomer, or a pharmaceutically acceptable salt thereof, wherein R^{E4} is C₃₋₇ cycloalkyl.

34. The compound according to claim 33, or a tautomer, or a pharmaceutically acceptable salt thereof, wherein R^{E4} is cyclohexyl.

35. The compound according to any one of claims 32 to 34, or a tautomer, or a pharmaceutically acceptable salt thereof, wherein Ring D is C₅₋₇ heteroaryl.

36. The compound according to claim 35, or a tautomer, or a pharmaceutically acceptable salt thereof, wherein Ring D is a C₅₋₇ heteroaryl group containing one N atom and one S atom.

37. The compound according to claim 36, or a tautomer, or a pharmaceutically acceptable salt thereof, wherein Ring D is a 1,3-thiazole linked in position 2 to the pyrrolidine ring and in position 4 to C=O.

38. The compound according to any one of claims 32 to 37, or a tautomer, or a pharmaceutically acceptable salt thereof, wherein Ring E is C₆₋₁₀ carboaryl.

39. The compound according to claim 38, or a tautomer, or a pharmaceutically acceptable salt thereof, wherein Ring E is:

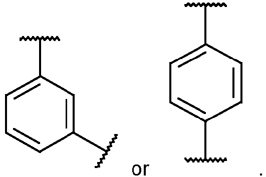

40. The compound according to any one of claims 32 to 39, or a tautomer, or a pharmaceutically acceptable salt thereof, wherein E has the following structure: 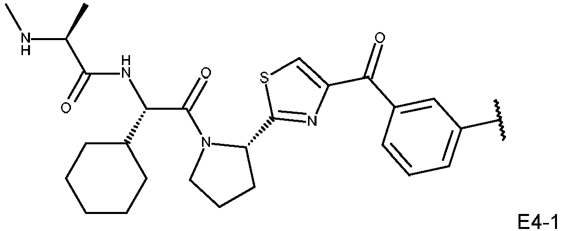 wherein 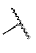 represents attachment point to L.

41. The compound according to any of claims 1 to 26, or a tautomer, or a pharmaceutically acceptable salt thereof, wherein E is selected from one of the following structures:
| **E** | **Structure** | **E** | **Structure** |
|---|---|---|---|
| E4-1 | 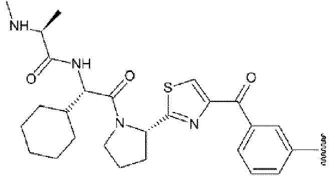 | E3-1 | 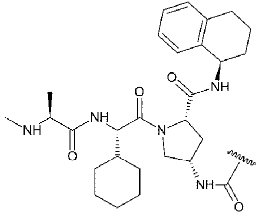 |

42. The compound of any one of claims 1 to 41, or a tautomer, or a pharmaceutically acceptable salt thereof, wherein L is a saturated or a partially or fully unsaturated framework comprising C and H atoms and at least one heteroatom, wherein said framework has a shortest length of 10-40 atoms; wherein said framework may include one or more straight and/or branched chains and/or rings and is optionally substituted on any available C atom(s) by one or more R^{L}, =O, OH, OR^{L}, O-CO-R^{L}, O-CO-NR^{L}₂, O-CO-NHR^{L}, SR^{L}, SO-R^{L}, SO₂-R^{L}, SO₂-NR^{L}₂, SO₂-NHR^{L}, NH₂, NR^{L}₂, NHR^{L}, NR^{L}-CO-R^{L}, NH-CO-R^{L}, NR^{L}-SO-R^{L}, NH-SO-R^{L}, NR^{L}-SO₂-R^{L}, NH-SO₂-R^{L}, NR^{L}-CO-OR^{L}, NH-CO-OR^{L}, NR^{L}-CO-NR^{L}₂, NH-CO-NR^{L}₂, NR^{L}-CO-NHR^{L}, NH-CO-NHR^{L}, F, CF₃, CHF₂, CH₂F, OCF₃, OCHF₂, OCH₂F, Cl, and CN;
wherein each R^{L} is independently selected from C₁₋₃ alkyl and cyclopropyl, and wherein the number of optional substituents is below 10;
wherein said Linker is attached via its point of attachment to an available C, N, O, or S atom at E and the O atom of S1.

43. The compound of any one of claims 1 to 42, or a tautomer, or a pharmaceutically acceptable salt thereof, wherein L connects the STING ligand to the E3 ubiquitin ligase ligand via a chain of atoms having 15-36 atoms in shortest length.

44. The compound according to claim 42 or 43, or a tautomer, or a pharmaceutically acceptable salt thereof, wherein L is a saturated or a partially or fully unsaturated framework comprising C and H atoms, and at least one heteroatom wherein said framework may include one or more of the groups selected from -O-, -C(=O)-, -N(R^{L1})-, -N(R^{L1})-C(=O)-, -C(=O)-N(R^{L1})-, -C₁₋₆alkyl-N(R^{L1}), -N(R^{L1})-C(=O)-C₁₋₆alkyl, - C₁₋₆alkyl-C(=O)-N(R^{L1})-, -C(=O)-N(R^{L1})-C₁₋₆alkyl-, -C₁₋₆alkyl-N(R^{L})-C(=O)-, and/or heterocyclic rings or heteroaryl rings,
wherein R^{L1} is selected from H, C₁₋₆alkyl.

45. The compound according to any one of claims 1 to 44, or a tautomer, or a pharmaceutically acceptable salt thereof, wherein L has the following structure:
*-L_{A}-[X]-L_{B}-*
wherein
L_{A} and L_{B} are each independently selected from a bond, *-C₁₋₆alkyl-, *-O-C₁₋₆alkyl-, *-C₃₋₇cycloalkyl, *-C₄₋₇heterocyclyoalkyl-, *-C₃₋₇cycloalkyl-C₃₋₇cycloalkyl-, *-C₄₋₇heterocyclyoalkyl-C₄₋₇heterocyclyoalkyl-, *-C₁₋₆alkyl-O-, *-O- , *-N(R^{L1})-, *-N(R^{L1})-C(=O)-, *-C(=O)-N(R^{L1})-, *-O-CH₂-C(=O)-N(R^{L1})-, *-N(R^{L1})-C(=O)-CH₂-O- *-N(R^{L1})-C₁₋₆alkyl-, *-C₁₋₆alkyl-N(R^{L1})-, *-N(R^{L1})-C(=O)-C₁₋₆alkyl-, *-C₁₋₆alkyl-C(=O)-N(R^{L1})-, *-C(=O)-, *-C(=O)-C₁₋₆alkyl-,*-C(=O)-N(R^{L1})-C₁₋₆alkyl-, *-C₁₋₆alkyl-N(R^{L1})-C(=O)-, and a functional group selected from carbonyl, ester, amide, carbamate and thiourea;
wherein R^{L1} is selected from H, C₁₋₆alkyl;
X is selected from: a bivalent, saturated or unsaturated, straight or branched, C₁₋₄₅ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, -N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, -S(=O)₂N(R^{L})-, - S(=O)₂N(H)-, C₅₋₇ carbocyclyl, a C₅₋₇ heterocyclyl, a C₆₋₁₀ carboaryl, or a C₅₋₁₀ heteroaryl group;
wherein R^{L} is a C₁₋₆alkyl group; and
* denotes the attachment to S1 or E.

46. The compounds according to claim 45, or a tautomer, or a pharmaceutically acceptable salt thereof, wherein X is selected from: a bivalent, saturated or unsaturated, straight or branched, C₆₋₃₆ hydrocarbon chain wherein one or more methylene groups are individually and optionally replaced by one or more of the groups selected from: -O-, -N(H)-, -N(R^{L})-, -OC(=O)-, -C(=O)O-, -C(=O)-, -N(H)C(=O)-, -N(R^{L})C(=O)-, -C(=O)N(H)-, -C(=O)N(R^{L})-, -S-, -S(=O)-, -S(=O)₂-, -N(R^{L})S(=O)₂-, -NHS(=O)₂-, -S(=O)₂N(R^{L})-, - S(=O)₂NH), C₅₋₇ carbocyclyl, a C₅₋₇ heterocyclyl, a C₆ carboaryl, or a C₅₋₇ heteroaryl group, 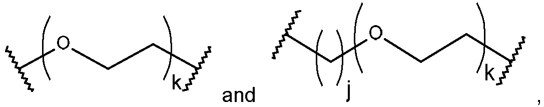 wherein k and j are each individually integers from 1 to 8;
wherein R^{L} is a C₁₋₆alkyl group.

47. The compounds according to claim 46, or a tautomer, or a pharmaceutically acceptable salt thereof, wherein X contains one or more -N(H)-C(=O)- or -C(=O)-N(H)- groups and 1 to 10 of the following group:

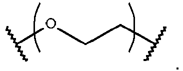

48. The compound of any of the preceding claims, or a tautomer, or a pharmaceutically acceptable salt thereof, wherein L connects the STING ligand to the E3 ubiquitin ligase ligand via the following linker structure:
E-(L7)ᵢ-(L6)ₕ-(L5)_{g}-(OC₂H₄)_{f}-(L4)ₑ-(L3)_{d}-(L2)_{c}-(OC₂H₄)_{b}-(NR^{X}C(=O))-(L1)ₐ-S1
wherein:
L1 is C₁₋₆ alkyl;
L2 is C₁₋₆ alkyl;
L3 is - N(methyl)-C(=O)-, -NH-C(=O)-, -C(=O)NH- or C(=O)N(methyl)-;
L4 is -C(=O)-C₅₋₇ heterocyclyl-C(=O)- or -C(=O)-C₅₋₇ heteroaryl-C(=O)-;
L5 is C₁₋₁₂ alkyl wherein one or more -CH₂- groups is optionally replaced by one or more groups selected from -O-, -NH-, -NMe-, -C(=O)NH, -C(=O)NMe, --NHC(=O)-, NMeC(=O)- group;
L6 is C₁₋₆ alkyl optionally substituted by a =O group;
L7 is -NH-, -NMe-, C₃₋₆cycloalkyl, or C₄₋₆heterocyclyl;
R^{X} is H or methyl.
a is 0 or 1;
b is 0 to 8;
c is 0 or 1;
d is 0 or 1;
e is 0 or 1;
f is 0 to 8;
g is 0 or 1;
h is 0 or 1;
i is 0 to 2;
E is the ubiquitin ligase as defined in any of the preceding claims; and
S1 is S1 as defined in any of the preceding claims.

49. The compound of claim 48, or a tautomer, or a pharmaceutically acceptable salt thereof, wherein L1 is methyl and a is 1.

50. The compound of claim 48 or 49, or a tautomer, or a pharmaceutically acceptable salt thereof, wherein b is 2 to 5.

51. The compound of any one of claim 48 to 50, or a tautomer, or a pharmaceutically acceptable salt thereof, wherein L2 is ethyl.

52. The compound of any one of claims 48 to 51, or a tautomer, or a pharmaceutically acceptable salt thereof, wherein L3 is NHC(=O) or N(methyl)C(=O) and d is 1.

53. The compound of claim any one of claims 48 to 52, or a tautomer, or a pharmaceutically acceptable salt thereof, wherein
L4 is 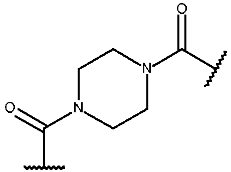 and e is 1.

54. The compound of any one of claims 48 to 52, or a tautomer, or a pharmaceutically acceptable salt thereof, wherein e is 0.

55. The compound of any one of claims 48 to 54, or a tautomer, or a pharmaceutically acceptable salt thereof, wherein f is 0 to 5.

56. The compound of any one of claims 48 to 55, or a tautomer, or a pharmaceutically acceptable salt thereof, wherein L5 is -O-C₄H₈-NHC(=O)-C₂H₄- or -O-C₄H₈-NH-C(=O)-C₂H₄-O-C₂H₄-.

57. The compound of any one of claims 48 to 56, or a tautomer, or a pharmaceutically acceptable salt thereof, wherein g is 0.

58. The compound of any one of claims 48 to 57, or a tautomer, or a pharmaceutically acceptable salt thereof, wherein L6 is methyl, ethyl, butyl or propyl substituted by =O and h is 1.

59. The compound of any one of claims 48 to 58, or a tautomer, or a pharmaceutically acceptable salt thereof, wherein i is 0.

60. The compound of any one of claims 48 to 58, or a tautomer, or a pharmaceutically acceptable salt thereof, wherein L7 is -NH- or C₃₋₆heterocyclyl, and i is 1 or 2.

61. The compound of any one of claims 1 to 40, or a tautomer, or a pharmaceutically acceptable salt thereof, wherein the linker L is selected from one of the following structures:
| **L** | **Structure** |
|---|---|
| LK1 | 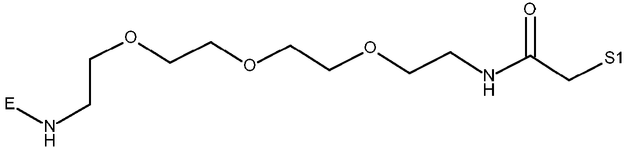 |
| LK2 | 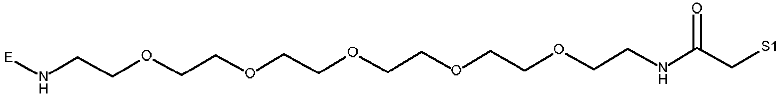 |
| LK3 | 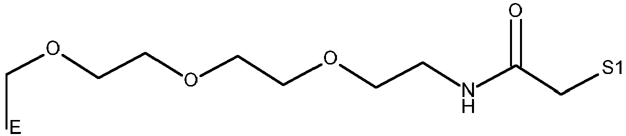 |
| LK4 | 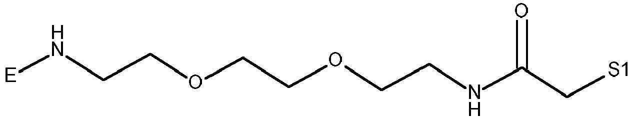 |
| LK5 | 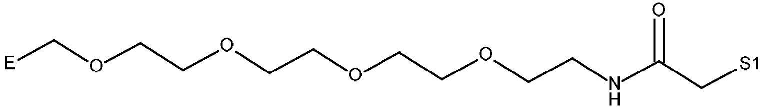 |
| LK6 | 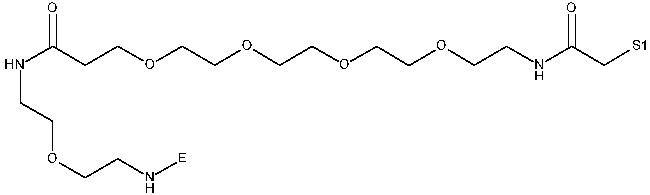 |
| LK7 | 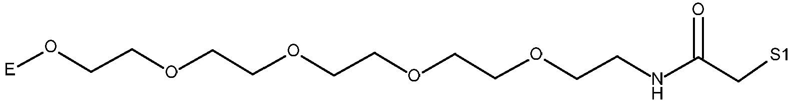 |
| LK8 | 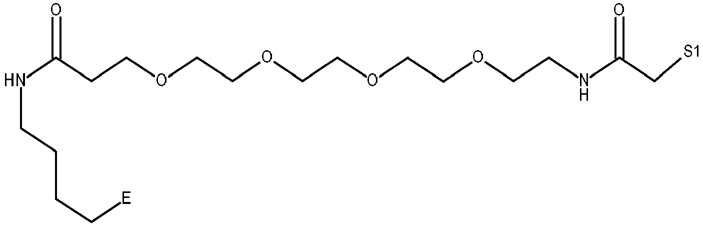 |
| LK9 | 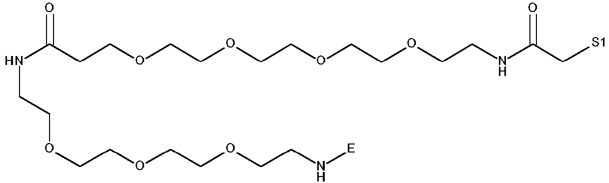 |
| LK10 | 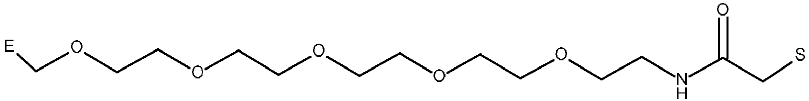 |
| LK11 | 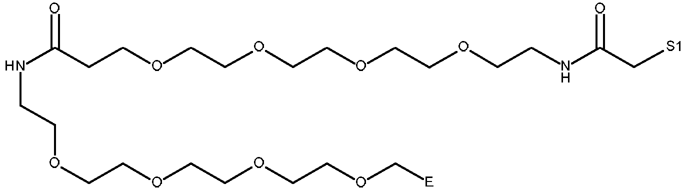 |
| LK12 | 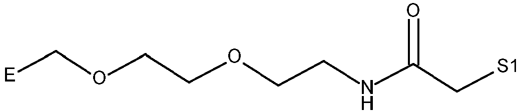 |
| LK13 | 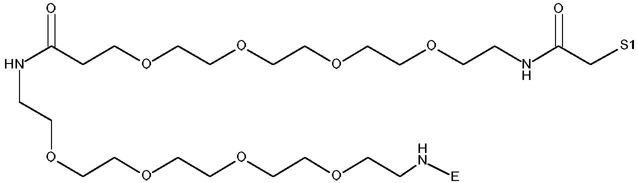 |
| LK14 | 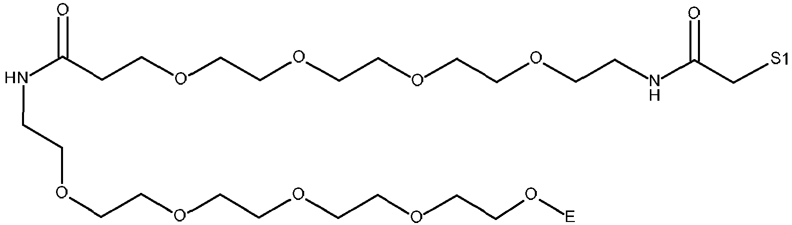 |
| LK15 | 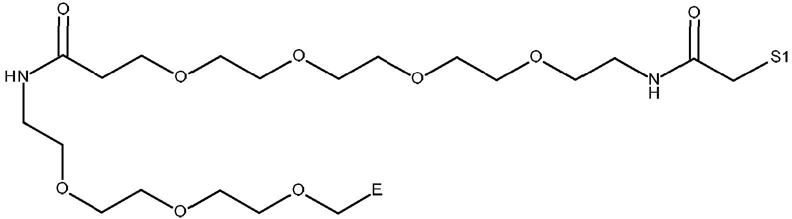 |
| LK16 | 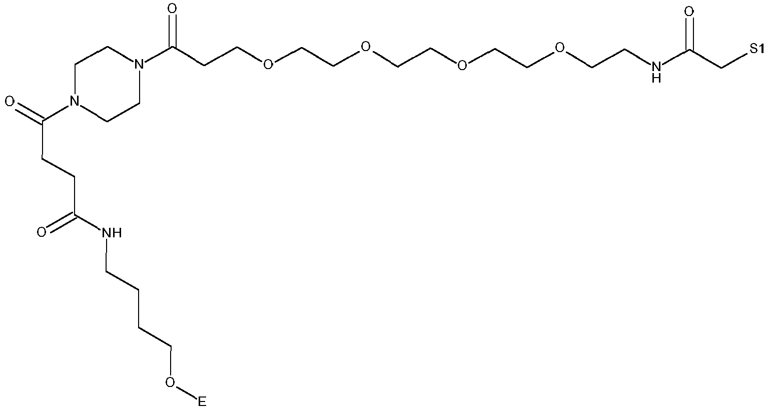 |
| LK17 | 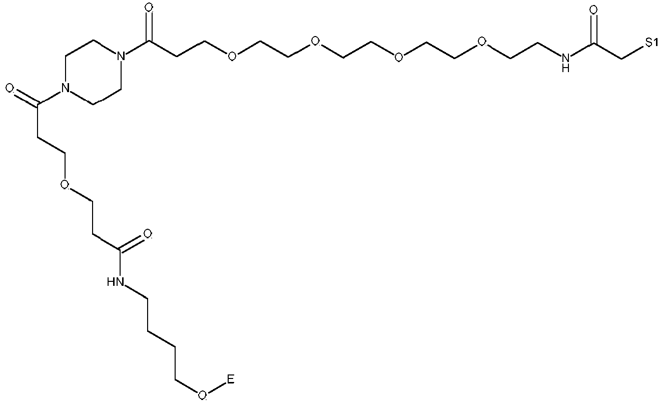 |
| LK18 | 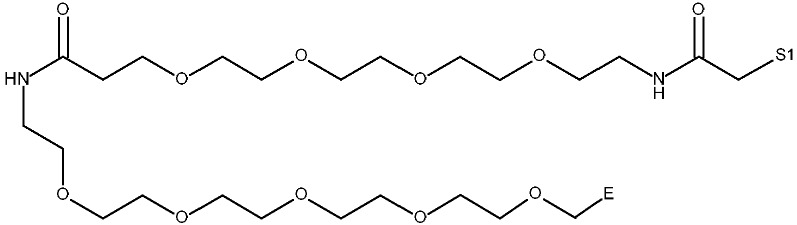 |
| LK19 | 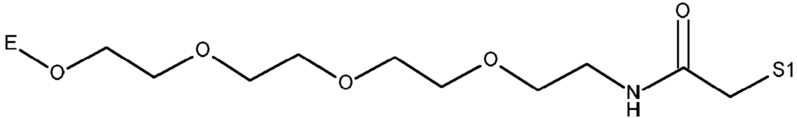 |
| LK20 | 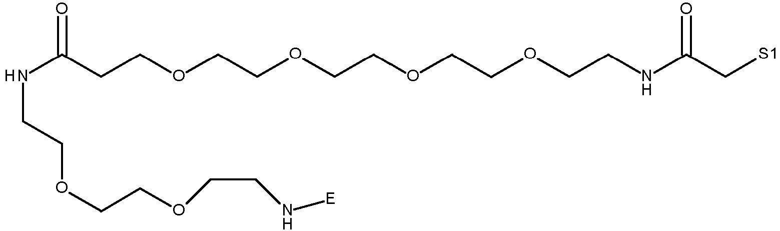 |
| LK21 | 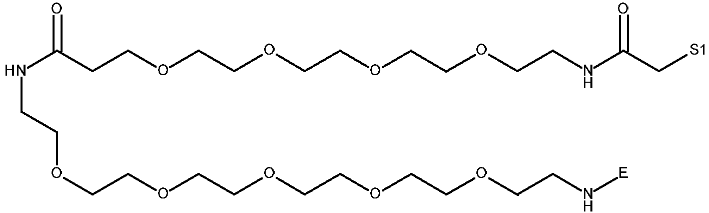 |
| LK22 | 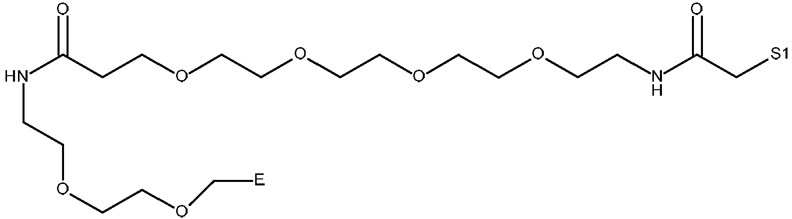 |
| LK23 | 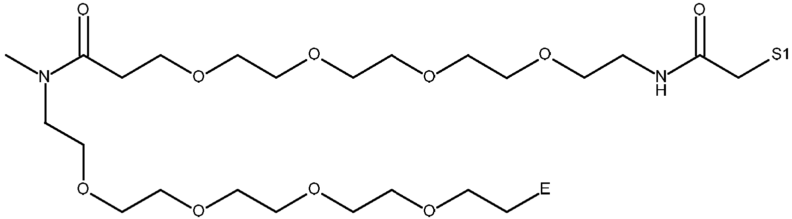 |
| LK24 | 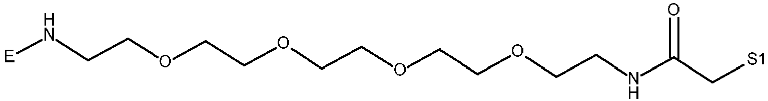 |
| LK25 | 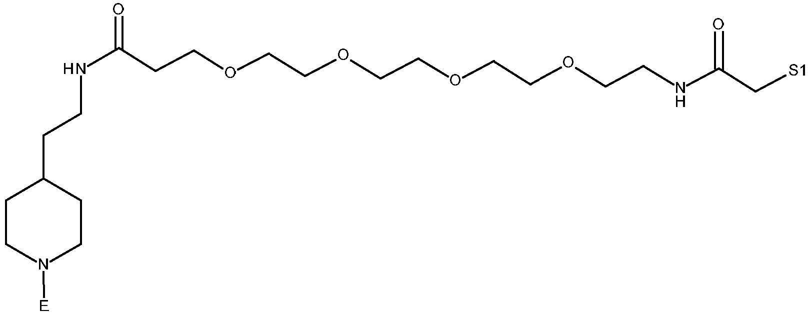 |
| LK26 | 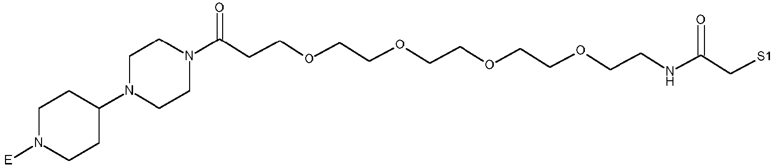 |

62. The compound of any one of claims 1 to 61, or a tautomer, or a pharmaceutically acceptable salt thereof, wherein S1-L-E is selected from one of the following formulae: 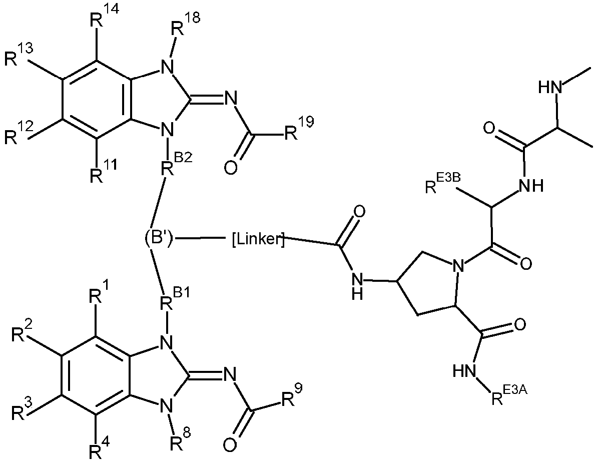 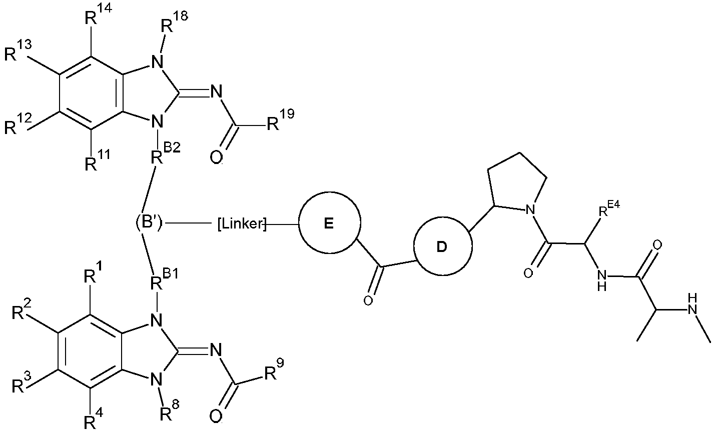 wherein the substituents R¹, R², R³, R⁴, R⁸, R⁹, R^{B1}, B', Linker, R^{B2}, R¹¹, R¹², R¹³, R¹⁴, R¹⁸, R¹⁹ Rings D, E, R^{E3A}, R^{E3B}, and R^{E4} are as defined in any of the preceding claims.

63. A compound of selected from compounds 1-5 or compounds 8-14 of Table 1 or a pharmaceutically acceptable salt thereof.

64. The compound of any one of the preceding claims, or a pharmaceutically acceptable salt thereof, wherein incubating cells with 1 µM of the compound for 16 hours reduces relative STING abundance by at least 35%, at least 40%, at least 45%, 50%, at least 55%, at least 60%, at least 65%, at least 70%, at least 75%, at least 80%, at least 85%, at least 90%, at least 95%, at least 96%, at least 97%, at least 98%, at least 99% as compared to cells not treated with the compound.

65. The compound of any one of the preceding claims, or a pharmaceutically acceptable salt thereof, for use in a method of reducing lymphocyte count in a subject, the method comprising administering a therapeutically effective amount of the compound, or a pharmaceutically acceptable salt thereof, to a subject in need thereof.

66. The compound, or a pharmaceutically acceptable salt thereof, for the use according to claim 65, wherein the subject has received, or is scheduled to receive, an organ or tissue transplant.

67. The compound, or a pharmaceutically acceptable salt thereof, for the use according to claim 65 or 66, wherein the organ or tissue transplant is a solid organ transplant (SOT).

68. The compound, or a pharmaceutically acceptable salt thereof, for the use according to claim 67, wherein the SOT is a kidney, liver, pancreas or heart transplant.

69. The compound, or a pharmaceutically acceptable salt thereof, for the use according to any one of claims 65 to 68, wherein the subject has received the transplant and is suffering from, or is at risk of, graft versus host disease (GvHD).

70. A compound according to any one of claims 1 to 64, or a pharmaceutically acceptable salt thereof, for use as a medicament.

71. Use of a compound according to any one of claims 1 to 64, or a pharmaceutically acceptable salt thereof, for the manufacture of a medicament for the treatment of a disease or condition a subject in need thereof.

72. The compound, or a pharmaceutically acceptable salt thereof, for the use according to any one of claims 65 to 69, wherein upon non-covalent binding of S1 to STING and E to an E3 ubiquitin ligase, STING is degraded.

73. A method of reducing lymphocyte count in a subject, comprising administering a therapeutically effective amount of a compound according to any one of claims 1 to 64, or a pharmaceutically acceptable salt thereof, to a subject in need thereof.

74. A use of the compound according to any one of claims 1 to 64, or a pharmaceutically acceptable salt thereof, in the manufacture of a medicament for reducing lymphocyte count in a subject in need thereof.

75. The compound or a pharmaceutically acceptable salt thereof, for the use according to any one of claims 65 to 69, wherein the subject is a mammal.

76. The compound, or a pharmaceutically acceptable salt thereof, for the use according to claim 75, wherein the mammal is a human.

77. A pharmaceutical composition comprising the compound according to any one of claims 1 to 64, or a pharmaceutically acceptable salt thereof, and a pharmaceutically acceptable excipient.
